(19) Europäisches Patentamt
European Patent Office
Office européen des brevets

(11) Publication number: **0 006 283**
**B1**

(12) **EUROPEAN PATENT SPECIFICATION**

(45) Date of publication of patent specification: **26.01.83**

(51) Int. Cl.³: **C 07 D 307/93**, C 07 D 311/94, C 07 D 333/50, C 07 D 333/78, C 07 D 335/04, A 61 K 31/34, A 61 K 31/35, A 61 K 31/38

(21) Application number: **79300617.2**

(22) Date of filing: **12.04.79**

(54) **Heterotricyclic compounds, processes for their production and pharmaceutical compositions containing them.**

(30) Priority: **14.04.78 JP 44386/78**
**20.10.78 JP 129812/78**
**07.03.79 JP 27170/79**

(43) Date of publication of application:
**09.01.80 Bulletin 80/1**

(45) Publication of the grant of the patent:
**26.01.83 Bulletin 83/4**

(84) Designated Contracting States:
**BE CH DE FR GB IT NL SE**

(56) References cited:
**FR - A - 1 474 107**
**FR - A - 1 498 493**
**FR - A - 2 305 495**
**GB - A - 1 446 632**
**US - A - 3 401 179**

**The file contains technical information submitted after the application was filed and not included in this specification**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Katsube, Junki**
**10-20 Machikaneyama-cho Toyonaka**
**Osaka (JP)**
Inventor: **Shimomura, Hiromi**
**4-1-202, Ryodo-cho Nishinomiya**
**Hyogo (JP)**
Inventor: **Inokuma, Shun**
**2-10-1-146. Sonehigashi-machi Toyonaka**
**Osaka (JP)**
Inventor: **Sugie, Akihiko**
**2-14-7, Mefu Takarazuka**
**Hyogo (JP)**

(74) Representative: **Allard, Susan Joyce et al,**
**BOULT, WADE & TENNANT 27 Furnival Street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 006 283 B1**

Courier Press, Leamington Spa, England.

# Heterotricyclic compounds, processes for their production and pharmaceutical compositions containing them

This invention relates to novel heterotricyclic compounds, a pharmaceutical composition containing at least one of them and processes for preparing them.

British Patent No. 1,446,632 discloses compounds of the formula:

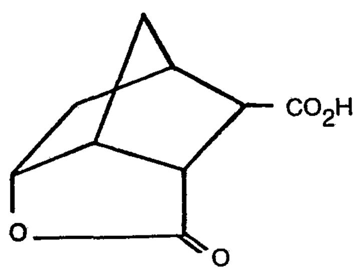

and

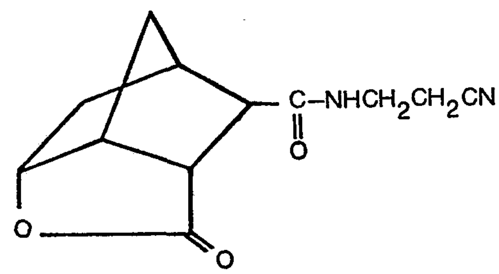

which are useful as intermediates for the production of the compounds of the formula:

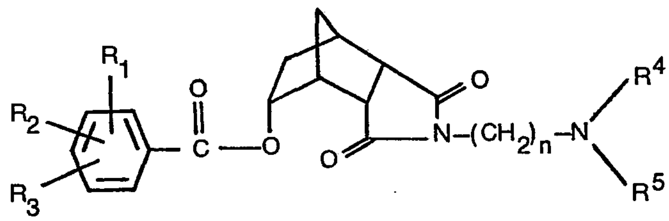

French Patent No. 2,305,495 discloses compounds of the formula:

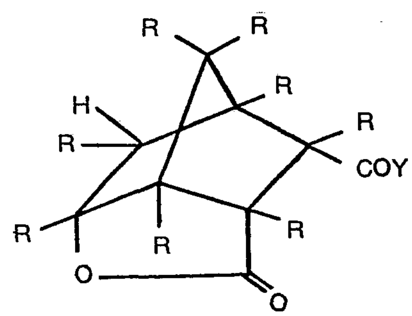

and its use as a lubricant additive.

French Patent No. 1,474,107 discloses compounds of the formula:

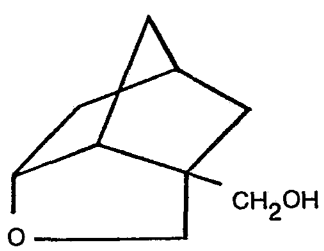

and

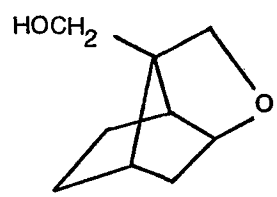

and their use as intermediates for the production of plasticizers and lubricants.

French Patent No. 1,498,493 discloses the compound of the formula:

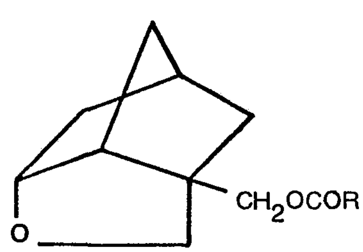

and its use as an intermediate in the production of plasticizers or lubricants.

U.S. Patent No. 3,401,179 discloses the compound of the formula:

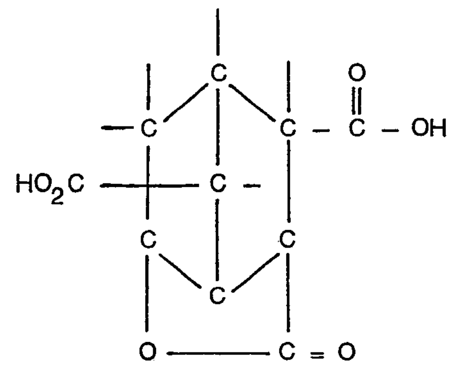

wherein the unsatisfied valences of the carbon atoms, are satisfied by hydrogen of $C_1$—$C_4$ alkyl radicals. The above compound can be used as a plasticizer, an additive for lubricating oils and greases, an alkyl-type resin, and in making agricultural chemicals.

None of the above prior art compounds discloses the antiviral activity of the compounds of the present invention and no medical uses of these prior art compounds have been described.

The novel heterotricyclic compounds of this invention are those represented by the following formula:

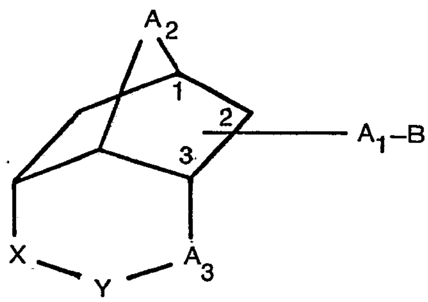

or a non-toxic salt thereof wherein $A_1$ is a single bond or a $C_1$—$C_5$ alkylene or a $C_2$—$C_5$ alkylidene group bonding at the 2- or 3-position, $A_2$ is a $C_1$—$C_3$ alkylene, cyclopropylene or $C_2$—$C_3$ alkylidene group, $A_3$ is a single bond or a methylene group, X—Y is —O—$CH_2$—, —O—CO— or —$SO_n$—$CH_2$— (wherein n is 0 or an integer of 1 or 2) and B is a carboxy group, a $C_2$—$C_5$ alkoxycarbonyl group, a cyano group, a halogen atom, a carbamoyl group of the formula:

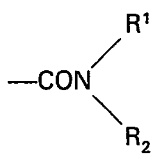

(wherein $R_1$ and $R_2$ are each a hydrogen atom, a $C_1$—$C_5$ alkyl group or a $C_3$—$C_5$ alkenyl group or, when taken together with the adjacent nitrogen atom, may represent a pyrrolidino, piperazino or morpholino group), or an amino group of the formula:

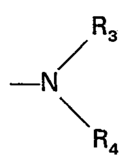

(wherein $R_3$ is a hydrogen atom, a $C_1$—$C_5$ alkyl group, a $C_3$—$C_5$ alkenyl group, a $C_3$—$C_5$ alkynyl group or a $C_7$—$C_9$ aralkyl group and $R_4$ is a hydrogen atom, a $C_1$—$C_5$ alkyl group, a $C_3$—$C_5$ alkenyl group, a $C_3$—$C_5$ alkynyl group, a $C_7$—$C_9$ aralkyl group, a $C_2$—$C_5$ alkoxycarbonyl group, a benzyloxycarbonyl group, a $C_2$—$C_5$ alkanoyl group or a $C_2$—$C_4$ haloalkanoyl group or, when taken together with the

adjacent nitrogen atom, $R_3$ and $R_4$ may represent a pyrrolidino, piperazino or morpholino group), provided that when X—Y is —O—CO—, B is an amino group of the formula:

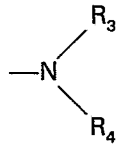

;

and that the alkenyl and alkynl groups do not contain a double or a triple bond at the 1-position:

In another aspect the present invention provides a pharmaceutical composition which comprises together with a pharmaceutically acceptable inert carrier or diluent as an active ingredient a pharmaceutically effective amount of at least one of the compounds of the general formula:

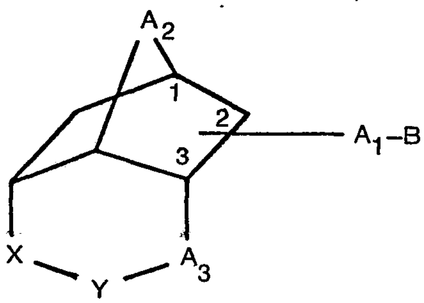

wherein $A_1$ is a single bond or a $C_1$—$C_5$ alkylene or a $C_2$—$C_5$ alkylidene group bonding at the 2- or 3-position, $A_2$ is a $C_1$—$C_3$ alkylene, cyclopropylene or $C_2$—$C_3$ alkylidene group; $A_3$ is a single bond or a methylene group, X—Y is —O—$CH_2$, —O—CO— or —$SO_n$—$CH_2$— (wherein n is 0 or an integer of 1 or 2) and B is a carboxy group, a $C_2$—$C_5$ alkoxycarbonyl group, a cyano group, a hydroxy group, a $C_2$—$C_5$ alkanoyloxy group, a halogen atom, a carbamoyl group of the formula:

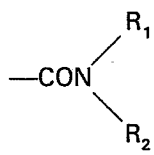

(wherein $R_1$ and $R_2$ are each a hydrogen atom, a $C_1$—$C_5$ alkyl group or a $C_3$—$C_5$ alkenyl group, or, when taken together with the adjacent nitrogen atom may represent a pyrrolidino, piperazino or mopholino group), or an amino group of the formula:

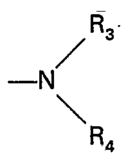

(wherein $R_3$ is a hydrogen atom, a $C_1$—$C_5$ alkyl group, a $C_3$—$C_5$ alkenyl group, a $C_3$—$C_5$ alkynyl group or a $C_7$—$C_9$ aralkyl group and $R_4$ is a hydrogen atom, a $C_1$—$C_5$ alkyl group, a $C_3$—$C_5$ alkenyl group, a $C_3$—$C_5$ alkynyl group, a $C_7$—$C_9$ aralkyl group, a $C_2$—$C_5$ alkoxycarbonyl group, a benzyloxycarbonyl group, a $C_2$—$C_5$ alkanoyl group or a $C_2$—$C_4$ haloalkanoyl group or, when taken together with the adjacent nitrogen atom, $R_3$ and $R_4$ may represent a pyrrolidino, piperazino or morpholino group), and non-toxic salts thereof, provided that when $A_1$ is a single bond, $A_2$ is methylene group or an ethylene group $A_3$ is a single bond, X—Y is —O—CO—, B is a carboxy group and $A_1$ is bonded at the 3-position;

that when X—Y is —O—$CH_2$—, $A_3$ is a single bond and $A_1$ is a methylene group then B is not a hydroxy group;

and that the alkenyl and alkynyl groups do not contain a double or a triple bond at the 1-position.

In the significances as used above, "$C_1$—$C_5$ alkylene" for $A_1$ means an alkylene group having 1 to 5 carbon atoms (e.g. methylene, ethylene or trimethylene), "$C_2$—$C_5$ alkylidene" means an alkylidene group having 2 to 5 carbon atoms (e.g. ethylidene; "$C_1$—$C_3$ alkylene" for $A_2$ means an alkylene group having 1 to 3 carbon atoms (e.g. methylene, ethylene, or trimethylene, "$C_1$—$C_3$ alkylidene" means an alkylidene group having 1 to 3 carbon atoms (e.g. ethylidene or propylidene); "$C_2$—$C_5$ alkoxycarbonyl" for B means an alkoxycarbonyl group having 2 to 5 carbon atoms (e.g. methoxycarbonyl, ethoxycarbonyl, t-butoxycarbonyl); and "$C_2$—$C_5$ alkanoyloxy" for B means an alkanoyl group having 2 to 5 carbon atoms (e.g. acetyloxy, propanoyloxy). Further, "$C_1$—$C_5$ alkyl" for $R_1$, $R_2$, $R_3$ and $R_4$ includes

methyl, ethyl, n-propyl, iso-butyl; "$C_3$—$C_5$ alkenyl" for $R_1$, $R_2$, $R_3$ and $R_4$ are e.g. allyl, crotyl, 3,3-dimethylallyl; "$C_3$—$C_5$ alkynyl for $R_3$ and $R_4$ means e.g. propargyl, 2-butynyl; "$C_7$—$C_9$ aralkyl" for $R_3$ and $R_4$ includes benzyl and phenethyl; "$C_2$—$C_5$ alkoxycarbonyl" for $R_4$ are e.g. methoxycarbonyl, ethoxycarbonyl or t-butoxycarbonyl; "$C_2$—$C_5$ alkanoyl" for $R_4$ are e.g. acetyl, propanoyl, butanoyl; and "$C_2$—$C_4$ haloalkanoyl" for $R_4$ are e.g. chloroacetyl, bromoacetyl or trifluoroacetyl.

It will be understood that the terms "alkenyl" and "alkynyl" as used throughout the specification and claims are not intended to cover the groups in which the double bond or triple bond, respectively, are at the 1-position.

Among the heterotricyclic compounds (I) of this invention, those of the following formulae (Ia—Ii) are preferable, including the non-toxic salts thereof.

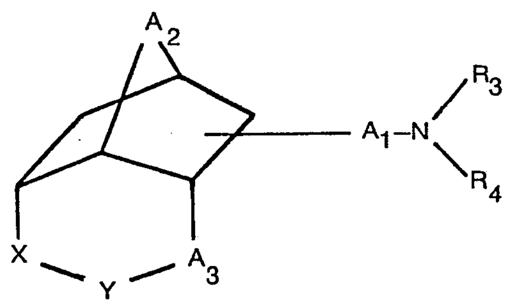

(Ia)

wherein $A_1$, $A_2$, $A_3$, X—Y, $R_3$ and $R_4$ are each as defined above.

Particularly preferred are those of the following formula (Ib):

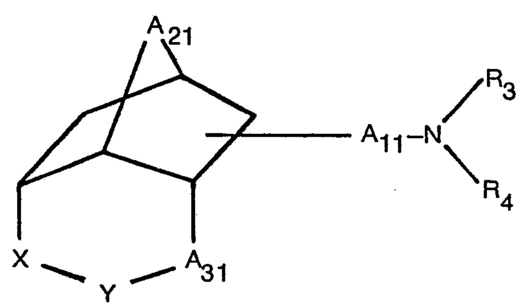

(Ib)

wherein $R_3$, $R_4$ and X—Y are each as defined above, $A_{11}$ is a single bond or a methylene group bonding at the 2- or 3-position, $A_{21}$ is a methylene group or an ethylene group and $A_{31}$ is a single bond or a methylene group.

More particularly preferred are those of the following formula (Ic):

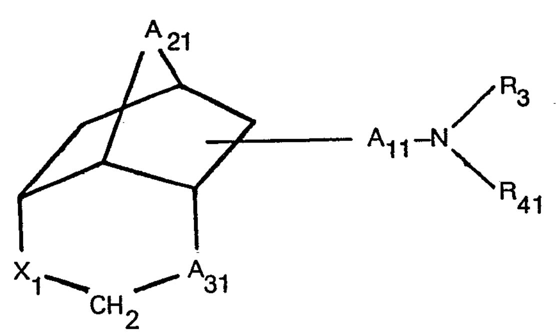

(Ic)

wherein $A_{11}$, $A_{21}$, $A_{31}$ and $R_3$ are each as defined above, $R_{41}$ is a hydrogen atom, a $C_1$—$C_5$ alkyl group or a $C_2$—$C_5$ alkoxycarbonyl group and $X_1$ is —O— or —S—.

The compounds of the following formula (Id):

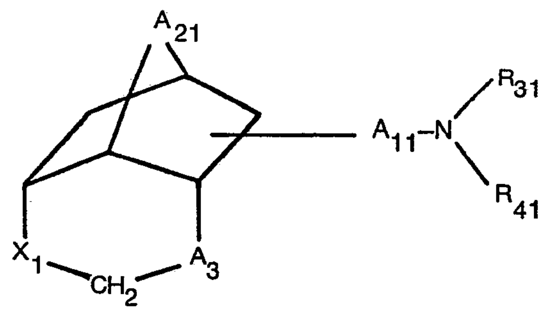

(Id)

wherein $A_{11}$, $A_{21}$, $A_{31}$, $R_{41}$ and $X_1$ are each as defined above and $R_{31}$ is a hydrogen atom or a $C_1$—$C_5$ alkyl group are more preferable.

More preferred are those of the following formula (Ie):

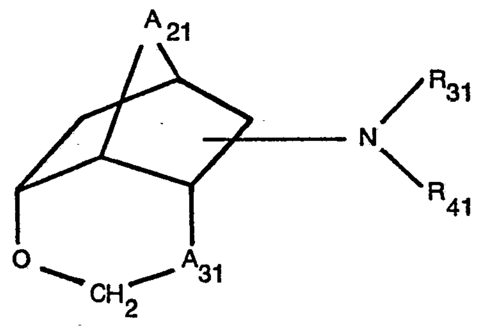

(Ie)

wherein $A_{21}$, $A_{31}$, $R_{31}$ and $R_{41}$ are as defined above.

When B is a carboxy group or a $C_2$—$C_5$ alkoxycarbonyl group in the formula (I), the compounds of the following formula (If):

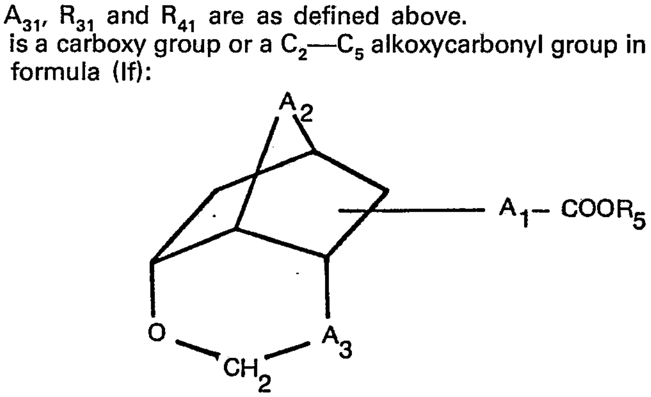

(If)

wherein $A_1$, $A_2$ and $A_3$ are each as defined above and $R_5$ is a hydrogen atom or a $C_1$—$C_4$ alkyl group, are preferable.

Particularly preferred are the compounds of the following formula (Ig):

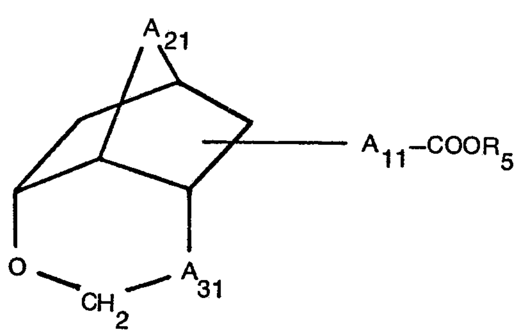

(Ig)

wherein $A_{11}$, $A_{21}$, $A_{31}$ and $R_5$ are each as defined above.

The most preferred are the compounds of the following formula (Ih):

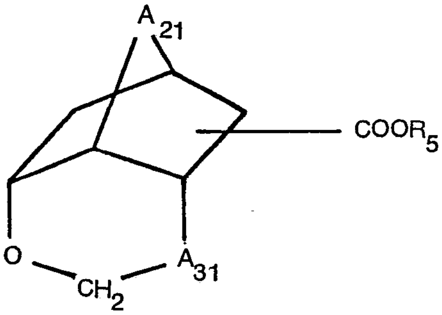

(Ih)

wherein $A_{21}$, $A_{31}$ and $R_5$ are each as defined above. In pharmaceutical compositions comprising compounds of formula (I) in which B is a hydroxyl group, the compounds of the following formula (Ii):

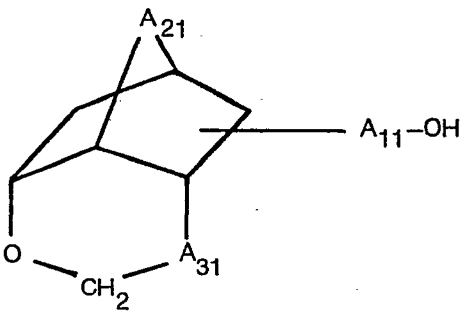

(Ii)

are preferable wherein $A_{11}$, $A_{21}$ and $A_{31}$ are each as defined above, with the provision that $A_{31}$ cannot be a single bond when $A_{11}$ is a methylene group.

The heterotricyclic compounds (I) of the invention possess remarkable antiviral activities against influenza virus and/or herpes simplex virus both in vitro and in vivo evaluation systems.

As a clinically useful anti-influenza A viral agent with a cage structure, amantadine of the following formula is already known (Merck Index 9th, p. 367—368).

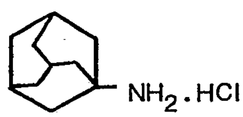

However, it possesses not only an antiviral activity but also strong effects on a central nervous system (CNS). Based on such CNS-effects, amantadine is now marketed as an anti-parkinsonism agent in several countries including Japan, West Germany, United Kingdom or France. For antiviral agents, the CNS-effects of amantadine is deemed as undesirable side effects.

The heterotricyclic compounds (I) of the present invention are found to have high potencies in their anti-influenza viral activities, but surprisingly, exerting no or almost no CNS-effects. In addition, the toxicities of the heterotricyclic compounds (I) are found to be significantly lower than those of amantadine. Thus, the heterotricyclic compounds (I) are very useful as antiviral agents with no or weak side effects or toxicities.

Some of the heterotricyclic compounds (I) of the invention are also useful as intermediates for the production of the heterotricyclic compounds (I).

The heterotricyclic compounds (I) and their non-toxic salts can be administered either alone or in combination with pharmaceutically acceptable carriers. The proportion of the agent administered is determined by the solubility and chemical nature of the compound, chosen route of administration and standard biological practice. For example, they may be administered orally in solid forms, such as starch, mild sugar and so forth. They may be also administered orally in the form of solutions or injected parenterally. For parenteral administration, they may be used in the form of sterile solutions containing other solutes, for example, enough saline or glucose to make the solutions isotonic.

The dosage of the active agents will vary with the form of administration and the particular compound chosen. Furthermore, it will vary with the particular host under treatment. Generally, treatment is initiated with small dosages substantially less than the optimum dose of the compound. Thereafter, the dosage is increased by small increments until the optimum effect under the circumstances is reached.

In general, the compounds of this invention are most desirably administered at a concentration level that will generally afford effective results without causing any harmful or deleterious side effects and preferably at a level that is a range of from 0.1 to 20 mg per kilogram although as aforementioned

variations will occur. However, a dosage level in the range of from 1 to 10 mg per kilogram is most satisfactory. Such doses may be administered once to four times a day, as required.

The heterotricyclic cage compounds (I) of this invention can be prepared by the following methods:

*Method A*

The compound of the formula (Ij):

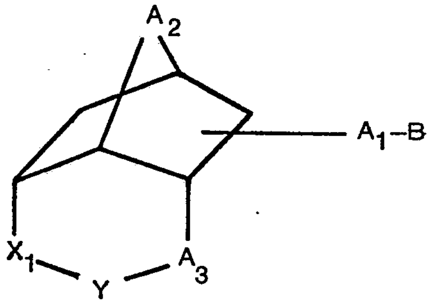

(Ij)

wherein $A_1$, $A_2$, $A_3$ and B are each as defined above and $X_1$—Y is —O—$CH_2$—, —S—$CH_2$— or —O—CO—, can be prepared by cyclizing the compound of the formula (IIa):

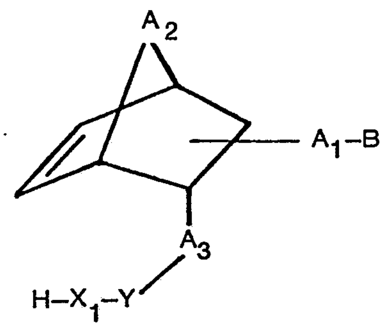

(IIa)

wherein $A_1$, $A_2$, $A_3$, B and $X_1$—Y are each as defined above.

The reaction may generally be carried out in an inert solvent (e.g. methanol, ethanol, dimethylformamide) at a temperature ranging from −20°C to the boiling point of the solvent used in the presence of a suitable catalyst.

When the compounds of the formula (Ij) wherein $X_1$—Y is —O—$CH_2$— or —O—CO— are to be prepared, the reaction may preferably be conducted in the presence of an acid catalyst such as sulfuric acid, alumina, silica-alumina or zeolite at a temperature from room temperature (about 20°C) to the boiling point of the solvent used. On the other hand, when the compounds of the formula (Ij) wherein $X_1$—Y is —S—$CH_2$— are to be prepared, the reaction may preferably be carried out in the presence of such catalysts as tertiary amines or caustic alkalis.

The heterotricyclic compound thus obtained can be separated from the reaction mixture and purified by conventional procedures.

*Method B*

The compound of the formula (Ik):

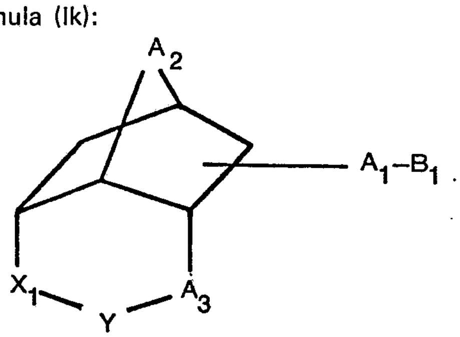

(Ik)

wherein $A_1$, $A_2$ and $A_3$ are each as defined above, $B_1$ is the same as B excluding a halogen atom and

$X_1$—Y is —O—$CH_2$—, —S—$CH_2$— or —O—CO—, can be prepared by reducing the compound of the formula (IIb):

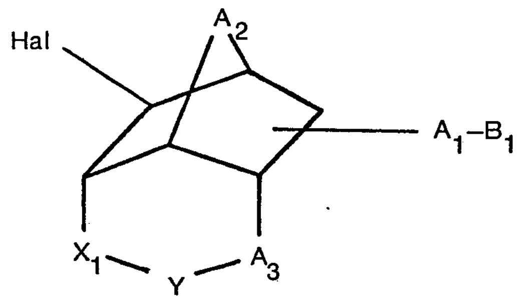

(IIb)

wherein $A_1$, $A_2$, $A_3$, $B_1$ and $X_1$—Y are each as defined above and Hal is a halogen atom, in an inert solvent.

The reducing reaction may be carried out by treating the compound of the formula (IIb) with a suitable reducing agent in an inert solvent at a temperature ranging from −78°C to the boiling point of the solvent used, preferably from room temperature to the boiling point of the solvent used. Examples of such reducing agent are metal hydride compounds such as lithium aluminum hydride, sodium trialkoxyaluminum hydride, sodium diethylaluminum hydride, sodium bis(methoxyethoxy)aluminum hydride, sodium borocyanohydride, tributyltin hydride, dibutyltin hydride and triphenyltin hydride. As the solvent, there may be exemplified ether, tetrahydrofuran, dimethoxyethane, methanol, ethanol, benzene, toluene.

When the compound of the formula (IIb) having a functional group susceptible to reduction (e.g. ether, tetrahydrofuren and cyclohexane) is to be used as the starting material, the reducing reaction may preferably be conducted by using an alkyltin hydride with a suitable radical initiator such as azobisisobutyronitrile. The reaction may also be achieved by hydrogenation in the presence of a suitable catalyst such as palladium charcoal (Pd—C), platinum oxide ($PtO_2$) or Raney-Ni. The hydrogenation is generally carried out in an inert solvent, preferably at a temperature of from room temperature to the boiling point of the solvent used. As the solvent, those as exemplified above may also be used.

The heterotricyclic compound thus obtained can be separated from the reaction mixture and purified by the conventional procedures.

*Method C*

The compound of the formula (II):

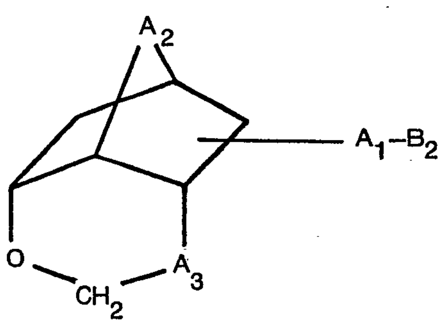

(II)

wherein $A_1$, $A_2$ and $A_3$ are each as defined above and $B_2$ is a cyano group, a carbamoyl group of the formula:

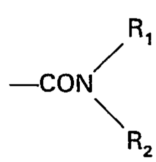

wherein $R_1$ and $R_2$ are each as defined above, or an amino group of the formula:

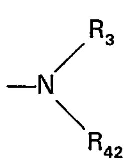

wherein $R_3$ is as defined above and $R_{42}$ is a $C_2$—$C_5$ alkoxycarbonyl group, a benzyloxycarbonyl group, a

$C_2$—$C_5$ alkanoyl group or a $C_2$—$C_4$ haloalkanoyl group, can be prepared by reacting the compound of the formula (IIc):

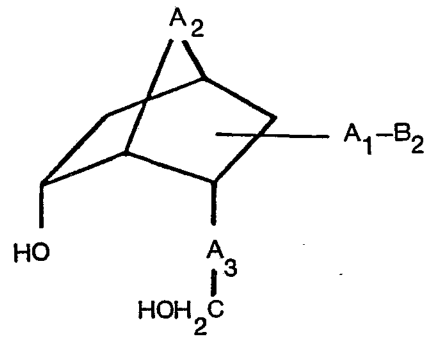

(IIc)

wherein $A_1$, $A_2$, $A_3$ and $B_2$ are each as defined above, with a dehydrating agent.

As the dehydrating agent, a hydroxy-activating reagent such as p-toluenesulfonyl chloride, mesyl chloride, thionyl chloride or phosphorus oxychloride may be used. The reaction may be carried out in the presence of an inert solvent (e.g. ether, benzene, toluene, pyridine, triethylamine). The reaction temperature may vary from −20°C to the boiling point of the solvent used.

The heterotricyclic compound thus obtained can be separated from the reaction mixture and purified by the conventional procedures.

*Method D*

The compound of the formula (Im):

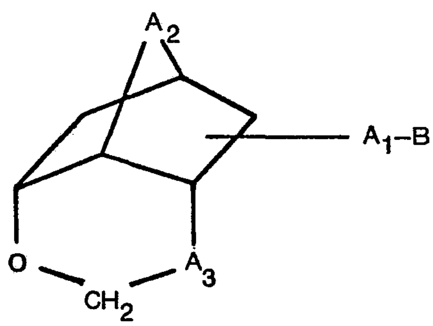

(Im)

wherein $A_1$, $A_2$, $A_3$ and B are each as defined above, can be prepared by cyclizing the compound of the formula (IId):

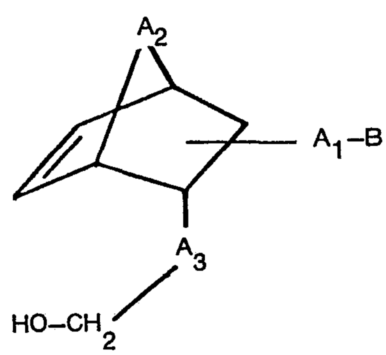

(IId)

wherein $A_1$, $A_2$, $A_3$ and B are each as defined above, according to the oxymercuration-demercuration reaction.

The oxymercuration-demercuration reaction may be carried out by treating the compound of the formula (IId) with a mercuric salt (e.g. $Hg(OCOCH_3)_2$, $Hg(OCOCCl_3)_2$, $Hg(OCOCF_3)_2$, $Hg(NO_3)_2$ and reducing the resulting product (oxymercuration adduct) with a reducing agent such as sodium borohydride. The reaction can be carried out in the presence of an inert solvent (e.g. water, tetrahydrofuran, dioxane, dimethylformamide). The reaction temperature may be from 0°C to the boiling point of the solvent used.

The heterotricyclic compound thus obtained can be separated from the reaction mixture and purified by the conventional procedures.

*Method E*

The compound of the formula (In):

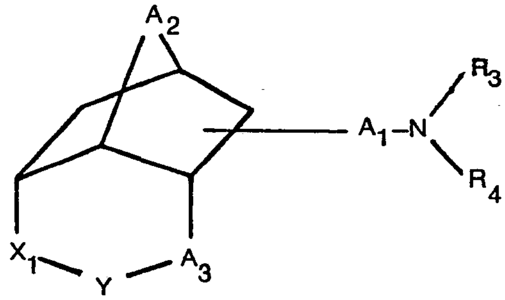

(In)

wherein $A_1$, $A_2$, $A_3$, $R_3$, $R_4$ and $X_1$—Y are each as defined above, can be prepared from the corresponding tricyclic compound having a suitable substituent by the following methods thereby converting the substituent into an amino group represented by

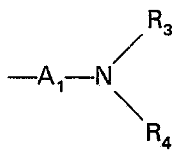

:

a) *via Reduction*

(1) $-A_{12}-CN \longrightarrow -A_{12}-CH_2NH_2$

(wherein $A_{12}$ is a single bond or a $C_1$–$C_4$ alkylene group)

(2) 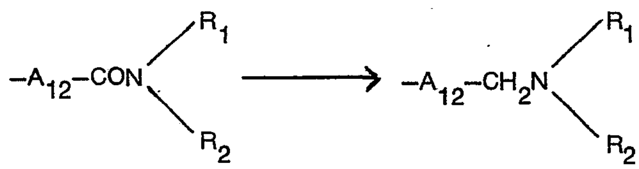

(wherein $R_1$ and $R_2$ are each as defined above)

(3) 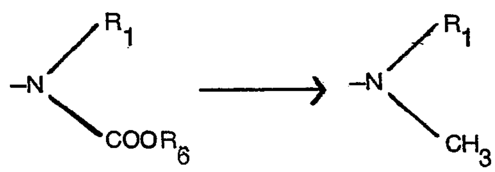

(wherein $R_6$ is a $C_1$–$C_4$ alkyl group)

(4) 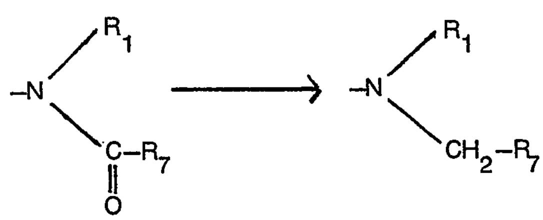

(wherein $R_7$ is a hydrogen atom, a $C_1$–$C_4$ alkyl group or a $C_2$–$C_4$ alkenyl group)

(5) 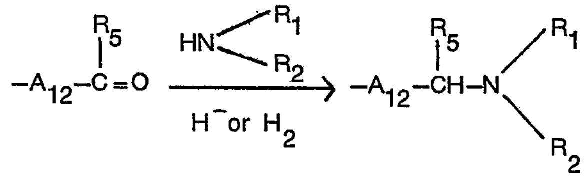


(wherein $R_5$ is as defined above)

(6) 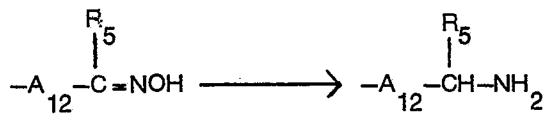

(7) 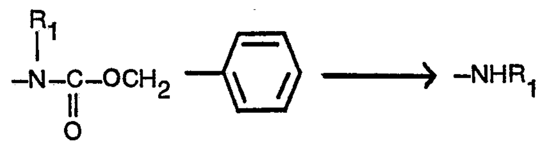

The reduction (1), (2), (3), (4) or (6) may preferably be carried out by treatment with a metal hydride compound such as lithium aluminum hydride, sodium trialkoxyaluminum hydride, sodium diethylaluminum hydride or sodium bis(methoxyethoxy)aluminum hydride in an inert solvent such as ether, tetrahydrofuran or toluene. In this type of reaction, the compound of the formula (In) wherein Y is a methylene moiety is obtained.

The reaction (5) is known as the reductive amination and it may be effected by reacting the carbonyl compound with ammonia or an amine in the presence of a reducing agent such as a metal borohydride, an active metal and an acid or formic acid or its derivative in a solvent such as methanol, ethanol, benzene, toluene.

The reduction (1), (5), (6) or (7) may be also effected by hydrogenation in the presence of a catalyst such as palladium charcoal, platinum oxide or Raney-Ni in an inert solvent such as methanol, ethanol, benzene or toluene. The temperature for the above reduction may vary depending on the type of reduction or reducing agent to be used therein, but these reactions (1) to (7) may generally be carried out at a temperature of from —20°C to the boiling point of the solvent to be used therein.

*b) via Substitution*

(1) 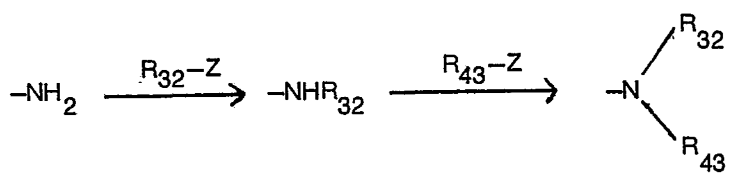


(wherein Z is the residue of a strong acid, $R_{32}$ is the same as $R_3$ excluding a hydrogen atom and $R_{43}$ is the same as $R_4$ excluding a hydrogen atom)

(2) 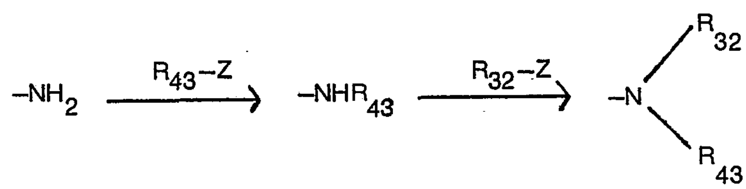


(3) 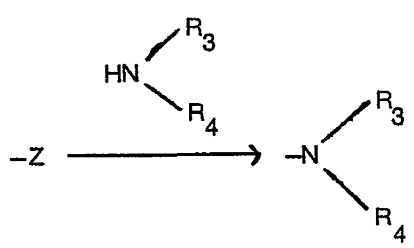

The reaction (1) or (2) may be effected by reacting the amine compound (—$NH_2$, —$NHR_{32}$ or

—$NHR_{43}$) with the activated radical such as $R_{32}$—Z or $R_{43}$—Z. The reaction (3) may also be achieved by reacting the compound of the formula (Io):

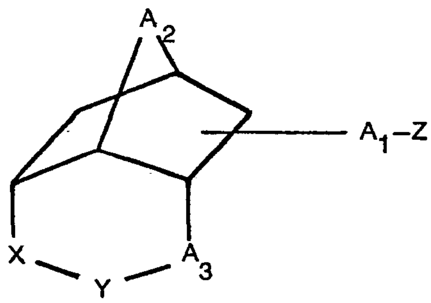

(Io)

wherein $R_1$, $A_2$, $A_3$, X—Y and Z are each as defined above, with the amine

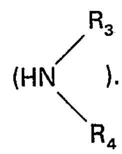

Examples of the residue of a strong acid represented by Z are chloride (—Cl), bromide (—Br), p-toluenesulfonate

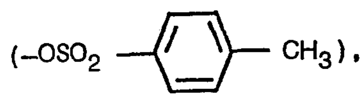

methanesulfonate (—$OSO_2$—$CH_3$), sulfate (—O—$SO_2$—O—). The reaction (1), (2) or (3) may preferably be carried out by using an organic or inorganic base.

A variety of bases can be used in these reactions (1) to (3) (e.g. NaH, NaOMe, t-BuOK, NaOH, KOH, $Na_2CO_3$, $K_2CO_3$, $NaHCO_3$, triethylamine, pyridine, dimethylaniline). The base may preferably be used from 1 to 3 equivalent to the activated radical to be used therein.

In the reaction (1) or (2), $R_{32}$—Z or $R_{43}$—Z may preferably be used from 1 to 3 equivalent to the amine to be used therein, and in the reaction (3), the amine

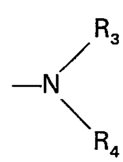

may preferably be used in an excess to the compound of the formula (Io).

The reactions (1) to (3) can be carried out in the presence of an inert solvent (e.g. benzene, toluene, tetrahydrofuran, ether, dimethoxyethane, methanol, ethanol). The reaction conditions may vary depending upon the reaction temperature and the activated radicals to be used therein. The reaction temperature may be from 0°C to boiling point of the solvent used.

When each of $R_{32}$ and $R_{43}$ means an alkyl group, an alkenyl group, an alkynyl group or an aralkyl group, over-substitution may often occur giving a mixed product, and therefore in these cases, milder conditions may preferably be employed.

*c) via Hydrolysis*

(1) 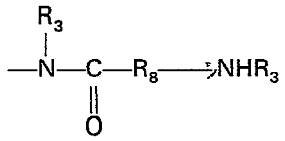

(wherein $R_8$ is a hydrogen atom, a $C_1$—$C_4$ alkyl group, a $C_1$—$C_3$ haloalkyl group, a $C_1$—$C_4$ alkoxy group or a benzyloxy group)

(2) 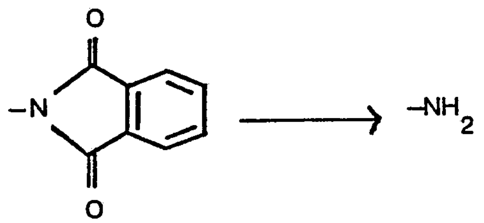

The reaction (1) or (2) may be effected by treating the amide, the urethane or the phthalimide under a basic or acidic aqueous medium.

Caustic alkalis such as sodium hydroxide, potassium hydroxide and barium hydroxide may preferably be used for these reactions, and hydrohalogenic acids such as hydrochloric acid, hydrobromic acid and hydroiodic acid may also preferably be used.

The reactions (1) and (2) may be carried out using a co-solvent such as methanol, ethanol, butanol, ethylene-glycol, dimethylformamide or dimethylsulfoxide. The reactions (1) and (2) may mostly be carried out by heating the reaction mixture at a temperature from room temperature to the refluxing temperature of the reaction system in order to complete the hydrolysis.

The reaction (2) may also be achieved by the modified method of Manske (J. Chem. Soc., 1926, 2348); namely, treatment of the phthalimide with hydrazine hydrate may afford the phthalhydrazide, and the resulting phthalhydrazide may easily be hydrolyzed by treatment with hydrochloric acid.

*d) via Rearrangement*

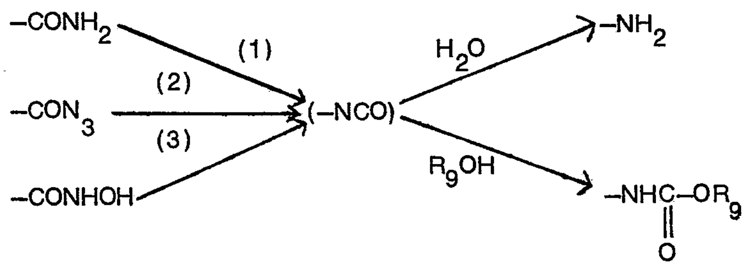


(wherein $R_9$ is a $C_1$—$C_4$ alkyl group or a benzyl group).

The types of the reactions (1), (2) and (3) are all well known rearrangement reactions; the reaction (1) is the Hofmann rearrangement (Org. Reactions, *3*, 267); the reaction (2) is the Curtius rearrangement (Org. Reactions, *3*, 337); and the reaction (3) is the Lossen rearrangement (Chem. Review, *33*, 209 (1943)).

By these reactions, the isocyanate may initially be formed, from which the amine may be obtained by hydrolysis, and the urethane may be obtained by treatment with an alcohol ($R_9$—OH).

The reaction (1) may be effected by treatment of the amide with an alkali hypohalite (e.g. sodium hypobromite, sodium hypochlorite). The reaction (2) or (3) may be effected by heating the acyl azide or the hydroxamic acid. The reaction (3) may also be effected by treating the hydroxamic acid with a base (e.g. sodium hydride).

The reactions (1) to (3) may generally be carried out in a suitable solvent (e.g. water, methanol, ethanol, tetrahydrofuran, benzene, toluene) at a temperature from room temperature to the refluxing temperature of the reaction system.

The isocyanate obtained as above may be hydrolyzed into the amine in a conventional way. For example, the hydrolysis can be carried out by treating the isocyanate with an alkali (e.g. NaOH, KOH) or an acid (e.g. HCl, $H_2SO_4$) in water or a mixture of water and an organic solvent (e.g. dioxane, ethylene-glycol, dimethylformamide) at a temperature ranging from room temperature to the refluxing temperature of the reaction system.

When the reaction (1), (2) or (3) is carried out in the presence of an alcohol ($R_9$—OH), the urethane may be obtained as a product of the rearrangement.

The heterotricyclic compound thus obtained can be separated from the reaction mixture and purified by the conventional procedures.

*Method F*

The compound of the formula (Ip):

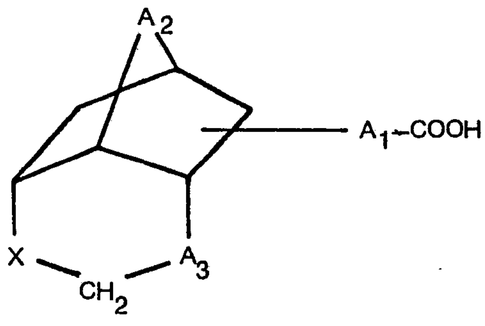

(Ip)

wherein $A_1$, $A_2$ and $A_3$ are each as defined above and X is —O— or —$SO_n$— (wherein n is 0, 1 or 2), can be prepared by hydrolysis of the compound of the formula (Iq):

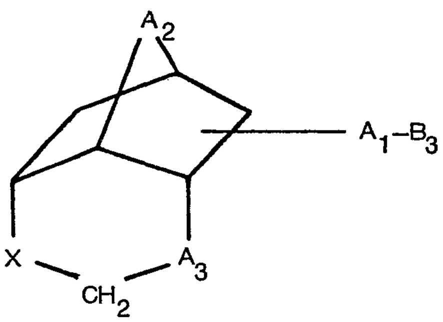

(Iq)

wherein $A_1$, $A_2$, $A_3$ and X are each as defined above and $R_3$ is a cyano group, a $C_2$—$C_5$ alkoxycarbonyl group, a benzyloxycarbonyl group or a carbamoyl group of the formula:

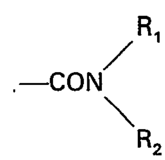

wherein $R_1$ and $R_2$ are each as defined above.

The hydrolysis may preferably be carried out by heating the compound of the formula (Iq) with a caustic alkali (e.g. sodium hydroxide, potassium hydroxide, barium hydroxide) in water or a mixture of water and an organic solvent at room temperature to the refluxing point of the reaction system. The objective compound of the formula (Ip) can be isolated by acidifying the resulting reaction mixture. As the organic solvent, methanol, ethanol, butanol, ethyleneglycol, dimethylformamide, dimethylsulfoxide or the like may be used for this reaction.

The heterotricyclic cage compound thus obtained can be separated from the reaction mixture and purified by the conventional procedures.

*Method G*

The compound of the formula (Ir):

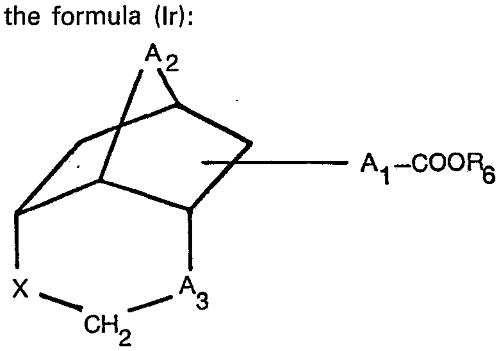

(Ir)

wherein $A_1$, $A_2$, $A_3$, X and $R_6$ are each as defined above, can be prepared by forming the alkoxycarbonyl

group represented by $-COOR_6$ on the tricyclic system by the following procedure:

(1) $-COOH + R_6-OH \xrightarrow{H^+} -COOR_6$

(2) $-COOH + CH_2N_2 \longrightarrow -COOCH_3$

(3) $-COZ_2 + R_6OH \longrightarrow -COOR_6$

(wherein $COZ_2$ means an activated carbonyl group (e.g.) $-COCl$,

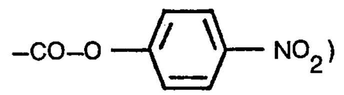

(4) $-COO^-M^+ + R_6-Z \longrightarrow -COOR_6$

(wherein $M^+$ is a metal cation and Z is as defined above)

(5) $-CN + R_6-OH + H_2O \xrightarrow{H^+} -COOR_6$

The reactions (1), (2), (3), (4) and (5) may be carried out in a usual manner. That is, the reaction (1) can be effected by heating the carboxy compound with the alcohol in the presence of an acid catalyst. The reaction (2) may be carried out by treating the carboxy compound with diazomethane at room temperature in an inert solvent such as ether or ethyl acetate. The reaction (3) may be effected by reacting the activated acid compound with the alcohol. In this reaction, some bases such as triethylamine, pyridine or dimethylaniline may preferably be used as a condensing agent. The reaction (3) can be effected by reacting the metal salt (M being, for instance, $Na^+$, $K^+$, $Mg^{++}$ or $Ag^+$) of the acid with the activated radical ($R_6$—Z). The reaction (5) can be effected by treatment of the cyano group with the alcohol and water in the presence of an acid catalyst.

The reaction (1), (3), (4) or (5) may be carried out in a suitable solvent such as methanol, ethanol, benzene, toluene, xylene, tetrahydrofuran or dimethylsulfoxide and the like at a temperature ranging from 0°C to the refluxing temperature of the reaction system, preferably above room temperature.

The heterotricyclic compound thus obtained can be separated from the reaction mixture and purified by the conventional procedures.

*Method H*

The compound of the formula (Is):

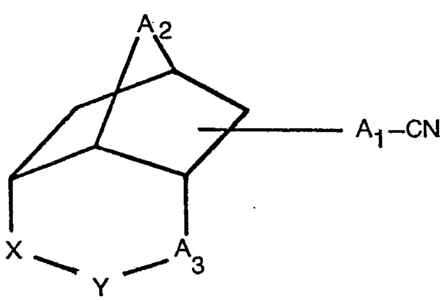

(Is)

wherein $A_1$, $A_2$, $A_3$ and X—Y are each as defined above, can be prepared by reacting the compound of the formula (Io):

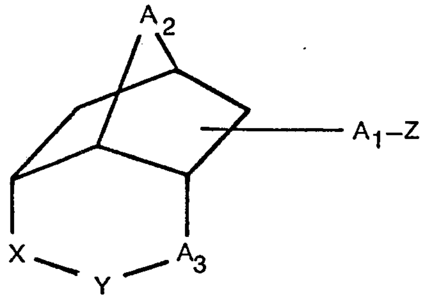

(Io)

wherein $A_1$, $A_2$, $A_3$, X—Y and Z are each as defined above, with a cyanide compound.

Examples of such cyanide compound are sodium cyanide or potassium cyanide. The reaction may preferably be carried out in an inert solvent such as methanol, ethanol, tetrahydrofuran, benzene, toluene, dimethylformamide or dimethylsulfoxide. The temperature may be from room temperature to the boiling point of the solvent to be used therein.

The heterotricyclic compound thus obtained can be separated from the reaction mixture and purified by the conventional procedures.

*Method I*

The compound of the formula (It):

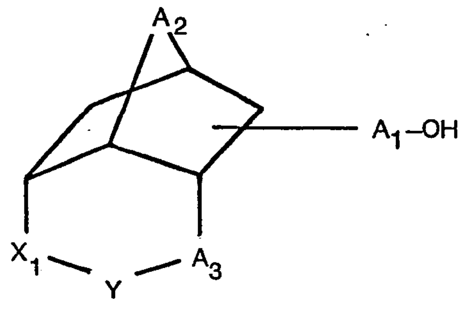

(It)

wherein $A_1$, $A_2$, $A_3$ and $X_1$—Y are each as defined above, can be prepared by formation of the hydroxy group on the tricyclic ring via the following procedure:

*a) via Reduction*

(1) 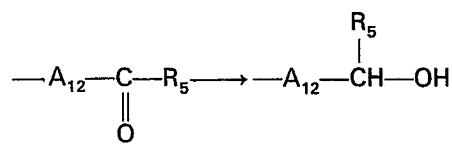

(2) $-A_{12}-COOR_6 \longrightarrow -A_{12}-CH_2OH$

(wherein $R_5$, $R_6$ and $A_{12}$ are each as defined above).

The reaction may preferably be carried out by treatment with a metal hydride compound such as lithium aluminum hydride, sodium trialkoxyaluminum hydride, sodium diethylaluminum hydride, sodium bis(methoxyethoxy)aluminum hydride, sodium borohydride or calcium borohydride. In the reaction (2), the compound of the formula (It) wherein Y is methylene is obtainable. The reaction is usually carried out in an inert solvent such as ether, tetrahydrofuran, dimethoxyethane, dioxane or toluene. The temperature may vary depending on the type of the substrate or the reducing agent to be used therein, but may be from −20°C to the boiling point of the solvent to be used therein.

*b) via Hydrolysis*

(1) 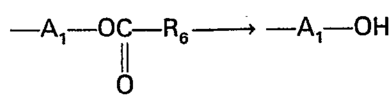

(2) $-A_1-Hal \longrightarrow -A_1-OH$

(wherein Hal means a halogen atom).

The reaction (1) or (2) may preferably be effected by treating the acyloxy compound or the halogen compound in an aqueous alkali medium such as aqueous sodium carbonate, aqueous potassium carbonate, aqueous sodium hydroxide, aqueous potassium hydroxide or aqueous barium hydroxide. In these reactions, a co-solvent such as methanol, ethanol, butanol, ethyleneglycol, dimethylformamide or dimethylsulfoxide may preferably be used in addition to the said aqueous medium. The reaction (1) or (2) can generally be carried out from room temperature to the refluxing temperature.

The heterotricyclic compound thus obtained can be separated from the reaction mixture and purified by the conventional procedures.

*Method J*

The compound of the formula (Iu):

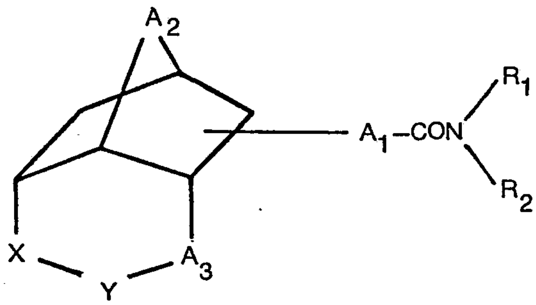

(Iu)

wherein $A_1$, $A_2$, $A_3$, X—Y, $R_1$ and $R_2$ are each as defined above, can be prepared by forming the carbamoyl group via the following procedure:

(1) 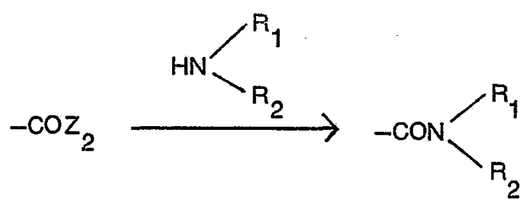

(2) 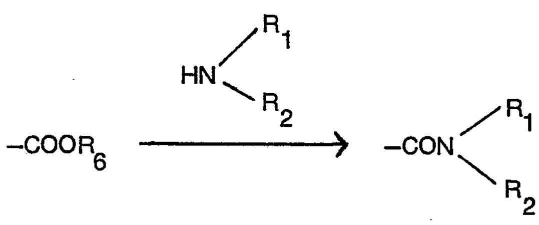

(wherein —$COZ_2$ and $R_6$ are each as defined above).

The reaction (1) may be carried out by reacting the activated acid derivative with the amine

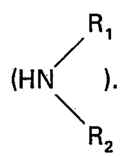

This reaction is preferably carried out by using the amine in an excess amount, preferably in the presence of a solvent such as methanol, ethanol, benzene or tetrahydrofuran.

The reaction (2) may be carried out by reacting the alkoxycarbonyl group with the amine, preferably in the presence of a solvent such as methanol, ethanol or tetrahydrofuran. These reactions may generally be conducted at a temperature from room temperature to the boiling point of the solvent used.

The heterotricyclic compound thus obtained can be separated from the reaction mixture and purified by the conventional procedures.

*Method K*

The compound of the formula (Iv):

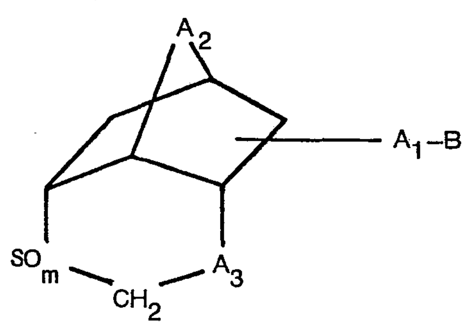

(Iv)

wherein $A_1$, $A_2$, $A_3$ and B are each as defined above and m is an integer of 1 or 2, can be prepared by reacting the compound of the formula (Iw):

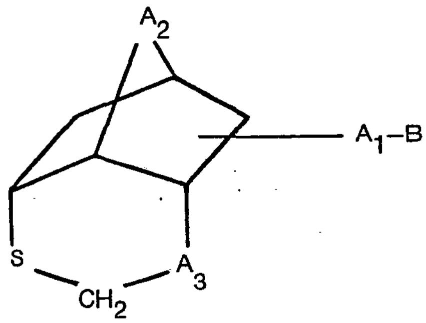

(Iw)

wherein $A_1$, $A_2$, $A_3$ and B are each as defined above, with an oxidizing agent.

Examples of the oxidizing agent are hydrogen peroxide, peracids (e.g. peracetic acid, perbenzoic acid, m-chloroperbenzoic acid), potassium permanganate, potassium persulfate, sodium hypochlorite, sodium metaperiodate. The reaction may preferably be carried out in a solvent such as water, acetic acid, chloroform, dichloroethane, methanol, ethanol or benzene.

The oxidation may proceed in two steps: oxidation of the sulfide into the sulfoxide and that of the sulfoxide into the sulfone. The first step may proceed under milder conditions, e.g. at a low temperature (—50°C to room temperature) or with a low molar ratio of the oxidizing agent toward the substrate. Thus, the objective sulfoxide can substantially be obtained as a sole reaction product.

The oxidation of the sulfide or the sulfoxide into the sulfone may preferably be effected by using an excess amount of the oxidizing agent under a relatively high temperature, i.e. from room temperature to the boiling point of the solvent to be used.

The heterotricyclic compound thus obtained can be separated from the reaction mixture and purified by the conventional procedures.

PREPARATION OF STARTING MATERIALS

*Method L*

The compound of the formula (IIb) (i.e. the starting compound for Method A):

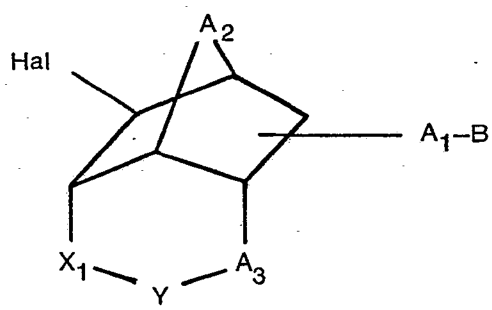

(IIb)

wherein $A_1$, $A_2$, $A_3$, $X_1$—Y, B and Hal are each as defined above, can be prepared by reacting the compound of the formula (IIa):

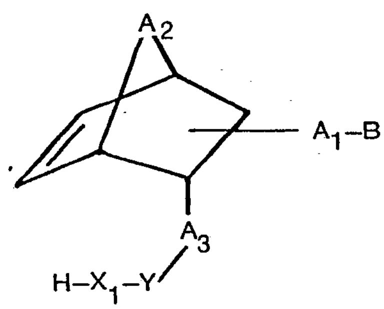

(IIa)

wherein $A_1$, $A_2$, $A_3$, $X_1$—Y and B are each as defined above, with a halogen compound.

Examples of the halogen compound are bromine, iodine, or N-bromosuccinic imide (NBS). The reaction may preferably be carried out in an inert solvent such as water, carbon tetrachloride, chloroform, dichloroethane or tetrahydrofuran. The reaction proceeds generally at a temperature from 0°C to room temperature.

When Hal is iodine and $X_1$—Y is —O—CO—, the iodolactonization procedure, e.g. iodine-

potassium iodide and bicarbonate in an aqueous medium (J. Am. Chem. Soc., *76*, 2315 (1954)), may preferably be adopted.

*Method M*

The compound of the formula (IIc) (i.e. the starting compound in Method C):

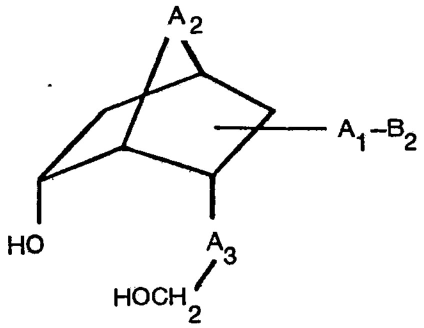

(IIc)

wherein $A_1$, $A_2$, $A_3$ and $B_2$ are each as defined above, can be prepared by reducing the compound of the formula (Ix):

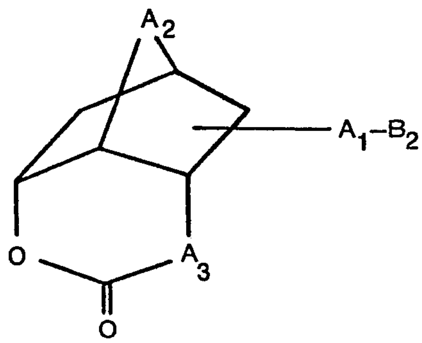

(Ix)

wherein $A_1$, $A_2$, $A_3$ and $B_2$ are each as defined above, with a metal hydride compound.

A variety of metal hydride compounds which have a reducing capability toward a lactone moiety can be widely used (e.g. lithium aluminum hydride, sodium trialkoxy-aluminum hydride, sodium diethylaluminum hydride, calcium borohydride or lithium borohydride). The metal hydride compound to be used therein may preferably be selected depending on the type of the side chain group ($B_1$) involved in the molecule of the compound (Ix). That is, when $B_1$ is susceptible toward a strong reducing agent such as lithium aluminum hydride, a milder reducing agent such as calcium borohydride or lithium borohydride may preferably be used. The reaction is usually effected in an inert solvent such as ether, tetrahydrofuran, toluene or dimethoxyethane. The reaction temperature may vary depending on the type of the substrate or the reducing agent to be used therein, but may mostly be from −20°C to the boiling point of the solvent to be used.

*Method N*

The compound of the formula (IIe) (i.e. the starting compound in Methods E, H and I):

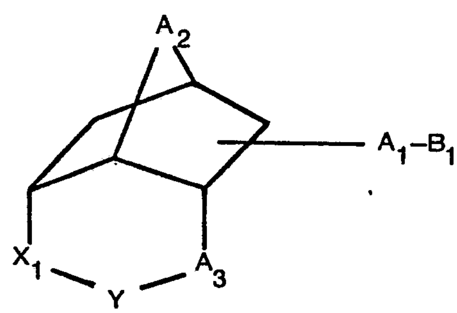

(IIe)

wherein $A_1$, $A_2$, $A_3$ and $X_1$—Y are each as defined above and $B_4$ is Z (wherein Z is as defined above), a carbonyl group of the formula:

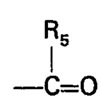

(wherein $R_5$ is as defined above), an oxime group of the formula:

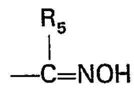

or a phthalimide group of the formula:

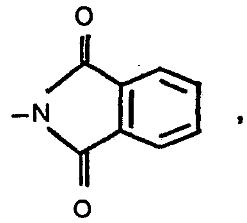,

can be prepared by formation of the said group ($B_4$) from the aforesaid group (B) according to conventional procedures.

Typical examples are illustrated as follows:

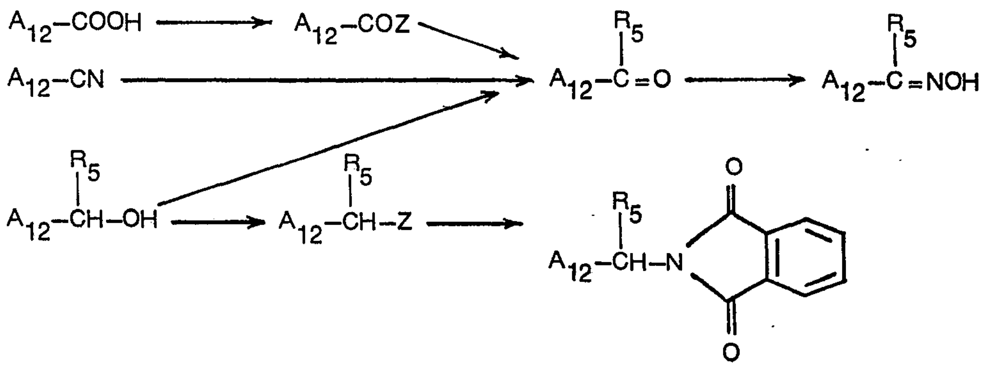

(wherein $A_{12}$, $R_5$ and Z are each as defined above).

*Method O*

The compound of the formula (IIa) (i.e. the common intermediate for the synthesis of the compound (I) of the present invention):

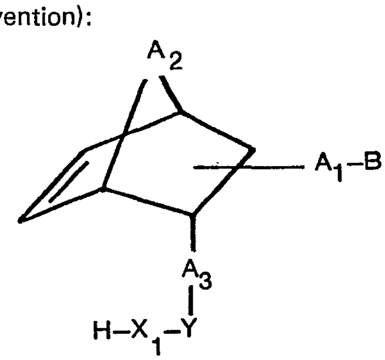

(IIa)

wherein $A_1$, $A_2$, $A_3$, $X_1$—Y and B are each as defined above, can be prepared from the Diels-Alder reaction adducts by appropriate functional modifications.

[Step 1] The Diels-Alder reaction:

The first step for the synthesis of the compound (IIa) is the Diels-Alder reaction using an alicyclic diene compound of the formula (IIIa):

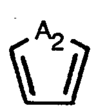

(IIIa)

wherein $A_2$ is as defined above, with a dienophile of the formula (IIIb) or (IIIc):

(IIIb) W—CH=CH—$A_4$—V

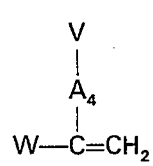

(IIIc)

wherein W is a carboxy group, an alkoxycarbonyl group, a cyano group, a formyl group, an acyl group or a carbamoyl group, V is a carboxy group, an alkoxycarbonyl group, a cyano group, a formyl group, an acyl group, a halogen atom or a hydroxyl group and $A_4$ is a single bond or a $C_1$—$C_5$ alkylene group.

The products (the diene adducts (IIf) and (IIg)) obtained by this reaction are shown as follows:

(IIf) 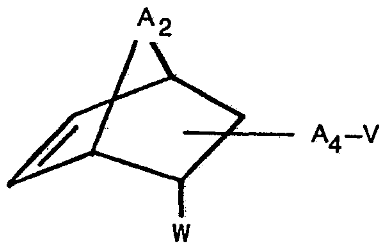

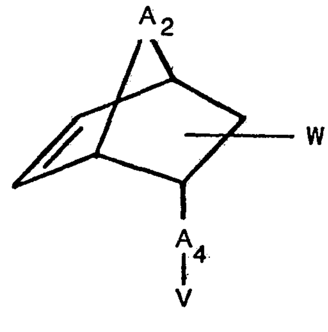 (IIg)

wherein $A_2$, $A_4$, W and V are each as defined above.

The diene (IIIa) to be used is such an alicyclic diene compound as cyclopentadiene, cyclohexadiene or cycloheptatriene.

The diene adducts (IIf) and (IIg) can be separated from each other by a conventional procedure such as chromatography or recrystallization. Either (IIf) or (IIg) can be utilized as an intermediate for further reactions.

The following diene adducts (1), (2), (3), (4) and (5), some of which are already well known, can also be utilized as the intermediates for the synthesis of the compound (IIa):

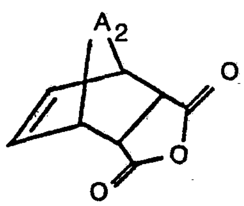

(1)

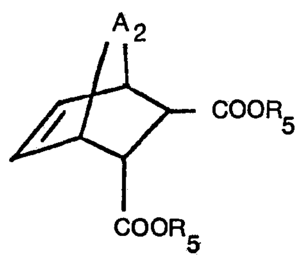

(2)

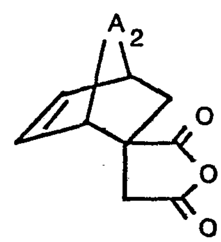

(3)

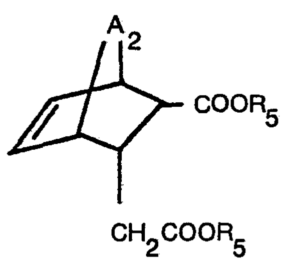

(4)

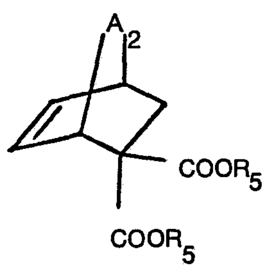

(5)

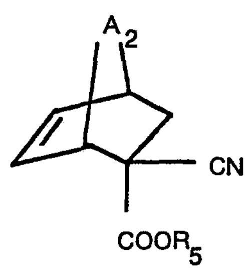

(6)

wherein $R_5$ is as defined above.

[Step 2] Functional modification

The second step for the synthesis of the compound (IIa) is the transformation of the group with the endo-orientation of the diene adduct obtained above into the group ($-A_3-Y-X_1-H$) of the compound (IIa):

the group ($-W$) of (IIf)

the group ($-A_3-Y-X_1-H$) of (IIa)

the group ($-A_4-V$) of (IIg)

The transformation of the other group of the diene adduct into the group ($-A_1-B$) of the

compound (IIa) may be carried out before, after or during the aforesaid transformation of the endo-group.

the group ($-A_4-V$) of (IIf) ↘

the group ($-A_1-B$) of (IIa)

the group ($-W$) of (IIg) ↗

Some of the typical transformation starting from the diene adduct into the objective compound (I) of the present invention are illustrated in Charts A, B, C and D.

CHART A

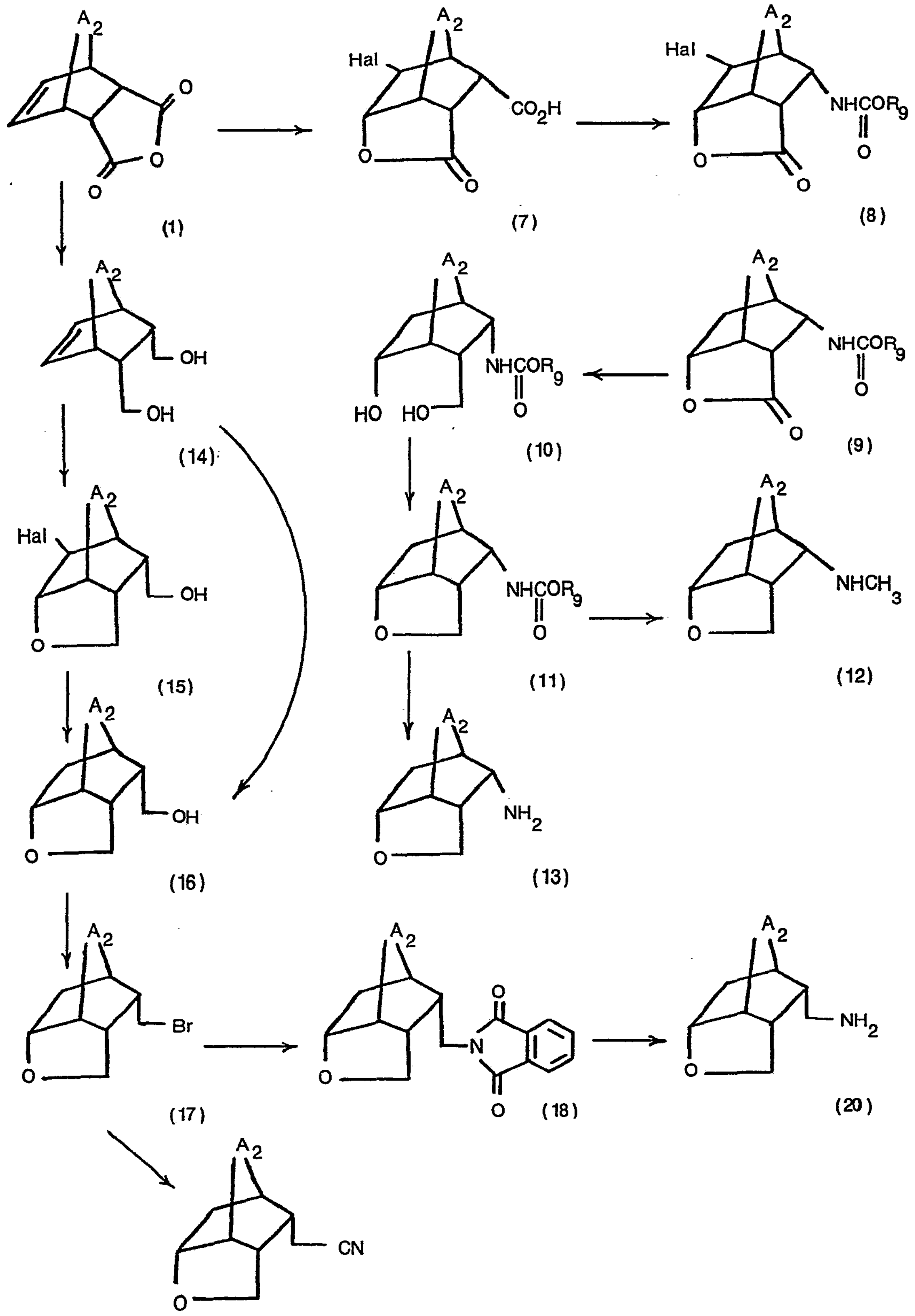

(1) (7) (8) (14) (10) (9) (15) (11) (12) (16) (13) (17) (18) (20) (19)

wherein Hal, $A_2$ and $R_9$ are each as defined above.

The compound (7) can be obtained from the compound (1) by hydrolysis and iodo-lactonization (Preparative example 3).

The acid azide of the compound (7), which can be obtained by reacting the acid chloride or mixed-anhydride of the compound (7) with sodium azide, can be transformed to the compound (8) with an alcohol under heating (Preparative example 4 and example 1).

Dehalogenation of the compound (8) with tributyltin hydride gives the lactone (9) (example 3).

Reduction of the compound (9) with $LiBH_4$ or $Ca(BH_4)_2$ gives the diol compound (10) (Preparative example 6).

Dehydration of the compound (10) with p-tosyl chloride and pyridine gives the ether (11) (Example 5).

Hydrolysis of the compound (11) gives the amine compound (13) and reduction of the compound (11) with $LiAlH_4$ gives the compound (12) (Examples 6 and 12).

Reduction of the compound (1) with $LiAlH_4$ gives the diol compound (14) (Example 15).

Haloetherization of the compound (14) with NBS gives the ether (15) (Preparative example 9).

Dehalogenation of the compound (15) with tributyltin hydride gives the ether (16) (Example 4).

Oxymercuration-demercuration of the compound (14) with mercuric acetate and sodium borohydride gives the ether (16) (Example 10).

Tosylation and halogenation of the compound (16) with tosyl chloride-pyridine and $LiBr.H_2O$ give the bromide (17) (Preparative example 11 and example 16).

Phthalimidation of the compound (17) with potassium phthalimide gives the compound (18) (Preparative example 12).

Hydrolysis of the compound (18) gives the amine compound (20) (Example 17).

Cyanation of the compound (17) with potassium cyanide gives the nitrile compound (19) (Example 40).

CHART B

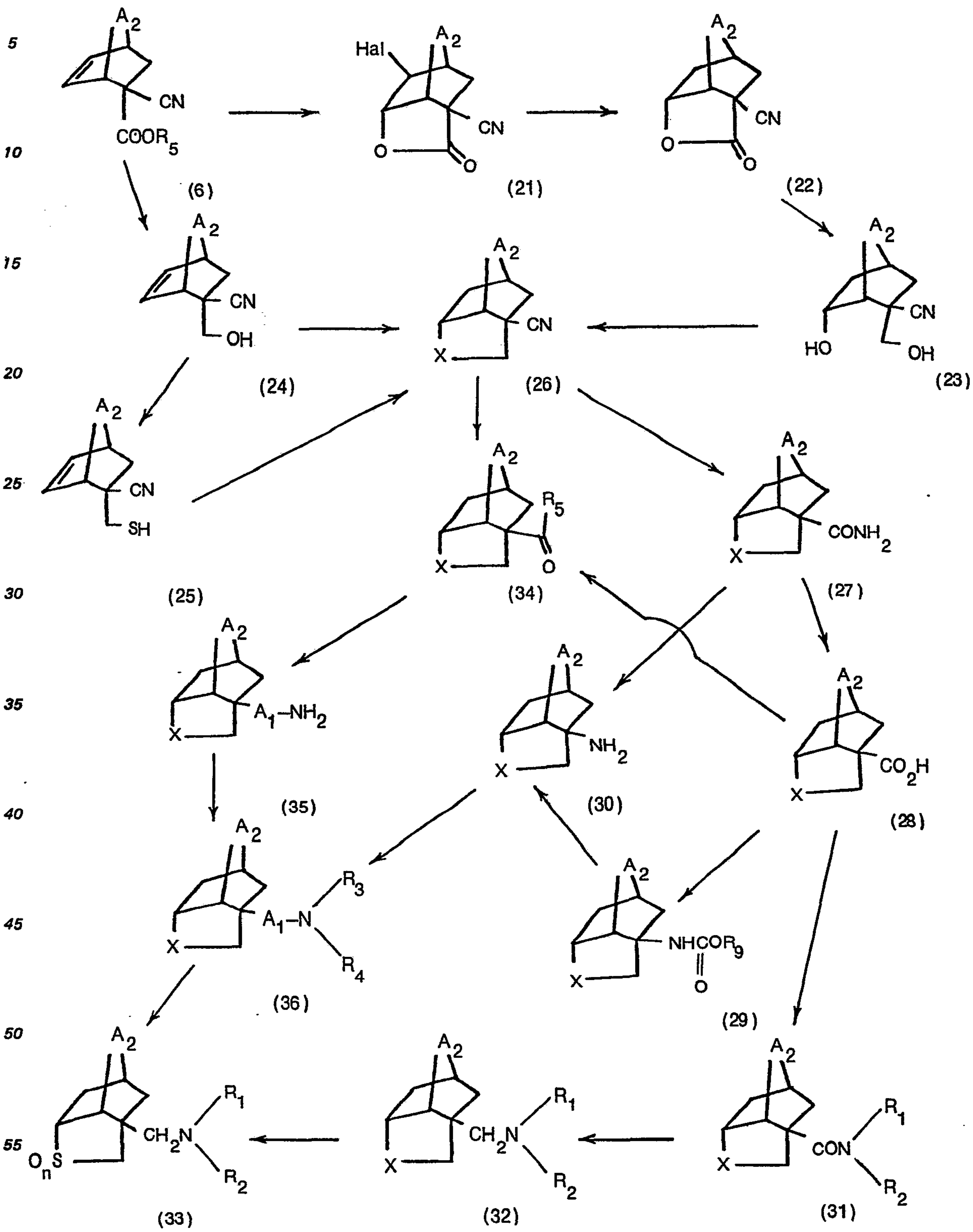

(6) (21) (22) (24) (26) (23) (25) (34) (27) (35) (30) (28) (36) (29) (33) (32) (31)

wherein $A_2$, Hal, $R_1$, $R_2$, $R_3$, $R_4$, $R_5$, $R_9$, X and n are each as defined above.

The compound (6), which can be obtained from a 1,3-diene compound and $\alpha$-cyanoarylate, can be hydrolyzed and iodo-lactonized to afford the compound (21) (Preparative Example 3).

The transformation of the compound (21) to the compound (26) (X=O) can be achieved by the

similar procedure to the one as described in Chart A (cf. the transformation from the compound (9) to the compound (11)).

The compound (26) (X=O) can also be obtained from the compound (24) by the oxymercuration-demercuration proceudre.

The compound (25) can be prepared from the compound (24) by halogenation of the hydroxyl group and thiolation of the resulting product with sodium hydrosulfite.

The thiolation can also be carried out by thioacetylation with sodium thioacetate, followed by hydrolysis.

The compound (26) (X=S) can be prepared from the compound (25) by cyclization with a base catalyst (e.g. sodium hydroxide, triethylamine).

Furthermore, cyclization can also be effected by thio-etherization with bromine, followed by debromination with a reducing agent (e.g. tributyltin hydride).

Partial hydrolysis of the compound (26) gives the compound (27) (Example 14).

The compound (30) can be prepared from the compound (27) by Hofmann rearrangement or by a sequence of hydrolysis, Curtius reaction and hydrolysis [(27)→(28)→(29)→(30)] (Examples 2, 6 and 8).

The compound (35) can be prepared from the compound (26) by a sequence of alkylation with Grignard reagent or alkyl lithium, oximation and reduction (Preparation Example 19 and Example 32).

The compound (34) ($R_5$=H) can be prepared from the compound (28) by a sequence of reduction of the carboxyl group and oxidation of the resulting hydroxyl group (Examples 15 and Preparative Examples 10 and 11).

The compound (35) can be prepared from the compound (34) by a sequence of the Wittig reaction with triphenyl cyanomethylene phosphorane, hydrogenation of the resulting double bond and reduction of the cyano group (Examples 19 and 25, Preparative Example 15).

The compound (36) can be prepared from the compound (30) or (35) by a sequence of alkylation (Examples 12, 13, 20, 29, 30, 31 and 34).

The compound (33) can be prepared from the compound (28) by a sequence of amidation, reduction and oxidation [(28)→(31)→(32)→(33)] (Examples 20, 37 and 38).

CHART C

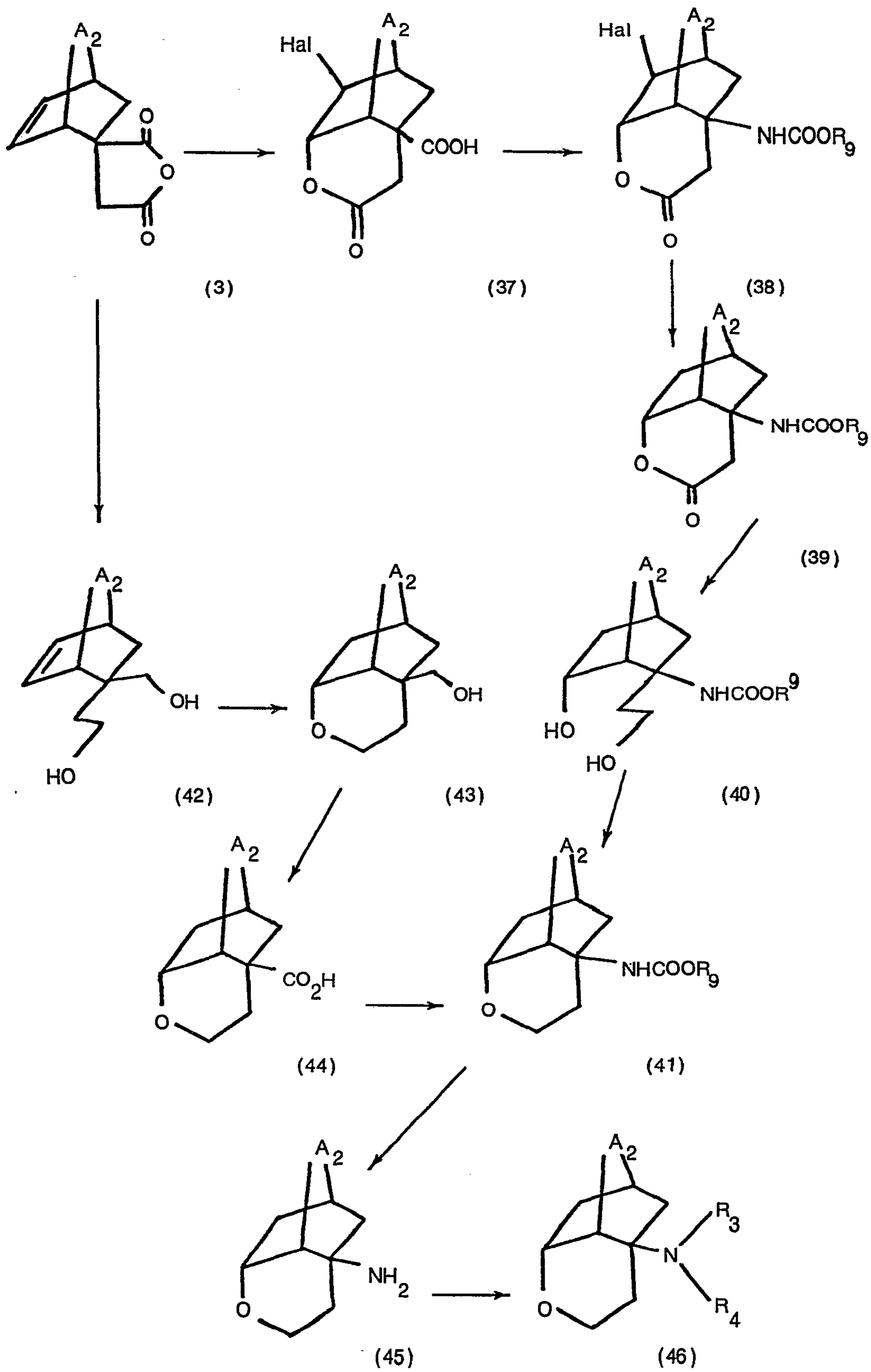

(3) (37) (38) (39) (42) (43) (40) (44) (41) (45) (46)

wherein $A_2$, Hal, $R_3$, $R_4$ and $R_9$ are each as defined above.

The compound (3), which can be obtained from a 1,3-diene compound and itaconic acid anhydride, can be hydrolyzed and iodo-lactonized to afford the compound (37).

The transformation of the compound (37) to the compound (41) can be achieved by the similar procedure to the one as described in Chart A (cf. the transformation from the compound (7) to the compound (11)) (Preparative Examples 3, 4, 6; Examples 3 and 5).

The compound (42) can be obtained from the compound (3) by reducing with $LiAlH_4$ (Preparative Example 10; Example 15).

The compound (41) can be prepared from the compound (42) by a sequence of oxymercuration-demercuration, oxidation and Curtius reaction (Examples 2, 11 and 22 and Preparative Example 5).

The transformation of the compound (41) to the compound (46) can be achieved by a similar procedure to the one as described in Chart B (cf. the transformation from the compound (35) to the compound (36)).

CHART D

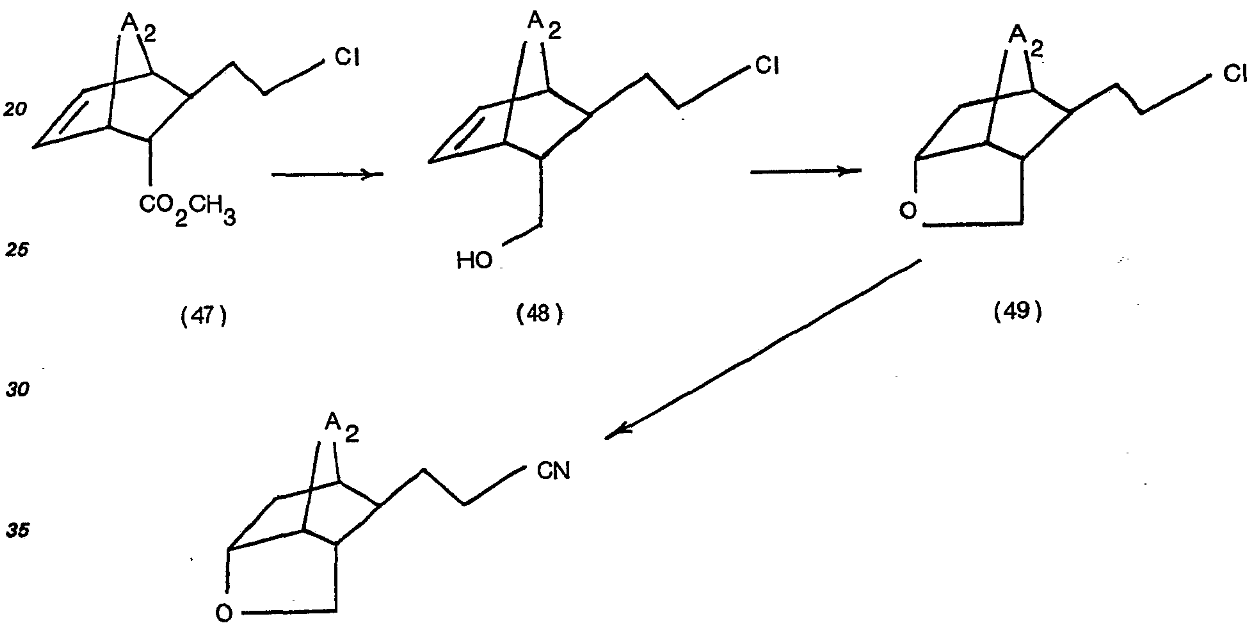

(47) (48) (49)

(50)

wherein $A_2$ is as defined above.

The compound (47), which can be obtained from a 1,3-diene compound and methyl-2-(β-chloroethyl)acrylate can be reduced, oxymercurated and demercurated to afford the compound (49) (Examples 9 and 10, Preparative Example 10).

Cyanation of the compound (49) with potassium cyanide gives the cyano compound (50) (Example 40).

Some of the skeletons of the compounds obtained by the present invention are designated according to the following common names, and the others are based on the nomenclature of IUPAC.

(a) Brendane

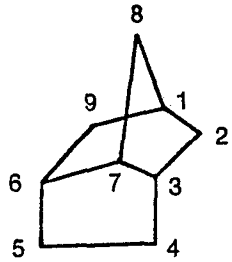

(b) Homobrendane

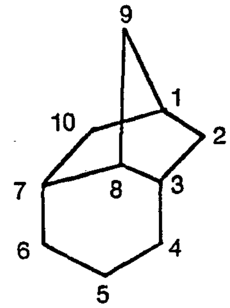

(c) Isotwistane

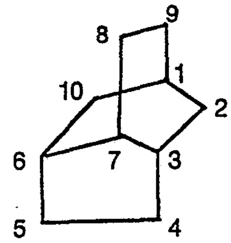

(d) Homoisotwistane

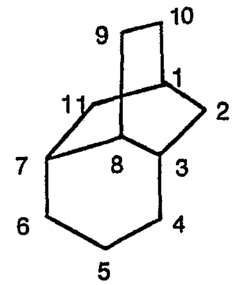

The following Preparative Examples (starting materials) and examples are given for the purpose of illustration (see also chart E—M).

## PREPARATIVE EXAMPLES

### Preparative Example 1

Into a solution of diethyl methylene malonate (21.5 g) in dry benzene (200 ml), cyclopentadiene (9.8 g) was added at an ambient temperature. The reaction solution was stirred at the same temperature for 1 hour. After evaporation of the solvent, distillation of the residue gave 11.8 g of 2,2-bisethoxycarbonyl-5-norbornene. (Compound No. P—1—1) B.P., 85—90°C/0.2 mmHg. IR$\nu_{max}^{film}$ ($cm^{-1}$): 2970, 1740, 1460.

According to the same procedure, using diethylmethylene malonate (47.3 g) and 1,3-cyclohexadiene (20 g) but benzene refluxed, there was obtained oily 2,2-bisethoxy-carbonyl-bicyclo[2,2,2]oct-5-ene (65.0 g). (Compound No. P—1—2) IR$\nu_{max}^{film}$ ($cm^{-1}$): 2970, 1740, 1450.

According to the same procedure, using itaconic acid anhydride (8.3 g) and 1,3-cyclohexadiene (10 g) but benzene refluxed, there was obtained as a solid exo-2-carboxy-endo-2-carboxymethyl-anhydride-bicyclo[2,2,2]oct-5-ene (10.5 g). (Compound No. P—1—3) M.P., 70—85°C (recrystallization from hexane). IR$\nu_{max}^{KBr}$ ($cm^{-1}$): 2950, 1850, 1780, 1240, 1030.

According to the same procedure, using methyl-$\alpha$-cyanoacrylate (35 g) and cyclopentadiene (20 g), there was obtained as an oil exo-2-cyano-endo-2-methoxycarbonyl-5-norbornene (56 g). (Compound No. P—1—4) (This substance contained 10% isomer of exo-2-methoxycarbonyl-endo-2-cyano-5-norbornene). IR$\nu_{max}^{film}$ ($cm^{-1}$): 2960, 2230, 1750, 1320, 1250.

According to the same procedure, using methyl-$\alpha$-cyanoacrylate (20 g) and 1,3-cyclohexadiene (14.4 g), there was obtained as an oil exo-2-cyano-endo-2-methoxycarbonyl-bicyclo[2,2,2]oct-5-ene (33 g) (Compound No. P—1—5). (This material contained 20% isomer of exo-2-methoxycarbonyl-endo-2-cyano-bicyclo[2,2,2]oct-5-ene). IR$\nu_{max}^{film}$ ($cm^{-1}$): 2950, 2250, 1750, 1440, 1270, 1260, 1080.

### Preparative Example 2

A mixture of methanol (12 ml), 10% aqueous sodium hydroxide (12 ml), tetrahydrofuran (12 ml) and 2,2-bisethoxy-carbonyl-5-norbornene (2.38 g) was stirred at room temperature for 24 hours and concentrated under reduced pressure. The residue was diluted with water, made acidic with 10% hydrochloric acid (HCl) solution and extracted with ether. The extract was washed with water and dried.

Removal of the solvent gave as an oil 2,2-dicarboxy-5-norbornene (Compound No. P—2—1) (1.63 g). IR$\nu_{max}^{film}$ ($cm^{-1}$): 2990, 1710, 1340.

According to the same procedure, there was obtained the following compounds.

2,2-Dicarboxy-bicyclo[2,2,2]oct-5-ene (Compound No. P—2—2), oily substance. IR$\nu_{max}^{film}$ ($cm^{-1}$): 2990, 1710, 1350;

Exo-2-cyano-endo-2-carboxy-5-norbornene (Compound No. P—2—3), oily substand IR$\nu_{max}^{film}$ ($cm^{-1}$): 2980, 2600, 2240, 1720;

Exo-2-cyano-endo-2-carboxy-bicyclo[2,2,2]oct-5-ene (Compound No. P—2—4), solid substance, M.P. 129—130°C. IR$\nu_{max}^{KBr}$ ($cm^{-1}$): 2980, 2230, 1700.

### Preparative Example 3

2,2-Dicarboxy-5-norbornene (1.63 g) was dissolved with warming in a solution of 0.5 N sodium bicarbonate (60 ml). After cooling to room temperature, a solution of iodine ($I_2$, 2.54 g) and potassium iodide (KI, 5.08 g) in 30 ml of water was added, and the mixture kept in the dark for 24 hours. It was then filtered, the filtrate was acidified with dilute HCl and treated with sodium thiosulfate until iodine color disappeared. It was extracted with ether, and the extract was washed with saturated sodium chloride (NaCl) solution and dried.

Removal of the solvent gave a solid which was recrystallized from benzene to yield 1.8 g of exo-9-iodo-5-oxa-4-oxo-3-carboxy-brendane (Compound No. P—3—1), M.P., 171—172°C. IR$\nu_{max}^{KBr}$ ($cm^{-1}$): 2950, 1780, 1740, 1480;

According to the same procedure, there were obtained the following compounds:

Exo-10-iodo-5-oxa-4-oxo-3-carboxy-isotwistane (Compound No. P—3—2), solid substance, M.P., 198—200°C (recrystallization from benzene). IR$\nu_{max}^{KBr}$ (cm$^{-1}$): 2950, 1780, 1710, 1380;

Exo-9-iodo-5-oxa-4-oxo-3-cyano-brendane (Compound No. P—3—3), solid substance, M.P., 192—193.5°C (recrystallization from benzene). IR$\nu_{max}^{Nujol}$ (cm$^{-1}$): 2250, 1780, 1100;

Exo-10-iodo-5-oxa-4-oxo-3-cyano-isotwistane (Compound No. P—3—4), solid substance, M.P., 178—179°C (recrystallization from benzene-ether). IR$\nu_{max}^{Nujol}$ (cm$^{-1}$): 2250, 1790.

Exo-11-iodo-6-oxa-5-oxo-3-carboxy-homoisotwistane (Compound No. P—3—5), solid substance, M.P., 205—210°C (recrystallization from ether). IR$\nu_{max}^{film}$ (cm$^{-1}$): 1720, 1340, 1300.

Exo-10-iodo-5-oxa-4-oxo-endo-2-carboxy-isotwistane (Compound No. P—3—6), solid substance, M.P., 191—193°C IR$\nu_{max}^{Nujol}$ (cm$^{-1}$): 1790, 1780, 1730, 1710.

## Preparative Example 4

A mixture of exo-9-iodo-5-oxa-4-oxo-3-carboxy-brendane (1.0 g) and thionly chloride (20 ml) was refluxed for 3 hours. The reaction mixture was evaporated to dryness, benzene (20 ml) was added thereto and evaporation was effected to remove thionyl chloride. Into a solution of thus obtained acid chloride in acetone (20 ml) was added sodium azide ($NaN_3$, 0.232 g) under cooling. The reaction solution was stirred for 24 hours at an ambient temperature. After evaporation of the solvent, the residue was dissolved in dry ethanol (50 ml) and refluxed for 2 hours. After evaporation of the solvent, the residue was extrcted with chloroform. The extract was washed with water and dried. Evaporation of the solvent gave a solid, which was recrystallized from benzene to yield 0.85 g of exo-9-iodo-5-oxa-4-oxo-3-ethoxycarbonylamino-brendane (Compound No. P—4—1), M.P., 165—167°C IR$\nu_{max}^{KBr}$ (cm$^{-1}$): 3320, 2990, 1800, 1690. According to an analogous procedure there were obtained the following compounds:

Exo-10-iodo-5-oxo-4-oxo-3-ethoxycarbonylaminoisotwistane (Compound No. P—4—2), solid substance. IR$\nu_{max}^{Nujol}$ (cm$^{-1}$): 3350, 1800, 1730—1700, 1540—1510;

Exo-10-iodo-5-oxa-4-oxo-endo-2-ethoxycarbonylamino-isotwistane (Compound No. P—4—3). solid substance, M.P., 158—159°C (recrystallization from ethyl acetate-n-hexane). IR$\nu_{max}^{Nujol}$ (cm$^{-1}$): 3300, 1800, 1690, 1550;

Exo-11-iodo-6-oxa-5-oxo-3-ethoxycarbonylaminohomoisotwistane (Compound No. P—4—4) (18.4 g) M.P., 139—140°C. IR$\nu_{max}^{Nujol}$ (cm$^{-1}$): 3300, 1720, 1530.

Exo-10-iodo-6-oxa-5-oxo-3-ethoxycarbonylaminohomobrendane (Compound No. P—4—5), solid substance, M.P., 112—114.5°C IR$\nu_{max}^{Nujol}$ (cm$^{-1}$): 3350, 1730—1700, 1450.

## Preparative Example 5

According to the Example 2 procedure, there were obtained the following compounds.

Endo-2-methoxycarbonyl-endo-3-ethoxycarbonylamino-5-norbornene (Compound No. P—5—1), oily substance. IR$\nu_{max}^{film}$ (cm$^{-1}$): 3400, 3000, 1720, 1520;

Endo-2-methoxycarbonyl-endo-3-ethoxycarbonylamino-bicyclo[2,2,2]oct-5-ene (Compound No. P—5—2), solid substance, M.P., 63—64°C. IR$\nu_{max}^{Nujol}$ (cm$^{-1}$): 3370, 1730, 1710, 1520;

Endo-8-ethoxycarbonylamino-endo-9-ethoxycarbonyl-tricyclo[3,2,2,2,$0^{2,4}$]non-6-ene (Compound No. P—5—3), oily substance. IR$\nu_{max}^{Nujol}$ (cm$^{-1}$): 3430, 1730, 1510, 1170.

## Preparative Example 6

An ethanol solution containing 5-oxa-4-oxo-3-ethoxy-carbonylamino-brendane (1.0 g) was added to calcium borohydride prepared from dry calcium chloride (1.46 g) and sodium borohydride (0.671 g) in dry ethanol, while stirring and cooling at 2—5°C. Stirring was continued at 5°C for 5 hours. After consumption of excess calcium borohydride by addition of a saturated $NH_4Cl$ solution, the mixture was concentrated to give a syrupy residue, which was extracted with chloroform. The extract was washed with saturated NaCL, dried and concentrated to give as an oily substance endo-5-hydroxy-endo-3-hydroxymethyl-exo-3-ethoxycarbonylamino-norobornane (Compound No. P—6—1) (0.9 g). IR$\nu_{max}^{film}$ (cm$^{-1}$): 3400, 3350, 1680, 1540.

According to the same procedure, there were obtained the following compounds:

Endo - 5 - hydroxy - endo - 3 - hydroxymethyl - endo - 2 - ethoxycarbonylamino - norbornane (Compound No. P—6—2), solid substance. IR$\nu_{max}^{Nujol}$ (cm$^{-1}$): 3430, 3350, 3250, 1700, 1520;

Endo-5-hydroxy-endo-3-hydroxymethyl-endo-2-ethoxycarbonylamino-bicyclo[2,2,2]octane (Compound No. P—6—3), oily substance. IR$\nu_{max}^{film}$ (cm$^{-1}$): 3600—3100, 1700, 1520;

Endo-5-hydroxy-endo-3-hydroxymethyl-endo-2-ethoxycarbonylamino-bicyclo[2,2,2]octane (Compound No. P—6—4), solid substance. IR$\nu_{max}^{Nujol}$ (cm$^{-1}$): 3600, 3100, 1720, 1700, 1520;

Endo-5-hydroxy-endo-3-$\beta$-hydroxyethyl-exo-3-ethoxy-carbonylamino-norbornane (Compound No. P—6—5), solid substance. IR$\nu_{max}^{Nujol}$ (cm$^{-1}$): 3330, 1700, 1470, 1070;

Endo-5-hydroxy-endo-3-$\beta$-hydroxyethyl-exo-3-ethoxy-carbonylamino-bicyclo[2,2,2]octane (Compound No. P—6—6), solid substance. IR$\nu_{max}^{Nujol}$ (cm$^{-1}$): 3350, 1700, 1470;

Endo-5-hydroxy-endo-3-hydroxymethyl-endo-2-cyanomethyl-norbornane (Compound No. P—6—7), oily substance. IR$\nu_{max}^{film}$ (cm$^{-1}$): 3350, 2250, 1460, 1430, 1140, 1120, 1060.

Preparative Example 7

An ethanol solution (300 ml) containing 5-oxa-4-oxo-3-cyano-brendane (9.8 g) was added to calcium borohydride prepared from dry calcium chloride (20 g) and sodium borohydride (9.12 g) in dry ethanol, while stirring and cooling at 2—5°C. Stirring was continued at room temperature for 5 hours. After consumption of excess calcium borohydride by addition of 10% HCl solution, the mixture was concentrated to give a syrupy residue. The resulting oily layer was extracted with chloroform. The extract was washed with saturated NaCl, dried and concentrated to give as an oily substance endo-5-hydroxy-endo-3-hydroxymethyl-exo-3-cyano-norbornane (Compound No. P—7—1) (9.5 g), M.P., 123—127°C. IR$\nu_{max}^{Nujol}$ (cm$^{-1}$): 3300, 2230, 1110.

According to the same procedure, there was obtained the following compound:

Endo-5-hydroxy-endo-3-hydroxymethyl-endo-2-N,N-diethylcarbamoyl-norbornane (Compound No. P—7—2), oily substance. IR$\nu_{max}^{film}$ (cm$^{-1}$): 3300, 2960, 1620, 1460.

Preparative Example 8

An ethanol solution containing endo-3-ethoxy-carbonylamino-endo-2-methoxycarbonyl-5-norbornene (1.8 g) was added to calcium borohydride prepared from dry calcium chloride (3.2 g) and sodium borohydride (1.4 g) in dry ethanol, while stirring and cooling at 2—5°C. Stirring was continued at room temperature for 5 hours. After consumption of excess calcium borohydride by addition of a saturated $NH_4Cl$ solution, the mixture was concentrated to give a syrupy residue, which was extracted with chloroform. The extract was washed with saturated NaCl, dried and concentrated to give as an oily substance endo-3-ethoxy-carbonylamino-endo-2-hydroxymethyl-5-norobornene (Compound No. P—8—1) (1.5 g) IR$\nu_{max}^{film}$ (cm$^{-1}$): 3500, 2870, 1720, 1520.

According to the same procedure, there were obtained the following compounds:

Exo-2-cyano-endo-2-hydroxymethyl-5-norbornene (Compound No. P—8—2), oily substance. IR$\nu_{max}^{film}$ (cm$^{-1}$): 3400, 2230, 1650, 1480, 1440, 1280;

Exo-2-cyano-endo-2-hydroxymethyl-bicyclo[2,2,2]oct-5-ene (Compound No. P—8—4), oily substance. IR$\nu_{max}^{film}$ (cm$^{-1}$): 3400, 2240, 1470, 1460, 1380;

Endo-3-ethoxycarbonylamino-endo-2-hydroxymethyl-bicyclo[2,2,2]oct-5-ene (Compound No. P—8—4), oily substance. IR$\nu_{max}^{film}$ (cm$^{-1}$): 3500, 2950, 1710, 1520;

Endo-3-N,N-diethylcarbamoyl-endo-2-hydroxymethyl-5-norbornene (Compound No. P—8—5), oily substance. IR$\nu_{max}^{film}$ (cm$^{-1}$): 3400, 2950, 1630, 1270;

Endo-8-ethoxycarbonylamino-endo-9-hydroxymethyl-tricyclo[3,2,2,0$^{2,4}$]non-6-ene (Compound No. P—8—6), oily substance. IR$\nu_{max}^{film}$ (cm$^{-1}$): 3430, 3350, 1700, 1510;

Endo-3-hydroxymethyl-exo-2-$\beta$-chloroethyl-5-norbornene (Compound No. P—8—7), oily substance. IR$\nu_{max}^{film}$ (cm$^{-1}$): 3400, 2950, 1450, 1340, 1060.

Preparative Example 9

To a mixture of dry chloroform (93 ml), dry tetrahydrofuran (62 ml) and 2,2-bishydroxymethyl-5-norbornene 5.5 g) was added N-bromosuccinimide (NBS, 7.0 g) at room temperature with stirring for 1 hour. After consumption of excess NBS by addition of 10% sodium thiosulfate solution, the reaction mixture was concentrated.The resulting oily layer was extracted with chloroform, and the chloroform layer was washed with a saturated NaCl solution, dried and concentrated to give as a solid substance exo-9-bromo-5-oxa-3-hydroxymethyl-brendane (Compound No. P—9—1) (7.19 g), M.P., 72—73°C (recrystallization from ether-n-hexane). IR$\nu_{max}^{Nujol}$ (cm$^{-1}$): 3450, 1310, 1280.

According to the same procedure, there were obtained the following compounds:

Exo-9-bromo-5-oxa-endo-2-hydroxymethyl-brendane (Compound No. P—9—2), oily substance. IR$\nu_{max}^{film}$ (cm$^{-1}$): 3450, 1460, 1210, 1100;

Exo-9-bromo-5-oxa-3-cyano-brendane (Compound No. P—9—4), solid substance, M.P., 45—46.5°C. IR$\nu_{max}^{Nujol}$ (cm$^{-1}$): 2230, 1290, 1270, 1200, 1160;

Exo-10-bromo-5-oxa-3-cyano-isotwistase (Compound No. P—9—5), solid substance, M.P., 108.5—109°C. IR$\nu_{max}^{Nujol}$ (cm$^{-1}$): 2250, 1020.

Preparative Example 10

Into a solution containing 2,2-bisethoxycarbonyl-5-norbornene (10 g) in dry tetrahydrofuran (100 ml) was added a mixture of lithium aluminium hydride (4.3 g) and dry tetrahydrofuran (100 ml) with stirring at room temperature. The reaction mixture was refluxed for 2 hours. After consumption of excess $LiAlH_4$ by addition of water, the reaction mixture was concentrated to give a residue, which was extracted with chloroform. The extract was washed with a saturated NaCl solution, dried and concentrated to give as a solid substance 2,2-bishydroxymethyl-5-norbornene (Compound No. P—10—1) (9.1 g), m.p., 108—110°C (recrystallization from isopropanol). IR$\nu_{max}^{Nujol}$ (cm$^{-1}$): 3300, 2950, 1020.

According to the same procedure, there were obtained the following compounds:

Endo-2,3-bishydroxymethyl-5-norbornene (Compound No. P—10—2), solid substance, M.P., 82—83°C. IR$\nu_{max}^{Nujol}$ (cm$^{-1}$): 3300—3200, 1220, 1030;

Endo-2,3-bishydroxymethyl-bicyclo[2,2,2]oct-5-ene (Compound No. P—10—3), solid substance, M.P., 94.5—95°C. (recrystallization from benzene-n-hexane). IR$\nu_{max}^{Nujol}$ (cm$^{-1}$): 3300—3200, 1210, 1030, 1020;

Exo-2-hydroxymethyl-endo-2-β-hydroxyethyl-5-norbornene (Compound P—10—4), oily substance. IR$\nu_{max}^{film}$ (cm$^{-1}$): 3300, 1570, 1450, 1340, 1250, 1050;

Exo-2-hydroxy-endo-2-hydroxymethyl-bicyclo[2,2,2]-oct-5-ene (Compound No. P—10—5), solid substance, M.P., 115—116.5°C IR$\nu_{max}^{Nujol}$ (cm$^{-1}$): 3350, 3270, 1290, 1050, 710.

Preparative Example 11

Into a mixture of dry benzene (30 ml), pyridine (13 ml) and 5-oxa-3-hydroxymethyl-brendane (5.0 g) was added p-toluenesulfonyl chloride (7.4 g) at 0—5°C, and the reaction mixture was stirred at room temperature overnight. The reaction mixture was discharged into a mixture of ethyl acetate (200 ml) and 10% HCl (50 ml), and the organic layer was separated, washed with a saturated $NaHCO_3$ solution, dried and concentrated under reduced pressure to afford as a solid substance 5-oxa-3-p-toluenesulfonyloxymethyl-brendane (Compound No. P—11—1) (6.6 g). IR$\nu_{max}^{Nujol}$ (cm$^{-1}$): 1600, 1480, 1360, 960, 860.

According to the same procedure, there were obtained the following compounds:

5-Oxa-endo-2-p-toluenesulfonyloxymethyl-brendane (Compound No. P—11—2), solid substance. IR$\nu_{max}^{Nujol}$ (cm$^{-1}$): 1600, 960, 860;

5-Oxa-endo-2-p-toluenesulfonyloxymethyl-isotwistane (Compound No. P—11—3), solid substance. IR$\nu_{max}^{Nujol}$ (cm$^{-1}$): 1600, 960, 860.

Preparative Example 12

A mixture of dimethylformamide (DMF, 20 ml), 5-oxa-3-bromomethyl-brendane (300 mg) and potassium phthalimide (250 mg) was stirred at 150°C for 2.5 hours. After cooling, the reaction mixture was discharged into a mixture of chloroform (50 ml) and water (200 ml), and the organic layer was separated and washed with 5% $Na_2CO_3$ solution and then water. The solvent was concentrated to give as a solid substance 5-oxa-3-phthalimidomethyl-brendane (Compound No. P—2—1) (300 mg), M.P., 135—137°C (recrystallization from isopropanol). IR$\nu_{max}^{Nujol}$ (cm$^{-1}$): 1770, 1630, 1610, 1430, 1400.

According to the same procedure, there were obtained the following compounds.

5-Oxa-endo-2-phthalimidomethyl-brendane (Compound No. P—12—2), solid substance, M.P., 123—124°C (recrystallization from isopropanol). IR$\nu_{max}^{Nujol}$ (cm$^{-1}$): 1780, 172—, 1700, 1470, 1440;

5-Oxa-endo-2-phthalimidomethyl-isotwistane (Compound No. P—12—3), solid substance. IR$\nu_{max}^{Nujol}$ (cm$^{-1}$): 1780, 1720, 1470.

Preparative Example 13

According to the Example 20 procedure, there was obtained the following compound:

Endo-3-N,N-diethylcarbamoyl-endo-2-methoxycarbonyl-5-norbene (Compound No. P—13—1), solid substance, M.P., 167—169°C IR$\nu_{max}^{KBr}$ (cm$^{-1}$): 2980, 1730, 1640, 1460.

Preparative Example 14

To a stirred solution of 5-oxa-3-hydroxymethyl-isotwistane (0.5 g) in benzene (0.8 ml) and dimethyl sulfoxide (2 ml) were added pyridine (0.61 ml) and orthophosphoric acid (0.08 ml), and dicyclohexylcarbonimide (1.8 g) was added portionwise thereto while cooling. The temperature was kept at a temperature of 25 to 30°C for 6 hours. The mixture was filtered, and the insoluble bicyclohexylurea was washed with a small amount of benzene. The filtrate and the washing were combined together, washed with 10% HCl, dried and concentrated to give as an oily substance 5-oxa-3-formyl-isotwistane (Compound No. P—14—1) (370 mg). IR$\nu_{max}^{film}$ (cm$^{-1}$): 2930, 2860, 1720, 1010.

Preparative Example 15

A mixture of 5-oxa-3-formyl-isotwistane (1.2 g), triphenyl cyanomethylidene phosphorane (2.2 g) and tetrahydrofuran (THF, 50 ml) was heated under reflux for 4 hours. Water was added thereto and the solvent was distilled off under reduced pressure. The residue was dissolved in ether, and the solution was washed with brine, dried and concentrated under reduced pressure. Chromatography on silica gel using hexane-isopropyl ether for elution yielded as an oily substance 5-oxo-3-(β-cyano-ethylene)-isotwistance (Compound No. P—15—1) (800 mg). IR$\nu_{max}^{film}$ (cm$^{-1}$): 2960, 2220, 1630.

Preparative Example 16

A mixture of endo-3-(β-chloroethyl)-exo-3-methoxycarbonyl-5-norbornene (1 g), potassium acetate (5 g), acetic acid (1 ml) and N,N-dimethylformamide (DMF, 5 ml) was heated at 120°C for 3 hours. The mixture was discharged into a mixture of benzene (50 ml) and water (50 ml), and the organic layer was separated, washed, dried and concentrated under reduced pressure to afford as an oily substance endo-3-(β-acetoxyethyl)-exo-2-methoxycarbonyl-5-norbornene (Compound No. P—1601) (1 g). IR$\nu_{max}^{film}$ (cm$^{-1}$): 1740, 1430, 1360.

Preparative Example 17

A mixture of endo-3-($\beta$-acetoxyethyl)-exo-2-methoxy-carbonyl-5-norobornene (1 g), conc. $H_2SO_4$ (several drops) and methanol (100 ml) was stirred at room temperature for 3 hours and concentrated under reduced pressure. The residue was diluted with water and extracted with ether. The extract was washed with water and dried. Removal of the solvent gave as an oily substance endo-3-($\beta$-hydroxyethyl)-exo-2-methoxycaronyl-5-norbornene (Compound No. P—17—1) (850 mg). IR$\nu_{max}^{film}$ ($cm^{-1}$): 3400, 1730, 1430, 1240.

Preparative Example 18

Into a solution of sodium metal (0.133 g) in dry ethanol (8.6 ml) was added a solution of tricyclo-[3,2,2,$0^{2,4}$]nonene-(6)-endo-8,9 dicarboxylic acid anhydride (1.0 g) in dry ethanol (5.8 ml) with stirring at room temperature for 2 hours and concentrated under reduced pressure. The residue was diluted with water, made acidic with 10% HCl and extracted with chloroform. The extract was washed with water and dried. Removal of the solvent gave a solid endo-8-carboxy-endo-9-ethoxycarbonyl-tricyclo[3,2,2$0^{2,4}$]nonene-(6) (Compound No. P—18—1), M.P., 85—87°C IR$\nu_{max}^{KBr}$ ($cm^{-1}$): 2735, 2650, 1735, 1700, 1185.

Preparative Example 19

A solution of methyllithium (1% in ether, ca.20 mmole) was added dropwise to 5-oxa-3-cyano-isotwistane (2.2 g) in dry ether (10 ml) at room temperature under nitrogen. After stirring at room temperature for 6 hours, 50 ml of 10% HCl solution was added cautiously. The mixture was extracted with benzene, dried and concentrated to give as an oily substance 5-oxa-3-acetyl-isotwistane (Compound No. P—19—1). IR$\nu_{max}^{film}$ ($cm^{-1}$): 2950, 1700, 1440, 1360, 1280.

5-Oxa-3-acetyl-isotwistane oxime was obtained by the usual method, M.P., 87—90°C. IR$\nu_{max}^{film}$ ($cm^{-1}$): 3300, 1280, 1000, 890.

Preparative Example 20

Into a mixture of dry benzene (30 ml), pyridine (30 ml) and exo-3-cyano-endo-3-hydroxymethyl-5-norbornene (10 g) was added methanesulfonyl chloride (0.20 g) at 0—5°C, and the mixture was stirred at room temperature over-night. The reaction mixture was discharged into a mixture of ethyl acetate and 10% HCl, and the organic layer was separated, washed, dried and concentrated under reduced pressure to afford as an oily substance exo-3-cyano-endo-3-mesyloxymethyl-5-norbornene (Compound No. P—20—1). IR$\nu_{max}^{film}$ ($cm^{-1}$): 2980, 2870, 2230, 1460, 1360.

According to the same procedure but replacing methanesulfonyl chloride with p-toluenesulfonyl chloride, there was obtained the following compound:

Exo-3-cyano-endo-3-p-toluenesulfonyloxymethylbicyclo[2,2,2]oct-5-ene (Compound No. P—20—2), solid substance, M.P., 95—96.5°C. IR$\nu_{max}^{Nujol}$ ($cm^{-1}$): 2250, 1600, 1180, 860.

Preparative Example 21

A mixture of exo-3-cyano-endo-3-mesyloxymethyl-5-norbornene (15 g), potassium thioacetate (16.3 g) and dry acetone (300 ml) was refluxed for 12 hours. After evaporation of the solvent, the residue was extracted with ether. The extract was washed with a saturated NaCl solution, dried and evaporated to give and oily substance, which was chromatographed on silica gel. Exo-3-cyano-endo-3-acetylthiomethyl-5-norbornene (Compound No. P—21—1) (4.5 g) as an oily substance was eluted with benzene. IR$\nu_{max}^{film}$ ($cm^{-1}$): 2970, 2230, 1700, 1340, 1130.

Preparative Example 22

A mixture of exo-3-cyano-endo-3-acetylthiomethyl-5-norbornene (4.5 g), sodium carbonate (3.0 g) and dry methanol (50 ml) was stirred at room temperature for 24 hours. After evaporation of the solvent, the residue was acidified with dilute HCl solution and extracted with ether. The extract was washed with a saturated NaCl solution, dried and concentrated to give as an oily substance exo-3-cyano-endo-3-mercaptomethyl-5-norbornene (Compound No. P—22—1) (2.5 g). IR$\nu_{max}^{film}$ ($cm^{-1}$): 2970, 2230, 1360, 1180, 960.

Preparative Example 23

Into a solution of exo-3-cyano-endo-3-mercaptomethyl-5-norbornene (2.5 g) in dry chloroform (10 ml) was added bromine ($Br_2$, 2.9 g) with stirring at 0°C for 1 hour. After consumption of excess $Br_2$ by addition of a dilute sodium thiosulfate solution, the mixture was extracted with chloroform. The extract was washed with a saturated NaCl solution, dried and concentrated to give a solid substance, which was chromatographed on silica gel to give exo-9-bromo-5-thia-3-cyano-brendane (Compound No. P—23—1) (1.0 g), 106—108°C. IR$\nu_{max}^{KBr}$ ($cm^{-1}$): 2930, 2230, 1450, 1270, 700.

Preparative Example 24

A mixture of exo-2-cyano-endo-2-toluenesulfonyloxymethyl-bicyclo[2,2,2]oct-5-ene (5.0 g), lithium bromide monohydrate (8.24 g), N,N-dimethylformamide (50 ml) and dimethylsulfoxide (50 ml)

was heated at 100°C for 3 hours. After cooling, the reaction mixture was discharged into a mixture of water and ether, and the organic layer was separated, washed, dried and concentrated under reduced presure to afford as an oily substance exo-2-cyano-endo-2-bromomethyl-bicyclo[2,2,2]oct-5-ene (Compound No. P—24—1) (3.4 g). IR$\nu_{max}^{film}$ ($cm^{-1}$): 3050, 2950, 2240, 1460, 1380, 1250.

## EXAMPLES

### Example 1

A solution of 5-oxa-4-oxo-endo-2-carboxy-brendane (10 g), N,N-dimethylformamide (2 drops) and thionyl chloride (83 ml) in dry dichloromethane was refluxed for 3 hours. The reaction mixture was evaporated to dryness, benzene (20 ml) was added thereto, and evaporation was effected to remove thionyl chloride. Into a solution of thus obtained acid chloride in acetone (40 ml), $NaN_3$ (3.93 g) was added under cooling. The reaction mixture was stirred for 24 hours at an ambient temperature. After evaporation of the solvent, the residue was dissolved in dry ethanol (100 ml) and refluxed for 2 hours. After evaporation of the solvent, the residue was extracted with chloroform. The extract was washed with water and dried. Evaporation of the solvent gave a solid, which was recrystallized from benzene to yield 6.9 g of 5-oxa-4-oxo-endo-2-ethoxycarbonylamino-brendane (Compound No. 1—1), M.P. 119—120°C. IR$\nu_{max}^{Nujol}$ ($cm^{-1}$): 3250, 3140, 1780, 1720.

According to the same procedure but replacing the ethanol with benzyl alcohol (2 equivalent molar ratio) in benzene, there were obtained the following compounds:

5-Oxa-4-oxo-3-benzyloxycarbonylamino-brendane (Compound No. 1—2), M.P., 134—136°C (recrystllization from isopropyl alcohol-isopropyl ether). IR$\nu_{max}^{Nujol}$ ($cm^{-1}$): 3330, 1770, 1720, 1530, 1260, 1080;

5-Oxa-4-oxo-3-benzyloxycarbonylamino-isotwistane (Compound No. 1—3), solid substance, M.P., 139—141°C (recrystallization from isopropyl alcohol-isopropyl ether). IR$\nu_{max}^{Nujol}$ ($cm^{-1}$): 3330, 1770, 1720, 1530, 1260, 1060;

6-Oxa-5-oxo-3-benzyloxycarbonylamino-homobrendane (Compound No. 1—4), solid substance, M.P., 106—107.5°C (recrystallization from isopropyl alcohol-isopropyl ether). IR$\nu_{max}^{Nujol}$ ($cm^{-1}$): 3330, 1710, 1530, 1240, 1060;

5-Oxa-4-oxo-endo-2-benzyloxycarbonylamino-brendane (Compound No. 1—5), solid substance, M.P., 136—137.5°C (recrystallization from isopropyl alcohol-isopropyl ether). IR$\nu_{max}^{Nujol}$ ($cm^{-1}$): 3310, 1780, 1690, 1530, 1170, 1030;

5-Oxa-4-oxo-endo-2-benzyloxycarbonylamino-isotwistane (Compound No. 1—6), solid substance, M.P., 147—147.5°C (recrystallization from isopropyl alcohol-isopropyl ether). IR$\nu_{max}^{Nujol}$ ($cm^{-1}$): 3370, 1770, 1720, 1530, 1260, 1050.

### Example 2

Into a solution of 5-oxa-3-carbony-brendane (4.0 g), triethylamine (3.1 g) in dry acetone (50 ml), ethylchloro-carbonate (3.1 g) was added with stirring at −20°C for 1 hour. Into the reaction mixture, sodium azide (2.02 g) in water (10 ml) was added at −20°C for 1 hour. After warming to room temperature, the reaction mixture was extracted with benzene and dried. After evaporation of the solvent, the residue was dissolved in dry ethanol (150 ml) and refluxed for 3 hours. After removal of the solvent, chromatography on silica gel using chloroform the elution yielded 5-oxa-3-ethoxycarbonylamino-brendane (Compound No. 2—1) (4.3 g) as an oil. IR$\nu_{max}^{film}$ ($cm^{-1}$): 3300, 2950, 1700, 1530.

According to an analogous procedure, there was obtained the following compounds:

5-Oxa-3-ethoxycarbonylamino-isotwistane (Compound No. 2—2), oily substance. IR$\nu_{max}^{film}$ ($cm^{-1}$): 3300, 2950, 1700, 1530;

6-Oxa-3-ethoxycarbonylamino-homobrendane (Compound No. 2—3), oily substance. IR$\nu_{max}^{film}$ ($cm^{-1}$): 3330, 2950, 2870, 1700;

6-Oxa-exo-2-ethoxycarbonylamino-homobrendane (Compound No. 2—4), oily substance. IR$\nu_{max}^{film}$ ($cm^{-1}$): 3340, 2960, 1720, 1540.

### Example 3

Into a solution of exo-9-iodo-5-oxa-4-oxo-3-ethoxycarbonylamino-brendane (4.5 g) and azobisisobutyronitrile (50 mg) in dry tetrahydrofuran (THF, 100 ml) was added tri-n-butyltin hydride (4.5 g) in dry ether (50 ml).

The reaction mixture was stirred for 2 hours at room temperature. After evaporation of the solvent, the residue was discharged into n-hexane (100 ml) to precipitate 5-oxa-4-oxo-3-ethoxycarbonylamino-brendane (Compound NO. 3—1) (2.8 g) as a solid. M.P., 91—92°C (recrystallization from benzene-n-hexane). IR$\nu_{max}^{Nujol}$ ($cm^{-1}$): 3320, 1790, 1690, 1380.

According to the same procedure, there were obtained the following compounds:

5-Oxa-4-oxo-3-ethoxycarbonylamino-isotwistane (Compound No. 3—2), solid substance, M.P., 157—159°C (recrystallization from isopropyl ether). IR$\nu_{max}^{Nujol}$ ($cm^{-1}$): 3330, 1780, 1710, 1540;

6-Oxa-5-oxo-3-ethoxycarbonylamino-homobrendane (Compound No. 3—3), solid substance, M.P., 90.5—92.0°C (recrystallization from benzene-n-hexane). IR$\nu_{max}^{KBr}$ ($cm^{-1}$): 3330, 1730—1700, 1520, 1450;

6-Oxa-5-oxo-3-ethoxycarbonylamino-homoisotwistane (Compound No. 3—4), solid substance, M.P., 118—120°C (recrystallization from benzene-n-hexane). IR$\nu_{max}^{Nujol}$ ($cm^{-1}$): 3300, 1730—1700;

5-Oxa-4-oxo-endo-2-ethoxycarbonylamino-isotwistane (Compound No. 3—5); solid substance, M.P., 157—159°C (recrystallization from isopropyl ether). IR$\nu_{max}^{Nujol}$ ($cm^{-1}$): 3300, 1780, 1700, 1540;

5-Oxa-4-oxo-3-cyano-brendane (Compound No. 3—6), solid substance, M.P., 150—151°C (recrystallization from benzene-cyclohexane). IR$\nu_{max}^{KBr}$ ($cm^{-1}$): 2950, 2250, 1780, 1000;

5-Oxa-4-oxo-3-cyano-isotwistane (Compound No. 3—7), solid substance, M.P., 203—205°C (recrystallization from benzene). IR$\nu_{max}^{KBr}$ ($cm^{-1}$): 2950, 2250, 1790;

6-Oxa-5-oxo-3-carboxy-homobrendane (Compound No. 3—8), solid substance, M.P., 194.5—196.0°C (recrystallization from ethyl acetate). IR$\nu_{max}^{Nujol}$ ($cm^{-1}$): 2600, 1730, 1690, 1400, 1220;

5-Oxa-4-oxo-3-carboxy-brendane (Compound No. 3—9), solid substance, M.P., 156—158°C (recrystallization from ethyl acetate). IR$\nu_{max}^{Nujol}$ ($cm^{-1}$): 1740, 1720, 1200, 1000;

5-Oxa-4-oxo-3-carboxy-isotwistane (Compound No. 3—10, solid substance, M.P., 199.5—200.5°C (recrystallization from ethyl acetate). IR$\nu_{max}^{Nujol}$ ($cm^{-1}$): 2650, 1780, 1710, 1300, 1070.

Example 4

Into a solution of exo-9-bromo-5-oxa-3-cyanobrendane (15.0 g) and azobisisobutyronitrile (100 mg) in dry tetrahydrofuran (300 ml) was added tri-n-butyltin hydride (11.5 g) in dry ether (100 ml). The reaction mixture was stirred for 5 hours at room temperature. Evaporation of the solvent gave an oily substance, which was chromatographed on silica gel.

5-Oxa-3-cyano-brendane (Compound No. 4—1) (10 g) as an oil was eluted with bezene. IR$\nu_{max}^{film}$ ($cm^{-1}$): 2950, 2870, 2240, 1450, 1100.

According to the same procedure, there were obtained the following compounds:

5-Oxa-3-cyano-isotwistane (Compound No. 4—2), solid substance M.P., 105°C IR$\nu_{max}^{Nujol}$ ($cm^{-1}$): 2250, 1480, 1440, 1340, 1080, 1040;

5-Oxa-3-hydroxymethyl-brendane (Compound No. 4—3), oily substance. IR$\nu_{max}^{film}$ ($cm^{-1}$): 3600—3100, 1450, 1340;

5-Oxa-3-hydroxymethyl-isotwistane (Compound No. 4—4), oily substance. IR$\nu_{max}^{film}$ ($cm^{-1}$): 3600—3100, 1450, 1340;

5-Oxa-endo-2-hydroxymethyl-brendane (Compound No. 4—5), oily substance. IR$\nu_{max}^{film}$ ($cm^{-1}$): 3400, 1440, 1360;

5-Oxa-endo-2-hydroxymethyl-isotwistane (Compound No. 4—6), oily substance. IR$\nu_{max}^{film}$ ($cm^{-1}$): 3500, 1100, 1080.

Example 5

Into a mixture of dry benzene (10 ml), pyridine (5 ml) and endo-5-hydroxy-endo-3-hydroxymethyl-exo-3-ethoxy-carbonylamino-norbornane (0.9 g) was added p-toluenesulfonyl chloride (P0.997 g) at 0—5°C for 1 hour, and the reaction mixture was stirred at room temperature overnight. The reaction mixture was discharged into a mixture of chloroform (100 ml) and 10% HCl (50 ml), and the organic layer was separated, washed, dried and concentrated under reduced pressure. Chromatography on silica gel using chloroform for elution yielded as an oily substance 5-oxa-3-ethoxycarbonylamino-brendane (Compound No. 5—1) (0.34 g). IR$\nu_{max}^{film}$ ($cm^{-1}$): 3300, 2950, 1700, 1530.

According to the same procedure, there were obtained the following compounds:

5-Oxa-endo-2-ethoxycarbonylamino-brendane (Compound No. 5—2), oily substance. IR$\nu_{max}^{film}$ ($cm^{-1}$): 3300, 1700, 1540, 1200;

5-Oxa-3-ethoxycarbonylamino-isotwistane (Compound No. 5—3), solid substance, M.P., 73—74.5°C. IR$\nu_{max}^{film}$ ($cm^{-1}$): 3300, 1700, 1550, 1240;

5-Oxa-endo-2-ethoxycarbonylamino-isotwistane (Compound No. 5—4), solid substance, M.P., 83—84°C. IR$\nu_{max}^{film}$ ($cm^{-1}$): 3320, 1720, 1540, 1260;

6-Oxa-3-ethoxycarbonylamino-homobrendane (Compound No. 5—5), oily substance IR$\nu_{max}^{film}$ ($cm^{-1}$): 3330, 2950, 2870, 1700;

6-Oxa-3-ethoxycarbonylamino-homoisotwistane (Compound No. 5—6), oily substance. IR$\nu_{max}^{film}$ ($cm^{-1}$): 3350, 1700, 1540, 1260;

5-Oxa-endo-2-N,N-diethylcarbamoyl-brendane (Compound No. 5—7), solid substance, M.P., 57—58.5°C. IR$\nu_{max}^{KBr}$ ($cm^{-1}$): 2850, 1640, 1485;

5-Oxa-3-cyano-brendane (Compound No. 5—8), oily substance. IR$\nu_{max}^{film}$ ($cm^{-1}$): 2950, 2870, 2240, 1450;

5-Oxa-endo-2-cyanomethyl-brendane (Compound No. 5—9), oily substance. IR$\nu_{max}^{film}$ ($cm^{-1}$): 2950, 2250, 1060, 1000.

Example 6

A mixture of 15% aqueous potassium hydroxide (40 ml) and 5-oxa-3-ethoxycarbonylaminobrendane (1 g) was refluxed for 7 hours and extracted with chloroform. The extract was washed with brine, dried and concentrated to give as an oily substance 5-oxa-3-amino-brendane (Compound No. 6-1). (0.83 g). $IR\nu_{max}^{film}$ ($cm^{-1}$): 3350, 3290, 2950, 1600, 1470.

According to the same procedure, there were obtained the following compounds.

5-Oxa-endo-2-amino-brendane (Compound No. 6-2), oily substance. $IR\nu_{max}^{film}$ ($cm^{-1}$): 3300, 3250, 1380, 1100;

5-Oxa-3-amino-isotwistane (Compound No. 6-3), oily substance. $IR\nu_{max}^{film}$ ($cm^{-1}$): 3350, 3280, 2930, 1250;

5-Oxa-endo-2-amino-isotwistane (Compound No. 6-4), oily substance. $IR\nu_{max}^{film}$ ($cm^{-1}$): 3350, 3280, 2940, 1590, 1090;

6-Oxa-3-amino-homobrendane (Compound No. 6-5), oily substance. $IR\nu_{max}^{film}$ ($cm^{-1}$): 3350, 3280, 2950, 2870, 1600, 1480;

6-Oxa-exo-2-amino-homobrendane (Compound No. 6-6), oily substance. $IR\nu_{max}^{film}$ ($cm^{-1}$): 3350, 3280, 1590, 1390;

6-Oxa-3-amino-homoisotwistane (Compound No. 6—7), oily substance. $IR\nu_{max}^{film}$ ($cm^{-1}$): 3330, 3270, 2920, 1520, 1250;

5-Oxa-endo-2-amino-tetracyclo[4,4,$1^{1,6}$,$0^{3,7}$,$0^{8,10}$]undecane (Compound No. 6-8), oily substance. $IR\nu_{max}^{film}$ ($cm^{-1}$): 3390, 3250, 2950, 2870, 1120.

Example 7

A mixture of 15% aqueous potassium hydroxide (30 ml) and 5-oxa-4-oxo-3-ethoxy-carbonylamino-brendane (3 g) was refluxed for 20 hours. After cooling, the reaction mixture was acidified with 10% HCl solution and stirred for overnight. The reaction mixture was basified (pH = 10) with aqueous sodium bicarbonate and extracted with chloroform. The extract was washed, dried and concentrated to give as a solid substance 5-oxa-4-oxo-3-amino-brendane (Compound No. 7-1), M.P., 142—143.5°C (recrystallization from benzene). $IR\nu_{max}^{KBr}$ ($cm^{-1}$): 3370, 3300, 2950, 1750, 1300, 1220

Example 8

A mixture of 10% aqueous potassium hydroxide (100 ml) and 5-oxa-3-cyano-brendane (10 g) was refluxed for 15 hours while bubbling $N_2$ gas into the solution. After cooling, the reaction mixture was acidified with 10% HCl solution and extracted with ether. The extract was washed with saturated NaCl solution, dried and concentrated to give as a solid substance 5-oxa-3-carboxy-brendane Compound No. 8-1) (8 g), M.P., 92—93°C (recrystallization from cyclohexane). $IR\nu_{max}^{KBr}$ ($cm^{-1}$): 2950, 2780 — 2650, 1710, 1270.

According to the same procedure, there were obtained the following compounds:

5-Oxa-3-carboxy-isotwistane (Compound No. 8-2), solid substance, M.P., 138—139°C (recrystallization from cyclohexane). $IR\nu_{max}^{KBr}$ ($cm^{-1}$): 2950, 2780 — 2650, 1710, 1270;

5-Oxa-4-oxo-3-carboxy-brendane (Compound No. 8-3), solid substance. (This product was identified as the compound obtained in Example 3 by IR spectrum and M.P.)

5-Oxa-4-oxo-3-carboxy-isotwistane (Compound No. 8-4), solid substance. (This product was identified as the compound obtained in Example 3 by IR spectrum and M.P.)

Example 9

According to the preparative Example 8 procedures, there was obtained the following compound:

5-Oxa-3-hydroxymethyl-brendane (Compound No. 9-1), oily substance. (This product was identified as the compound obtained in Example 4 by IR spectrum).

Example 10

A solution containing 2,2-bishydroxymethyl-5-norbornene (316 mg) in tetrahydrofuran (10 ml) was added to a mixture of mercuric acetate (637 mg) in water (20 ml) and tetrahydrofuran (20 ml) while stirring at room temperature for 3 hours. To the reaction mixture, sodium hydroxide (5.6 g) and then sodium borohydride (280 mg) were added while stirring in 2 hours so that the mercury had coagulated and settled. After decantation to remove the mercury, the solvent was concentrated, and the residue was extracted with chloroform. The extract was washed with water, dried and evaporated to dryness to yield 300 mg of 5-oxa-3-hydroxymethyl-brendane (Compound No. 10-1) as an oily substance. This compound was identified as the compound obtained in Example 4 by IR spectrum.

According to the same procedure, there were obtained the following compounds:

5-Oxa-3-hydroxymethyl-isotwistane (Compound No. 10-2), oily substance;

5-Oxa-endo-2-hydroxymethyl-brendane (Compound No. 10-3), oily substance;

5-Oxa-endo-2-hydroxymethyl-isotwistane (Compound No. 10-4), oily substance;

5-Oxa-3-cyano-brendane (Compound No. 10-5), oily substance;

5-Oxa-3-cyano-isotwistane (Compound No. 10-6), solid substance;

5-Oxa-endo-2-ethoxycarbonylamino-brendane (Compound No. 10-7), oily substance;

5-Oxa-endo-2-ethoxycarbonylamino-isotwistane (Compound No. 10-8), solid substance;

5-Oxa-endo-2-N,N-diethylcarbamoyl-brendane (Compound No 10-9), solid substance;

5-Oxa-endo-2-ethoxycarbonylamino-tetracyclo[$4,4,1^{1,6},0^{3,7},0^{8,10}$]undecane (Compound No. 10-10), solid substance, M.P., 100—102.5°C. $IR\nu_{max}^{Nujol}$ ($cm^{-1}$): 3300, 1710, 1530;

5-Oxa-exo-2-(β-chloroethyl)-brendane (Compound No. 10-11), oily substance. $IR\nu_{max}^{film}$ ($cm^{-1}$): 2950, 1470, 1360, 1280, 1120;

5-Oxa-3-hydroxy-isotwistane (Compound No. 10-12), oily substance. $IR\nu_{max}^{film}$ ($cm^{-1}$): 3300, 2950, 2870, 1420, 1100, 1050.

## Example 11

A solution containing endo-3-β-hydroxyethyl-exo-2-methoxycarbonyl-5-norbornene (1.96 g) in tetrahydrofuran (20 ml) was added to a mixture of mercuric trichloroacetate (4.02 g) (prepared from mercuric oxide (2.6 g) and trichloracetic acid) in water (50 ml) while stirring at room temperature for 24 hours. The reaction mixture was added to sodium borohydride (1.0 g) in 3 N sodium hydroxide (80 ml) with stirring for 3 hours. After decantation to remove the mercury, the solvent was concentrated and acidified with 10% HCl. The acidified mixture was extracted with chloroform, and the chloroform extract was washed with water, dried and concentrated. The residue was treated with an ethereal diazomethane solution and evaporated to afford as an oily substance 6-oxa-exo-2-methoxycarbonyl-homobrendane (Compound No. 11-1). $IR\nu_{max}^{film}$ ($cm^{-1}$): 2980, 1730, 1440

According to an analogous procedure there was obtained the following compound:

6-Oxa-3-hydroxymethyl-homobrendane (Compound no. 11-2), oily substance. $IR\nu_{max}^{film}$ ($cm^{-1}$): 3300, 2980, 1440.

## Example 12

A solution containing 5-oxa-3-ethoxycarbonylaminobrendane (4.3 g) in dry tetrahydrofuran (THF, 20 ml) was added to a mixture of lithium aluminum hydride ($LiAlH_4$, 2.8 g) in dry THF (100 ml) with refluxing for 6 hours. After consumption of excess $LiAlH_4$ by addition of water, the reaction mixture was concentrated to give a residue, which was extracted with chloroform. The extract was washed with a saturated NaCl solution, dried and concentrated to give an oily 5-oxa-3-N-methylamino-brendane (Compound No. 12-1) (2.8 g). $IR\nu_{max}^{film}$ ($cm^{-1}$): 3380, 2950, 1450, 1080.

According to the same procedure, there were obtained the following compounds:

5-Oxa-3-N-methylamino-isotwistane (Compound No. 19-2, 12-2), oily substance. $IR\nu_{max}^{film}$ ($cm^{-1}$): 3300, 2950, 1450, 1080;

5-Oxa-endo-2-N-methylamino-isotwistane (Compound No. 12-3), oily substance. $IR\nu_{max}^{film}$ ($cm^{-1}$): 3200, 2930, 1470, 1450;

5-Oxa-3-N,N-dimethylamino-brendane (Compound No. 12-4), oily substance. $IR\nu_{max}^{film}$ ($cm^{-1}$): 2950, 2870, 1460;

5-Oxa-3-N,N-dimethylamino-isotwistane (Compound No. 12-5), oily substance. $IR\nu_{max}^{film}$ ($cm^{-1}$): 2950, 2870, 1460.

## Example 13

Into a solution containing 5-oxa-3-N-methylaminobrendane (850 mg) and triethylamine (1.2 g) in dry ether (20 ml) was added a solution of ethyl chlorocarbonate (1.2 g) in dry ether (5 ml) with stirring at 0—5°C overnight. The mixture was filtered, and the filtrate was washed with 5% HCl solution and saturated NaCl solution, dried and concentrated to afford as an oily substance 5 - oxa - 3 - N - methyl - N - ethoxycarbonylamino - brendane (Compound No. 13-1) (900 mg). $IR\nu_{max}^{film}$ ($cm^{-1}$): 3050 — 2800, 1720 — 1680, 1440.

According to the same procedure, there were obtained the following compounds:

5-Oxa-3-N-methyl-N-ethoxycarbonylamino-isotwistane (Compound 13-2), oily substance. $IR\nu_{max}^{film}$ ($cm^{-1}$): 3050 — 2800, 1720, 1440;

5-Oxa-3-benzyloxycarbonylaminomethyl-isotwistane (Compound No. 13-3), oily substance. $IR\nu_{max}^{film}$ ($cm^{-1}$): 3300, 2930, 1720, 1540, 1250, 700.

## Example 14

To 4 g of 5-oxa-3-cyano-brendane in 20 ml of ethanol were added 2.8 ml of 6 N sodium hydroxide and 50 ml of 10% hydrogen peroxide. The mixture was heated cautiously for 30 minutes at 40—50°C and refluxed for 2 hours. Then, the reaction system was evaporated to dryness. The residue was extracted with ethyl acetate, the ethyl acetate solution was then evaporated to dryness. The new compound was 5-oxa-3-carbamoyl-brendane (Compound No. 14-1) (3.8 g), M.P., 138—139.5°C (recrystallization from benzene). $IR\nu_{max}^{KBr}$ ($cm^{-1}$): 3400, 3200, 2970, 2800, 1650.

According to the same procedure, there was obtained the following compound:

5-Oxa-3-carbamoyl-isotwistane (Compound No. 14-2), solid substance, M.P., 166.5—167.5°C. $IR\nu_{max}^{Nujol}$ ($cm^{-1}$): 3400, 3150, 1680, 1630.

Example 15

According to the preparative example 10 procedures, there were obtained the following compounds:

5-Oxa-3-hydroxymethyl-isotwistane (Compound No. 15-1), oily substance. IR$\nu_{max}^{film}$ ($cm^{-1}$): 3300, 2950, 2870, 1450;

5-Oxa-3-($\alpha$-hydroxy)ethyl-isotwistane (Compound No. 15-2), oily substance. IR$\nu_{max}^{film}$ ($cm^{-1}$): 3400, 2950, 1290, 1040.

Example 16

A mixture of methyl ethyl ketone (20 ml), 5-oxa-3-p-toluenesulfonyloxymethyl-brendane (430 mg) and lithium bromide monohydrate (400 mg) was refluxed for 3 hours and concentrated under reduced pressure. The residue was extracted with chloroform, and the chloroform later was washed, dried and concentrated to give as an oily substance 5-Oxa-3-bromomethyl-brendane (Compound No. 16-1) (300 mg). IR$\nu_{max}^{film}$ ($cm^{-1}$): 1400, 1240, 1100, 1040.

According to the same procedure, there were obtained the following compounds.

5-Oxa-endo-2-bromomethyl-brendane (Compound No. 16-2), oily substance. IR$\nu_{max}^{film}$ ($cm^{-1}$): 2950, 1230, 1040.

Example 17

A mixture of 300 mg of 5-oxa-3-phthalimidomethyl-brendane, 2 ml of ethanol and 100 mg of hydrazine monohydrate was heated under reflux for 10 minutes. After cooling, 2 ml of 10% HCl solution was added and heated for 10 minutes. The mixture was filtered to remove the precipitated substance, and the filtrate was basified with 10% NaOH and extracted with chloroform. The extract was washed with water, dried and concentrated to give as an oily substance 5-oxa-3-aminomethyl-brendane (Compound No. 17-1) (100 mg). IR$\nu_{max}^{film}$ ($cm^{-1}$): 3350, 3300, 1450, 1020.

According to the same procedure, there were obtained the following compounds:

5-Oxa-endo-2-aminomethyl-brendane (Compound No. 17-2), oily substance. IR$\nu_{max}^{film}$ ($cm^{-1}$): 3300, 3250, 2950, 1100;

5-Oxa-endo-2-aminomethyl-isotwistane (Compound No. 17-3), oily substance. IR$\nu_{max}^{film}$ ($cm^{-1}$): 3350, 3280, 2950, 1460.

Example 18

Into a mixture of sodium metal (4 g), 5-oxa-3-carbamoyl-brendane (14.5 g) and dry ethanol (240 ml) was added bromine (15.2 g) at room temperature, and the resulting mixture was stirred for 1 hour and refluxed for 10 minutes. After evaporation of the solvent, the residue was extracted with chloroform, washed with saturated NaCl and dried. The extract was concentrated to give as an oily substance 5-oxa-3-ethoxycarbonylamino-brendane (Compound No. 8-1) (15.1 g). This compound was identified as the compound obtained in Example 2 by IR spectrum.

According to the same procedure but replacing ethanol-sodium ethoxide by water-sodium hydroxide, there was obtained the following compound:

5-Oxa-3-amino-brendane (Compound No. 18-2), oily substance. (This compound was identified as the compound obtained in Example 6 by IR spectrum).

Example 19

Into a solution containing 5-oxa-3-cyano-brendane (1 g) in dry tetrahydrofuran (THF, 10 ml) was added a mixture of lithium aluminium hydride ($LiAlH_4$, 0.3 g) in dry THF (10 ml) with stirring at room temperature for 2 hours. After consumption of excess $LiAlH_4$ by addition of water, the reaction mixture was concentrated to give a residue, which was extracted with ether. The extract was washed with a saturated NaCl solution, dried and concentrated to give as an oily substance 5-oxa-3-aminomethyl-brendane (Compound No. 19-1) (0.8 g).

This compound was identified as the compound obtained in Example 17 by IR spectrum.

According to the same procedure, there were obtained the following compounds:

5-Oxa-3-aminomethyl-isotwistane (Compound No. 19-2), oily substance. IR$\nu_{max}^{film}$ ($cm^{-1}$): 3400, 3300, 1030, 1010;

5-Oxa-endo-2-$\beta$-aminoethyl-brendane (Compound No. 19-3), oily substance. IR$\nu_{max}^{film}$ ($cm^{-1}$): 3400, 3320, 1040;

5-Oxa-3-($\omega$-amino-n-propyl)-isotwistane (Compound No. 19-4), oily substance. IR$\nu_{max}^{film}$ ($cm^{-1}$): 3390, 3320, 1030, 1010;

5-Oxa-exo-3-($\omega$-amino-n-propyl)-brendane (Compound No. 19-5), oily substance. IR$\nu_{max}^{film}$ ($cm^{-1}$): 3400, 3320, 1040.

Example 20

A mixture of 5-oxa-4-oxo-endo-2-carboxy-brendane (10 g), thionyl chloride (20 ml) and methylene dichloride (60 ml) was refluxed for 6 hours. Removal of the solvent gave the corresponding acid chloride. A solution of such acid chloride in dry chloroform (50 ml) was added to a solution of

diethylamine (11.8 g) in dry ether (100 ml) with stirring at 0—5°C. The mixture was stirred at room temperature for 2 hours, and 5% HCl solution (100 ml) was added thereto. The mixture was extracted with chloroform, and the extract was washed with a saturated NaCl solution, dried and concentrated to give as a solid substance 5-oxa-4-oxo-endo-2-N,N-diethylcarbamoyl-brendane (Compound No. 20-1) (14.4 g), M.P., 78—81°C. $IR\nu_{max}^{KBr}$ ($cm^{-1}$): 2980, 1780, 1650, 1440.

According to the same procedure, there were obtained the following compounds:

6-Oxa-5-oxo-3-carbamoyl-homobrendane (Compound No. 20-2), solid substance, M.P., 163—165°C (recrystallization from isopropanol). $IR\nu_{max}^{KBr}$ ($cm^{-1}$): 3430, 3300, 1710, 1680, 1220;

5-Oxa-4-oxo-3-carbamoyl-isotwistane (Compound No. 20-3), solid substance, M.P., 139—140°C $IR\nu_{max}^{Nujol}$ ($cm^{-1}$): 3450, 3280, 1770, 1680, 1100;

5-Oxa-3-N,N-diallylcarbamoyl-brendane (Compound No. 20-4), oily substance. $IR\nu_{max}^{film}$ ($cm^{-1}$): 2950, 1640, 1410, 1240, 1040, 930;

5-Oxa-3-pyrrolidinocarbonyl-brendane (Compound No. 20-5), solid substance, M.P., 114.5—115.5°C $IR\nu_{max}^{Nujol}$ ($cm^{-1}$): 1610, 1260, 1040, 1020, 800;

5-Oxa-3-morpholinocarbonyl-brendane (Compound No. 20-6), solid substance, M.P., 128.5—131°C $IR\nu_{max}^{Nujol}$ ($cm^{-1}$): 1620, 1280, 1250, 1110, 1020, 860.

Example 21

A mixture of 5-oxa-3-carboxy-brendane (1.0 g), sodium hydroxide (360 mg), water (1.4 ml) and hexamethyl phosphoramide (20 ml) was stirred at room temperature for 1 hour. Into such solution was added methyl iodide (3.4 g) with stirring at the same temperature for 1 hour. The reaction mixture was discharged into a mixture of ether and 10% HCl (50 ml) and the organic layer was separated, washed, dried and concentrated to afford an oily substance. Chromatography on silica gel using benzene for elution yielded as a solid substance 5-oxa-3-methoxycarbonyl-brendane (Compound No. 21-1) (1.2 g). $IR\nu_{max}^{film}$ ($cm^{-1}$): 2950, 1730, 1440.

According to the same procedure, there was obtained the following compound:

6-Oxa-3-methoxycarbonyl-homobrendane (Compound No. 21-2), oily substance $IR\nu_{max}^{film}$ ($cm^{-1}$): 2950, 2870, 1730, 1270.

Example 22

Into a solution of 0.79 g of potassium hydroxide in 140 ml of water was suspended 5 g of 6-oxa-3-hydroxymethyl-homobrendane. To this solution, 6.9 g of potassium permanganate were added at such a rate that the temperature did not exceed 40°C. After 2 hours, manganese dioxide was filtered off, and excess of permanganate was reduced with a saturated sodium bisulfite solution. The mixture was again filtered, acidified with concentrated hydrochloric acid and extracted with chloroform. The extract was washed with saturated NaCl, dried and concentrated to give white solid 6-oxa-3-carboxyl-homobrendane (Compound No. 22-1) (4.2 g), M.P., 78.5—80.5°C (recrystallization from cyclohexane). $IR\nu_{max}^{KBr}$ ($cm^{-1}$): 2960, 2650, 1700, 1220, 1100.

Example 23

Into a solution of 5-oxa-4-oxo-endo-2-carboxybrendane (5.8 g) in dry tetrahydrofuran (THF, 110 ml) was added a solution of diborane in THF at 0°C. The mixture was stirred at the same temperature for 2 hours, and excess diborane was quenched with water. The reaction mixture was concentrated under reduced pressure. This residue was diluted with chloroform, washed with water, dried and then evaporated to afford as an oily substance 5-oxa-4-oxo-endo-2-hydroxymethyl-brendane (Compound No. 23-1) (4.8 g). $IR\nu_{max}^{film}$ ($cm^{-1}$): 3450, 2960, 2880, 1780, 1360, 1170, 1040, 1020.

Example 24

Into a solution of 5-oxa-4-oxo-endo-2-hydroxymethyl-brendane (4 g) in pyridine was added a solution of mesyl chloride (2.85 g) in dry benzene (5 ml) under cooling. After stirring for several hours, the reaction mixture was diluted with benzene and washed with 10% hydrochloric acid, aqueous sodium bicarbonate and aqueous sodium chloride in that order. The organic layer was dried and evaporated. The thus obtained mesylate was dissolved in dimethylsulfoxide (DMSO, 25 ml) and treated with sodium cyanide (2 g) at 90—105°C for 2 hours to afford as an oily substance 5-oxa-3-oxo-endo-2-cyanomethyl-brendane (Compound No. 24-1) (3.6 g). $IR\nu_{max}^{film}$ ($cm^{-1}$): 2250, 1780, 1360, 1170, 1040.

Example 25

5-Oxa-3-($\beta$-cyanoethylene)-isotwistane (800 mg) was hydrogenated with 5% Pd-C (160 mg) in ethanol under atmospheric pressure. The ethanolic solution thus obtained was filtered to remove the catalyst, and the filtrate was concentrated to afford an oily substance. Chromatography on silica gel using benzene for elution yielded as a solid substance 5-oxa-3-($\beta$-cyanoethyl)-isotwistane (compound No. 25-1) (800 mg), M.P., 53—55°C (recrystallization from isopropyl ether): $IR\nu_{max}^{Nujol}$ ($cm^{-1}$): 2240, 1080, 1030, 1010.

Example 26

5-Oxa-4-oxo-3-benzyloxycarbonylamine brendane (2.5 g) was hydrogenolyzed with 10% Pd-C (0.25 g) in ethanol (100 ml) under atmospheric pressure. The ethanolic solution thus obtained was filtered to remove the catalyst and the filtrate was concentrated to afford as a solid substance 5-oxa-4-oxo-3-amino-brendane (Compound No. 26-1) (1.37 g). This product was identified as the compound obtained in Example 7 by IR spectrum and M.P.

According to the same procedure, there were obtained the following compounds:

5-Oxa-4-oxo-3-amino-isotwistane (Compound No. 26-2), solid substance, M.P., 139—141°C (recrystallization from isopropanol-isopropyl ether). IR$\nu_{max}^{Nujol}$ ($cm^{-1}$): 3360, 3300, 1760, 1150, 1090, 760;

6-Oxa-5-oxo-3-amino-homobrendane (Compound No. 26-3), solid substance, M.P., 143—145°C (recrystallization from benzene-hexane). IR$\nu_{max}^{Nujol}$ ($cm^{-1}$): 3530, 3340, 3310, 1730, 1240, 1030;

5-Oxa-4-oxo-endo-2-amino-brendane (Compound No.26-4), solid substance, M.P., 182—184°C IR$\nu_{max}^{Nujol}$ ($cm^{-1}$): 3400, 3330, 1770, 1160, 1100, 1030, 980;

5-Oxa-4-oxo-endo-2-amino-isotwistane (Compound No. 26-5), solid substance. IR$\nu_{max}^{Nujol}$ ($cm^{-1}$): 3370, 3280, 1770, 1300, 1160, 1090.

5-Oxa-3-N-ethylaminomethyl-isotwistane (Compound No. 26-6), oily substance. IR$\nu_{max}^{film}$ ($cm^{-1}$): 3320, 2960, 1370, 1220.

Example 27

According to the preparative example 16 procedure, there was obtained the following compound:

5-Oxa-exo-2-($\beta$-acetoxyethyl)-brendane (Compound No. 27-1), oily substance IR$\nu_{max}^{film}$ ($cm^{-1}$): 2950, 1720, 1430, 1360.

Example 28

According to the preparative example 17 procedure, there was obtained the following compound:

5-Oxa-exo-2-($\beta$-hydroxyethyl)-brendane (Compound No. 28-1), oily substance. IR$\nu_{max}^{film}$ ($cm^{-1}$): 3400, 2950, 1430, 1360, 1040.

Example 29

Into a solution of 5-oxa-3-aminomethyl-isotwistane (1.0 g) in dry dichloromethane (10 ml) was added trifluoroacetic anhydride (960 mg) with stirring at room temperature for 3 hours. The mixture was discharged into a mixture of 5% aqueous sodium bicarbonate and chlorofrom, and the organic layer was washed with water and dried. Removal of the solvent gave solid 5-oxa-3-N-trifluoroacetylaminomethylisotwistane (Compound No. 29-1) (0.9 g), M.P., 81.5—84°C. IR$\nu_{max}^{Nujol}$ ($cm^{-1}$): 3350 — 3050, 1700.

According to the same procedure, using 5-oxa-3-amino-isotwistane (1.52 g), triethylamine (2 g) and acetyl chloride (0.86 g), there was obtained as an oily substance 5-oxa-3-N-acetylamino-isotwistane (Compound No. 29-2) (800 mg). IR$\nu_{max}^{Nujol}$ ($cm^{-1}$): 3400, 2960, 1660, 1560.

Example 30

Into a solution containing 5-oxa-3-N-acetylaminomethyl-isotwistane (600 mg) in dry tetrahydrofuran (THF, 10 ml) was added a mixture of lithium aluminum hydride ($LiAlH_4$ 218 mg) in dry tetrahydrofuran (10 ml) with refluxing for 6 hours. After consumption of excess $LiAlH_4$ by addition of water, the reaction mixture was concentrated to give a residue, which was extracted with chloroform. The extract was washed with a saturated NaCl solution, dried and concentrated to give as an oily substance 5-oxa-3-N-ethylaminomethyl-isotwistane (Compound No. 30-1) (420 mg).

According to the same procedure, there were obtained the following compounds:

5-Oxa-3-N,N-diallylaminomethyl-brendane (Compound No. 30-2), oily substance. IR$\nu_{max}^{film}$ ($cm^{-1}$): 2950, 1470, 1380, 1040;

5-Oxa-3-pyrrolidinomethyl-brendane (Compound No. 30-3), oily substance. IR$\nu_{max}^{film}$ ($cm^{-1}$): 2950, 1470, 1360, 1040;

5-Oxa-3-morpholinomethyl-brendane (Compound No. 30-4), oily substance. IR$\nu_{max}^{film}$ ($cm^{-1}$): 2950, 1470, 1360, 1040.

Example 31

Into a solution of 5-oxa-3-N-trifluoroacetylaminomethyl-isotwistane (1.0 g) and propargyl bromide (1.8 g) in dry acetone (45 ml) was added potassium hydroxide (709 mg) with stirring at 60°C. The reaction mixture was refluxed for 1 hour and concentrated under reduced pressure. A solution containing potassium hydroxide (709 mg) in water (10 ml) was added to a residue with refluxing for 15 minutes. After cooling, the mixture was extracted with chloroform, and the extract was washed with water and dried. Removal of the solvent gave as an oily substance 5-oxa-3-N-propargylaminomethyl-isotwistane (Compound No. 31-1) (200 mg). IR$\nu_{max}^{film}$ ($cm^{-1}$): 3400 — 3100, 2950, 1520.

According to the same procedure, but replacing propargyl bromide by allyl bromide, there was obtained as an oily substance 5-oxa-3-N-allylaminomethyl-isotwistane (Compound No. 31-2) IR$\nu_{max}^{film}$ ($cm^{-1}$): 3300, 2950, 1450, 1380.

Example 32

A solution containing 5-oxa-3-acetyl-isotwistane oxime (900 mg) in dry tetrahydrofuran (20 ml) was added to a mixture of lithium aluminum hydride ($LiAlH_4$, 700 mg) in dry tetrahydrofuran (10 ml) with refluxing for 8 hours. After consumption of excess $LiAlH_4$ by addition of water, the reaction mixture was concentrated to give a residue, which was extracted with chloroform. The extract was washed with a saturated NaCl solution, dried and concentrated to give as an oily substance 5-oxa-3-($\alpha$-aminoethyl)-isotwistane (Compound No. 32-1) (750 mg). IR$\nu_{max}^{film}$ ($cm^{-1}$): 3450, 3300, 2950, 1470, 1440, 1370.

Example 33

A solution containing exo-9-bromo-5-thia-3-cyanobrendane (0.9 g) in dry tetrahydrofuran (20 ml) was added to a mixture of lithium aluminum hydride ($LiAlH_4$, 1.05 g) in dry tetrahydrofuran (40 ml) with refluxing for 15 hours. After consumption of excess $LiAlH_4$ by addition of water, the reaction mixture was concentrated to give a residue, which was extracted with chloroform. The extract was washed, dried and concentrated to give as an oily substance 5-thia-3-aminomethyl-brendane (Compound No. 33-1). IR$\nu_{max}^{film}$ ($cm^{-1}$): 3380, 3330, 2950, 1580, 1440.

Example 34

Into a solution of 5-oxa-3-aminomethyl-isotwistane (1.5 g) in N,N-dimethylformamide (20 ml) was added benzyl chloride (2.5 g) with stirring at 80°C for 5 hours. After cooling, the solution was discharged into a mixture of water and ether, and the organic layer was separated, washed, dried and concentrated under reduced pressure to afford as an oily substance 5-oxa-3-N-benzylaminomethyl-isotwistane (Compound No. 34-1) (1.2 g) IR$\nu_{max}^{film}$ ($cm^{-1}$): 3300, 2950, 1320, 700.

Example 35

A mixture of exo-2-cyano-endo-2-bromomethylbicyclo[2,2,2]oct-5-ene (5.0 g), sodium hydrosulfide (4.4 g), N,N-dimethylformamide (20 ml) and dimethylsulfoxide (20 ml) was stirred at room temperature for 5 hours. The mixture was discharged into a mixture of water and ether, and the organic layer was separated, washed, dried and concentrated under reduced pressure to afford an oily substance. Chromatography on silica gel using benzene for elution yielded as an oily substance 5-thia-3-cyano-isotwistane (Compound No. 35-1) (1.5 g) IR$\nu_{max}^{film}$ ($cm^{-1}$): 2950, 2870, 2230, 1450, 1190.

Example 36

A solution of 5-thia-3-cyano-isotwistane (500 mg) in dry ether (10 ml) was added to a mixture of lithium aluminum hydride ($LiAlH_4$, 265 mg) in dry ether (10 ml) and stirred at 0—5°C for 2 hours. After consumption of excess $LiAlH_4$ by addition of water, the reaction mixture was extracted with ether. The extract was washed with a saturated NaCl solution, dried and concentrated to give a solid 5-thia-3-aminomethyl-isotwistane (Compound No. 36-1) (430 mg), M.P. 75—77°C. IR$\nu_{max}^{film}$ ($cm^{-1}$): 3360, 3300, 2950, 1600, 1470, 1450.

Example 37

A mixture of 5-thia-3-aminomethyl-isotwistane (250 mg), potassium metaperiodate (296 mg) in water (25 ml) and methanol (25 ml) was stirred at room temperature for 1 hour. After removal of methanol, the mixture was extracted with chloroform. The extract was washed, dried and concentrated to give as an oily substance 5-thia-5-oxo-3-aminomethyl-isotwistane (Compound No. 37-1) (160 mg) IR$\nu_{max}^{film}$ ($cm^{-1}$): 3370, 3300, 2930, 1450, 1040, 1020.

According to the same procedure, there was obtained the following compound:

5-Thia-5-oxo-3-amino-isotwistane (Compound No. 37-2), oily substance. IR$\nu_{max}^{film}$ ($cm^{-1}$): 3370, 3320, 2940, 1450, 1040, 1020.

Example 38

5-Thia-3-aminomethyl-isotwistane (300 mg) was dissolved in acetic acid (8N, 6 ml), and a solution of potassium permanganate (0.41 g) in water (3.4 ml) was added thereto with stirring at 25°C in 0.5 hour. This mixture was cooled in ice, decolorised by passing in sulfur dioxide, adjusted to pH 12 with sodium hydroxide and extracted with chloroform. The extract was washed, dried and concentrated to give as an oily substance 5-thia-5,5-dioxo-3-aminomethyl-isotwistane (Compound No. 38-1) (220 mg). IR$\nu_{max}^{film}$ ($cm^{-1}$): 3400, 3330, 2930, 1295, 1220, 1110, 760.

According to the same procedure, there was obtained the following compound:

5-Thia-5,5-dioxo-3-amino-isotwistane (Compound No. 38-2), oily substance. IR$\nu_{max}^{film}$ ($cm^{-1}$): 3370, 3310, 2940, 1290, 1220, 1110.

Example 39

A mixture of endo-2-ethoxycarbonylamino-endo-3-hydroxymethyl-brendane (500 mg), 50% $H_2SO_4$ solution (10 ml) and ethanol (20 ml) was heated at 70°C for 2 hours. After cooling, the reaction mixture was discharged into a sodium bicarbonate solution (200 ml) and extracted with chloroform. The extract was washed with a saturated NaCl solution, dried and concentrated to give as an oily substance 5-oxa-endo-2-ethoxycarbonylamino-brendane (Compound No. 39-1) (500 mg). This compound was identified as the compound obtained in Example 5 by IR spectrum.

According to the same procedure, there was obtained the following compound:

5-Oxa-3-ethoxycarbonyl-isotwistane (Compound No. 39-2), oily substance. $IR\nu_{max}^{film}$ ($cm^{-1}$): 2950, 2860, 1730, 1460, 1330.

Example 40

A mixture of 5-oxa-exo-3-($\beta$-chloroethyl)-brendane (1.86 g), sodium cyanide (1.47 g) and dimethyl sulfoxide (DMSO, 20 ml) was heated at 90—100°C for 5 hours. After cooling, the reaction mixture was discharged into a mixture of water and ether, and the organic layer was separated, washed, dried and concentrated under reduced pressure to afford an oily substance. Chromatography on silica gel using benzene for elution yielded as an oily substance 5-oxa-exo-2-($\beta$-cyanoethyl)-brendane (Compound No. 40-1) (1.2 g). $IR\nu_{max}^{film}$ ($cm^{-1}$): 2950, 2230, 1450, 1120.

According to the same procedure, there was obtained the following compound:

5-Oxa-endo-2-cyanomethyl-isotwistane (Compound No. 40-2), oily substance. $IR\nu_{max}^{film}$ ($cm^{-1}$): 2950, 2240, 1450, 1040.

Example 41

A mixture of 5-oxa-endo-2-($\beta$-chloroethyl)-brendane (1.9 g), benzylamine (1.56 g), potassium carbonate (1.4 g) and dry N,N-dimethylformamide (19 ml) was heated at 100°C for 2.5 hours. After cooling the solution was discharged into a mixture of water and ether, and the organic layer was separated, washed, dried and concentrated under reduced pressure to afford as an oily substance 5-oxa-2-[$\beta$-(benzylamino)ethyl]-brendane (Compound No. 41-1). $IR\nu_{max}^{film}$ ($cm^{-1}$): 3300, 2950, 2850, 1480, 1350, 760.

Example 42

Into a solution of 5-oxa-3-benzyloxycarbonylaminomethyl-isotwistane (300 mg) in dry dimethyl sulfoxide (5 ml) was added sodium hydride (65%, 123 mg) with stirring at 120—130°C for 1.5 hours. After cooling, a solution of ethyliodide (5.8 mg) in dry dimethylformamide (5 ml) was added to the resulting mixture and stirred at 9°C for 10 hours. The mixture was discharged into a mixture of benzene and water and extracted with benzene. The extract was washed, dried and concentrated to afford an oily substance. Chromatography on silica gel using chloroform for elution yielded as an oily substance 5-oxa-3-(N-ethyl-N-benzyloxycarbonyl)aminomethyl-isotwistane (Compound No. 42-1) (450 mg). $IR\nu_{max}^{film}$ ($cm^{-1}$): 2930, 2860, 1700, 1460, 1250, 700.

Example 43

A mixture of 5-thia-3-cyano-isotwistane (1 g), potassium hydroxide (85%, 1.25 g) and ethylene glycol (4.5 ml) was refluxed for 15 minutes. After cooling, the mixture was discharged into a mixture of 10% HCl (20 ml) and benzene and extracted with benzene. The extract was washed, dried and concentrated to give solid 5-thia-3-carboxy-isotwistane (Compound No. 43-1) (650 mg), M.P., 108—113°C. $IR\nu_{max}^{KBr}$ ($cm^{-1}$): 2950, 1700, 1410, 1290, 920.

Example 44

Into a solution of 5-thia-3-carboxy-isotwistane (640 mg), triethylamine (327 mg) in dry acetone (14 ml) was added ethyl chlorocarbonate (421 mg) with stirring at —20°C for 1 hour. Into a reaction mixture was added sodium azide (273 mg) in water (2 ml) at —20°C for 1 hour. After heating to room temperature, the reaction mixture was extracted with benzene and dried. After evaporation of the solvent, the residue was dissolved in dry ethanol (100 ml) and refluxed for 2 hours.

After removal of the solvent, chromatography on silica gel using chloroform for elution yielded solid 5-thia-3-ethoxycarbonylamino-isotwistane (Compound No. 44-1) (620 mg), M.P., 73—76°C. $IR\nu_{max}^{film}$ ($cm^{-1}$): 3330, 2940, 2870, 1700, 1520, 1270, 1060.

Example 45

A mixture of 5-thia-3-ethoxycarbonylamino-isotwistane (283 mg), potassium hydroxide (85%, 309 mg) and ethylene glyxol (1.2 ml) was refluxed for 15 minutes. After cooling, the mixture was discharged into a mixture of water and chloroform and extracted with chloroform.

The extract was washed, dried and concentrated to give an as oily substance 5-thia-3-amino-isotwistane (Compound No. 45-1) (155 mg). $IR\nu_{max}^{KBr}$ ($cm^{-1}$): 3330, 3250, 2930, 2850, 1450, 1110.

Example 46

Into a solution of 1.0 g of dimethylamine hydrochloride in 8 ml of methanol was added potassium hydroxide (0.5 g), and 1.8 g of 5-oxa-3-acetyl-isotwistane (1.8 g) was added thereto in one portion. The resulting suspension was stirred at room temperature for 15 minutes, and then a solution of sodium cyanoborohydride (0.25 g) in 5 ml of methanol was added dropwise over 30 minutes. The suspension was stirred for 30 minutes. Potassium hydroxide (0.72 g) was then added, and stirring was continued.

The reaction mixture was filtered with suction and the filtrate was extracted with benzene. The extract was washed, dried and concentrated to give as an oily substance 5 - oxa - 3 - ($\alpha$ - N,N - dimethylamino)ethyl - isotwistane (Compound No. 46-1). IR$\nu_{max}^{film}$ ($cm^{-1}$): 2950, 2870, 1460.

Example 47

A solution of 5-oxa-3-carboxy-brendane (2.0 g), thionyl chloride (20 ml) and methylene dichloride (20 ml) was refluxed for 3 hours. The reaction mixture was evaporated to dryness, benzene (20 ml) was added thereto, and the resulting mixture was evaporated to remove thionyl chloride. A solution of thus obtained acid chloride in tetrahydrofuran (6.5 ml) was added to a solution of hydroxylamine hydrochloride (3.3 g) and sodium hydroxide (1.9 g) in water (15 ml) at 0°C. The reaction mixture was stirred for 2 hours at the same temperature. The mixture was extracted with chloroform, washed, dried and concentrated to give as an oily substance 5-oxa-3-(N-hydroxycarbamoyl)-brendane (Compound No. 47-1) (2.0 g). IR$\nu_{max}^{film}$ ($cm^{-1}$): 3300, 3250, 2950, 1650, 1480, 1020.

To a solution of sodium ethoxide (0.25 g) in dry toluene (5 ml) was added a solution of 5-oxa-3-(N-hydroxycarbamoyl)-brendane (1.0 g) in dry toluene (5 ml) with stirring at room temperature. The reaction mixture was refluxed for 2 hours, ethanol (10 ml) was added thereto and then refluxing was made for 2 hours. After evaporation of the solvent, the residue was discharged into a mixture of 10% HCl solution and chloroform. The organic layer was separated, washed, dried and concentrated to give an oily residue. Chromatography on silica gel using chloroform for elution yielded as an oily substance 5-oxa-3-ethoxycarbonylamino-brendane (Compound No. 47-2) (0.20 g). This compound was identified as the compound obtained in Example 6 by IR spectrum.

Structure of the compounds of the Examples and Preparative Examples and their Relationship

CHART E

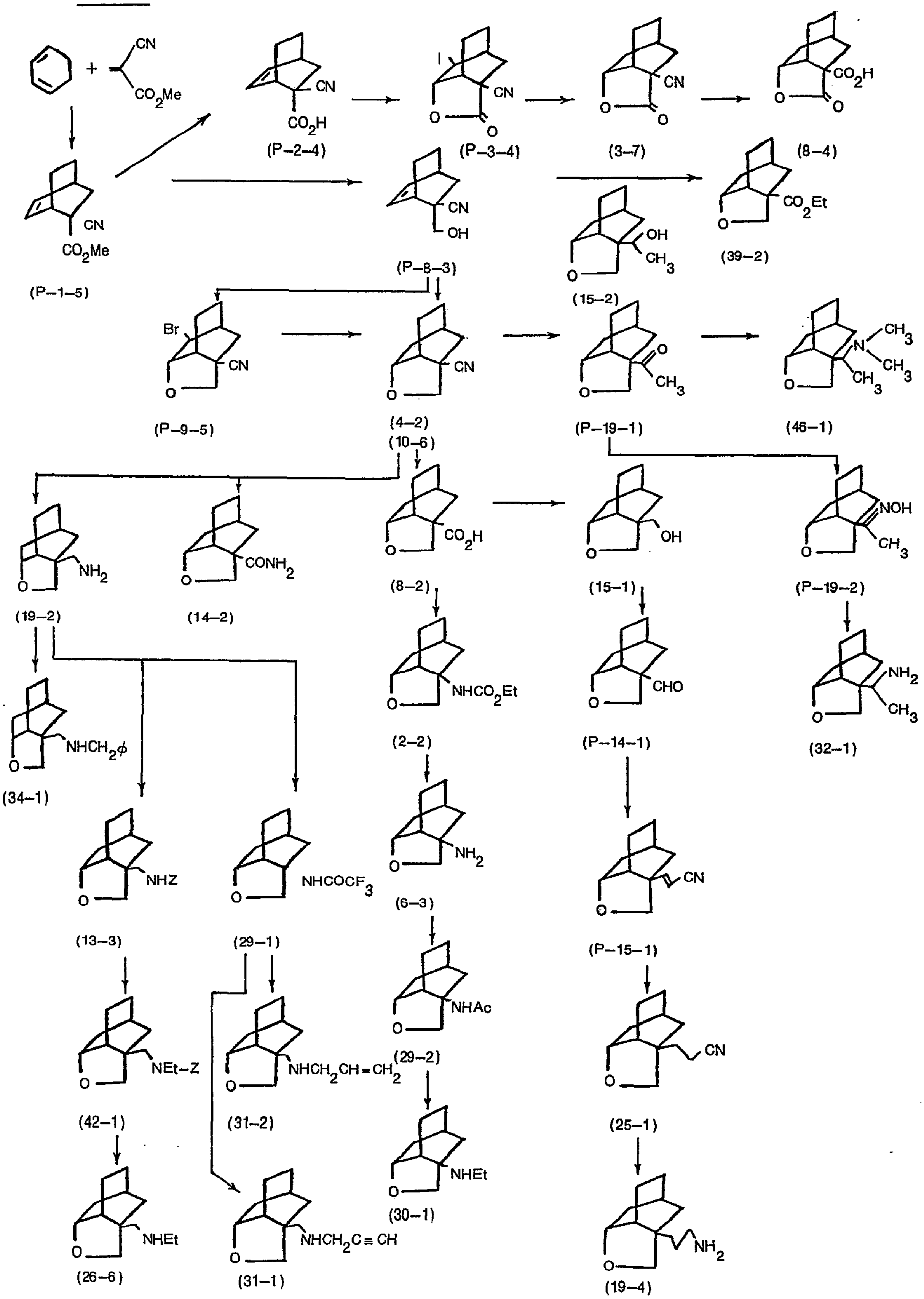

(P-2-4) (P-3-4) (3-7) (8-4)

(P-1-5) (P-8-3) (15-2) (39-2)

(P-9-5) (4-2) (10-6) (P-19-1) (46-1)

(8-2) (15-1) (P-19-2)

(19-2) (14-2) (2-2) (P-14-1) (32-1)

(34-1) (6-3)

(13-3) (29-1) (P-15-1)

(29-2)

(42-1) (31-2) (25-1)

(30-1)

(26-6) (31-1) (19-4)

CHART F

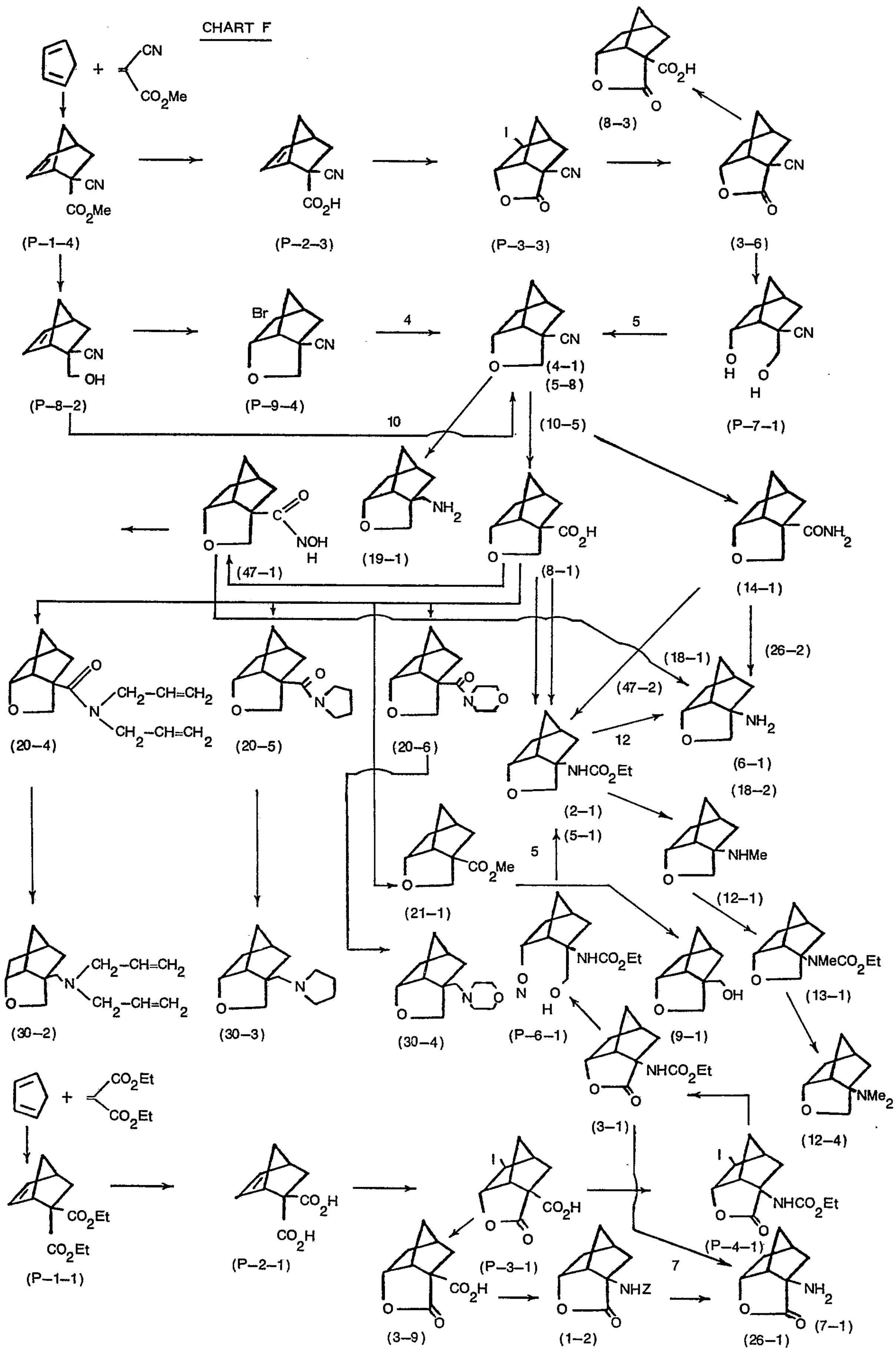


(8–3) (P–1–4) (P–2–3) (P–3–3) (3–6) (P–8–2) (P–9–4) (4–1) (5–8) (P–7–1) (10–5) (47–1) (19–1) (8–1) (14–1) (18–1) (26–2) (47–2) (20–4) (20–5) (20–6) (6–1) (18–2) (2–1) (5–1) (21–1) (12–1) (13–1) (30–2) (30–3) (30–4) (P–6–1) (9–1) (3–1) (12–4) (P–1–1) (P–2–1) (P–3–1) (P–4–1) (3–9) (1–2) (26–1) (7–1)

CHART G

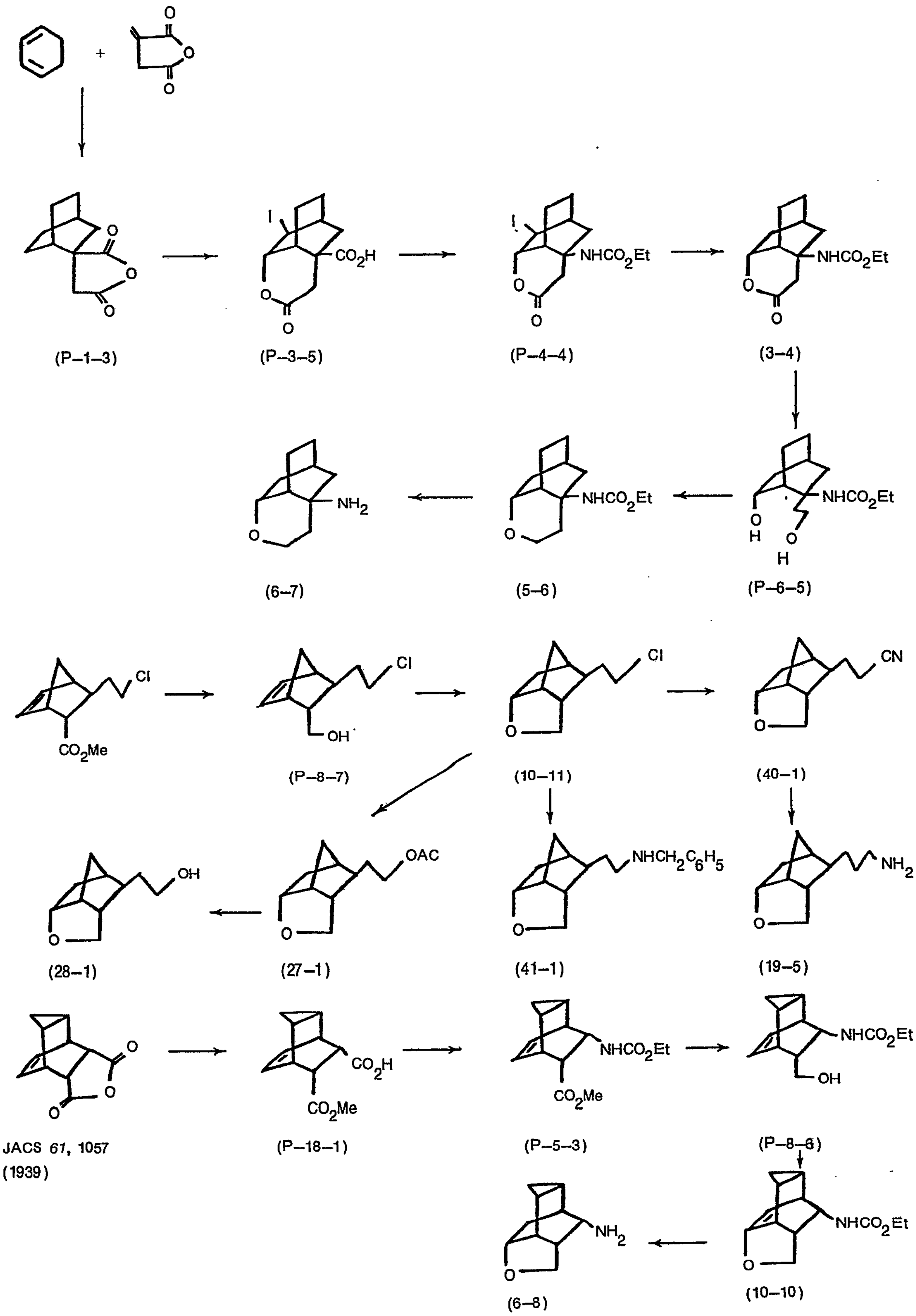

(P-1-3) (P-3-5) (P-4-4) (3-4)

(6-7) (5-6) (P-6-5)

(P-8-7) (10-11) (40-1)

(28-1) (27-1) (41-1) (19-5)

JACS *61*, 1057 (1939) (P-18-1) (P-5-3) (P-8-6)

(6-8) (10-10)

CHART H

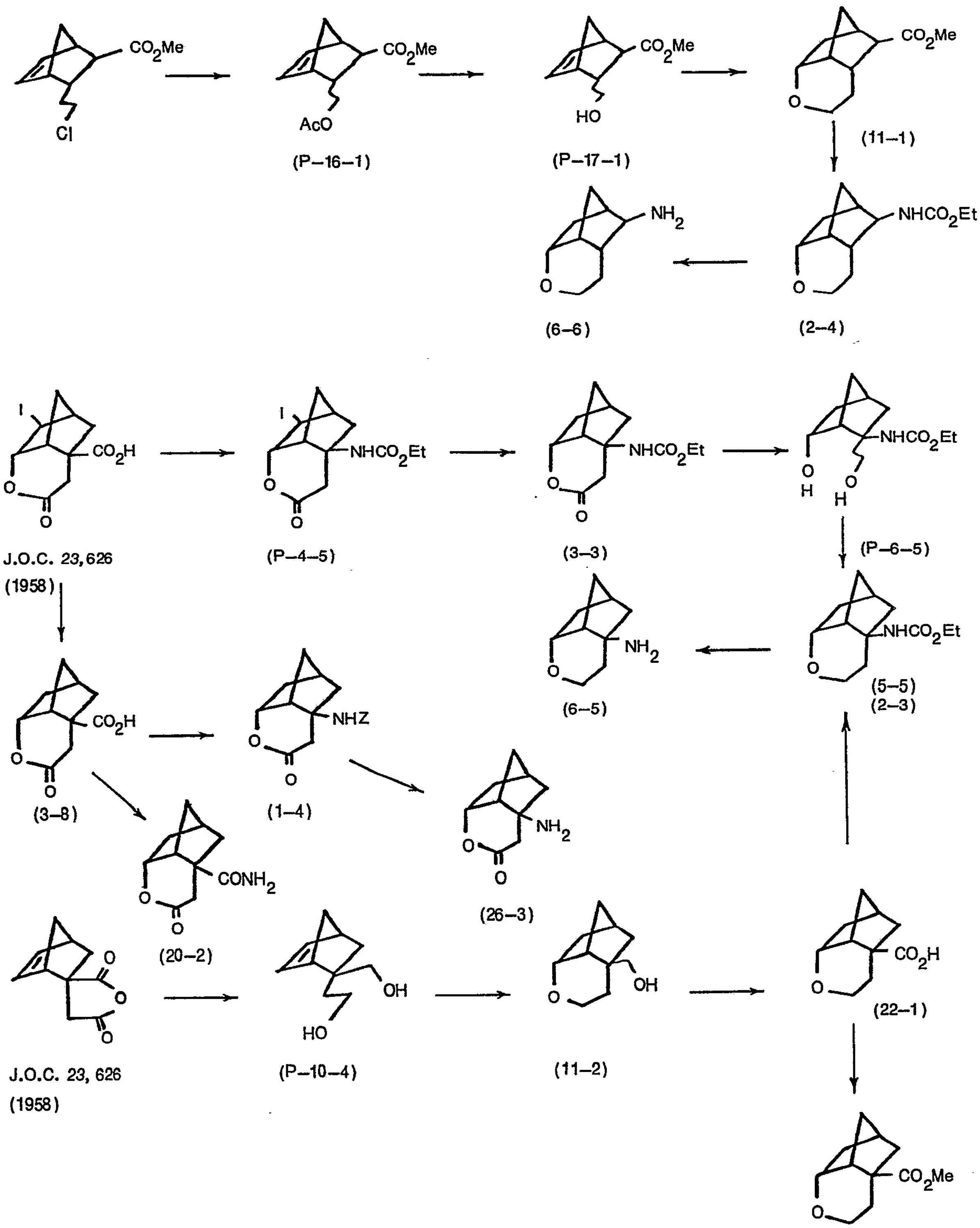

(P–16–1)

(P–17–1)

(11–1)

(6–6)

(2–4)

J.O.C. 23, 626 (1958)

(P–4–5)

(3–3)

(P–6–5)

(6–5)

(5–5)
(2–3)

(3–8)

(1–4)

(26–3)

(20–2)

J.O.C. 23, 626 (1958)

(P–10–4)

(11–2)

(22–1)

(21–2)

CHART I

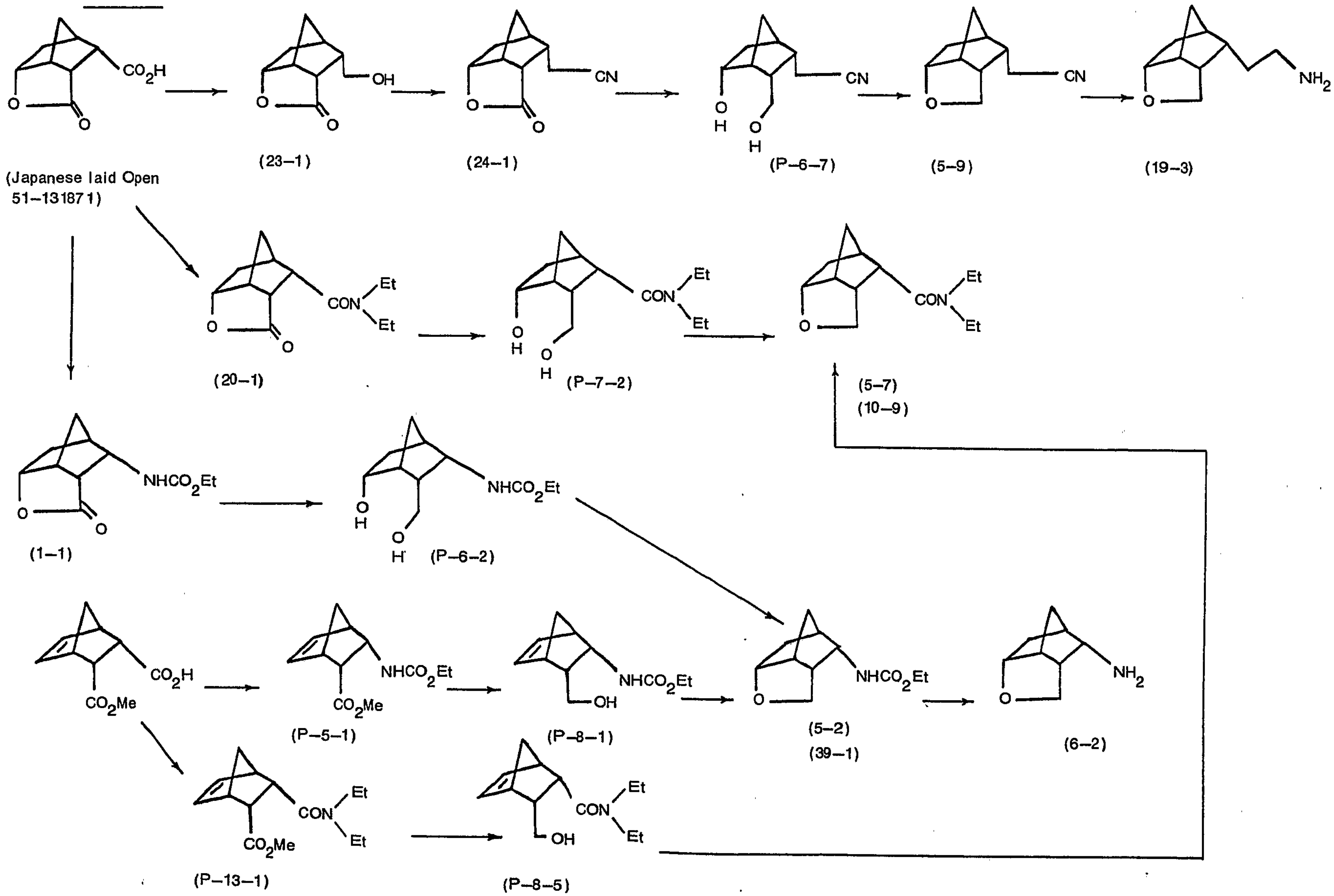
(Japanese laid Open
51–131871)
(23–1)
(24–1)
(P–6–7)
(5–9)
(19–3)
(20–1)
(P–7–2)
(5–7)
(10–9)
(1–1)
(P–6–2)
(P–5–1)
(P–8–1)
(5–2)
(39–1)
(6–2)
(P–13–1)
(P–8–5)

CHART J

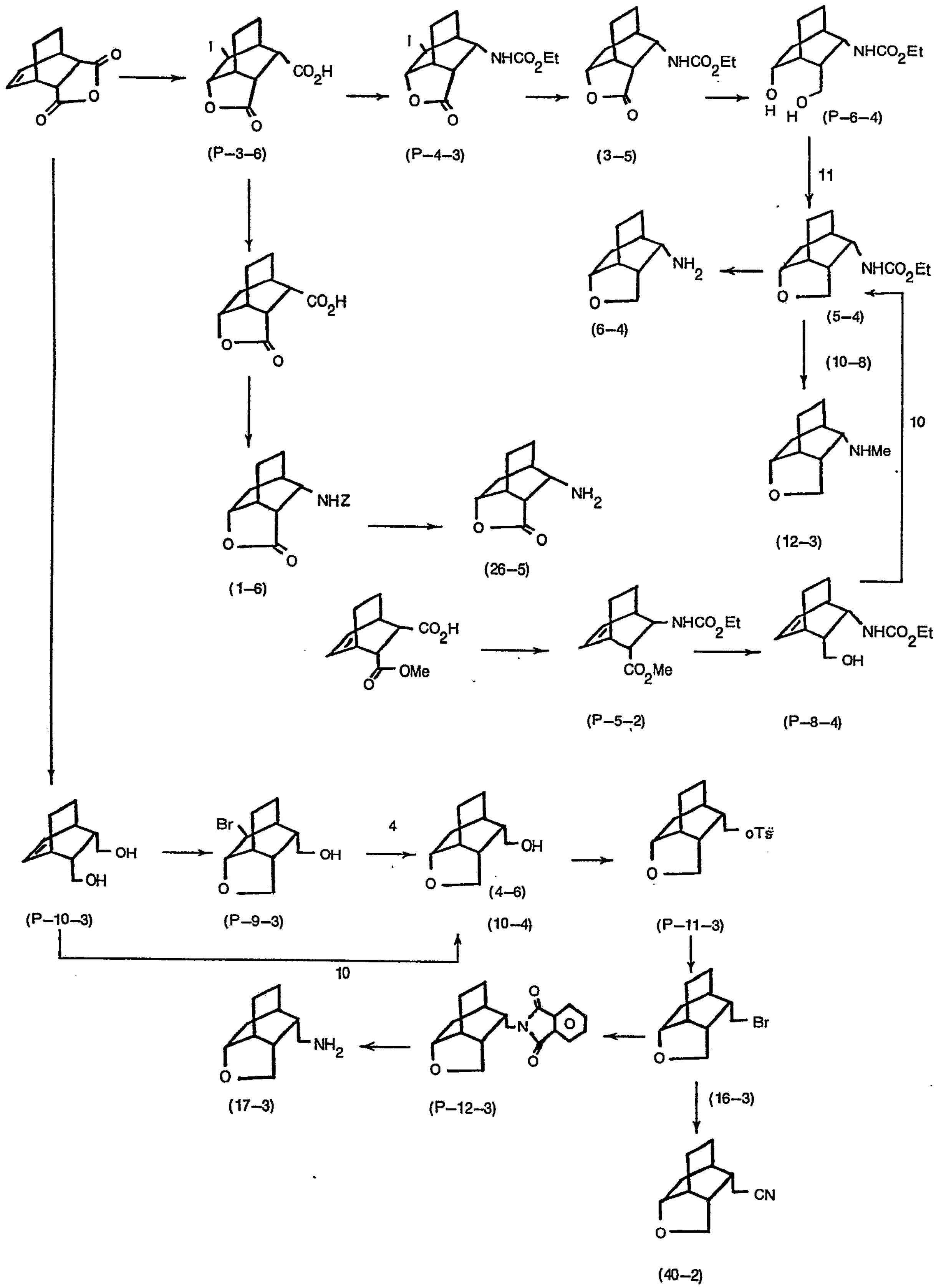


(P–3–6) (P–4–3) (3–5) (P–6–4)

(6–4) (5–4)

(12–3)

(1–6) (26–5)

(P–5–2) (P–8–4)

(P–10–3) (P–9–3) (4–6) (10–4) (P–11–3)

(17–3) (P–12–3)

(40–2)

CHART K

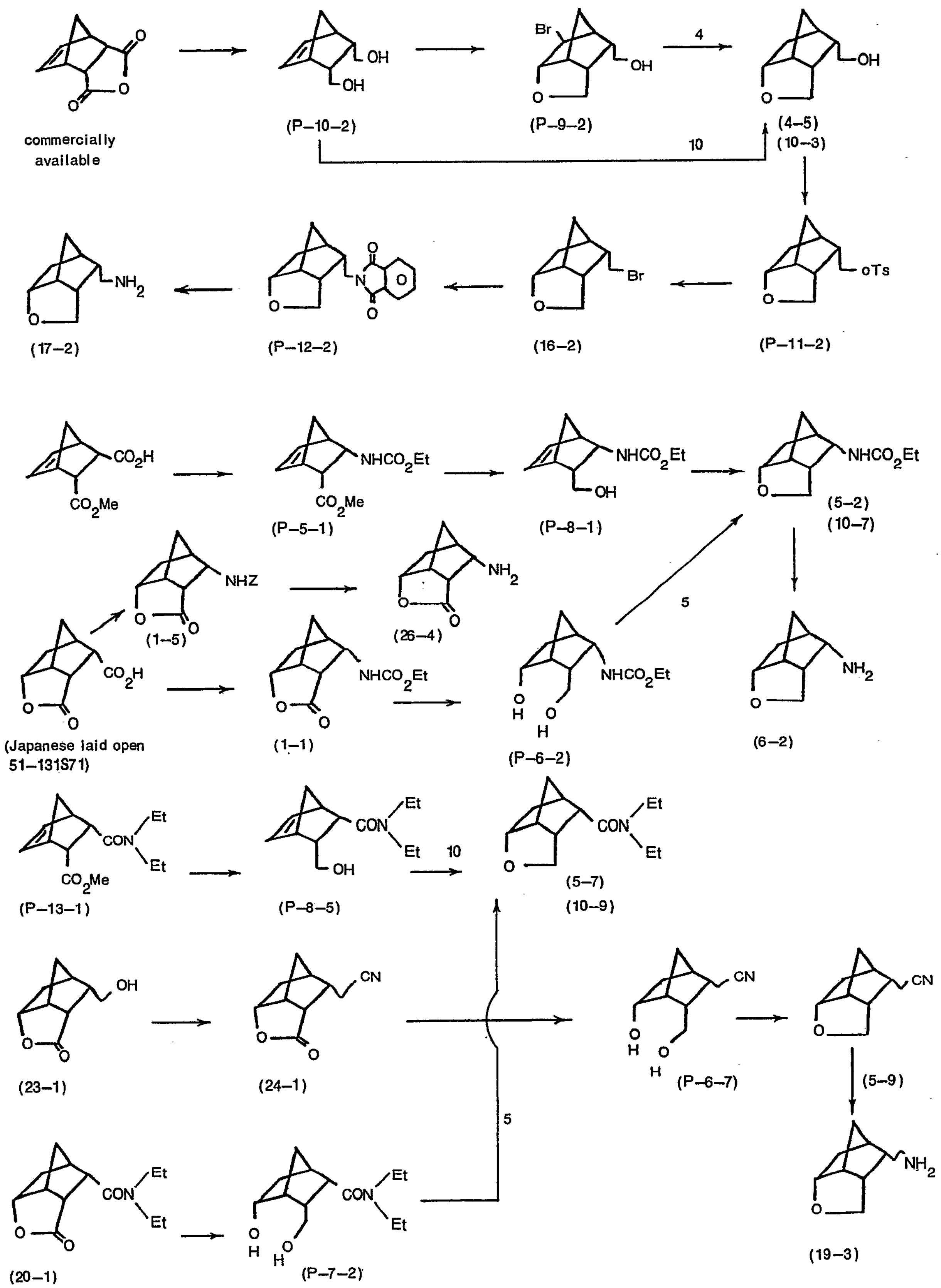


commercially available

(P–10–2)

(P–9–2)

(4–5)
(10–3)

(17–2)

(P–12–2)

(16–2)

(P–11–2)

(P–5–1)

(P–8–1)

(5–2)
(10–7)

(1–5)

(26–4)

(Japanese laid open 51–131S71)

(1–1)

(P–6–2)

(6–2)

(P–13–1)

(P–8–5)

(5–7)
(10–9)

(23–1)

(24–1)

(P–6–7)

(5–9)

(19–3)

(20–1)

(P–7–2)

CHART L

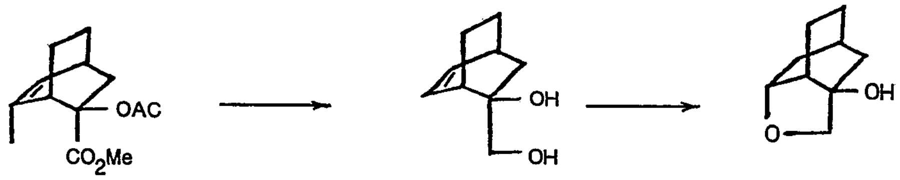

(THL, 5179 (1968))

(P–10–5)

(10–2)

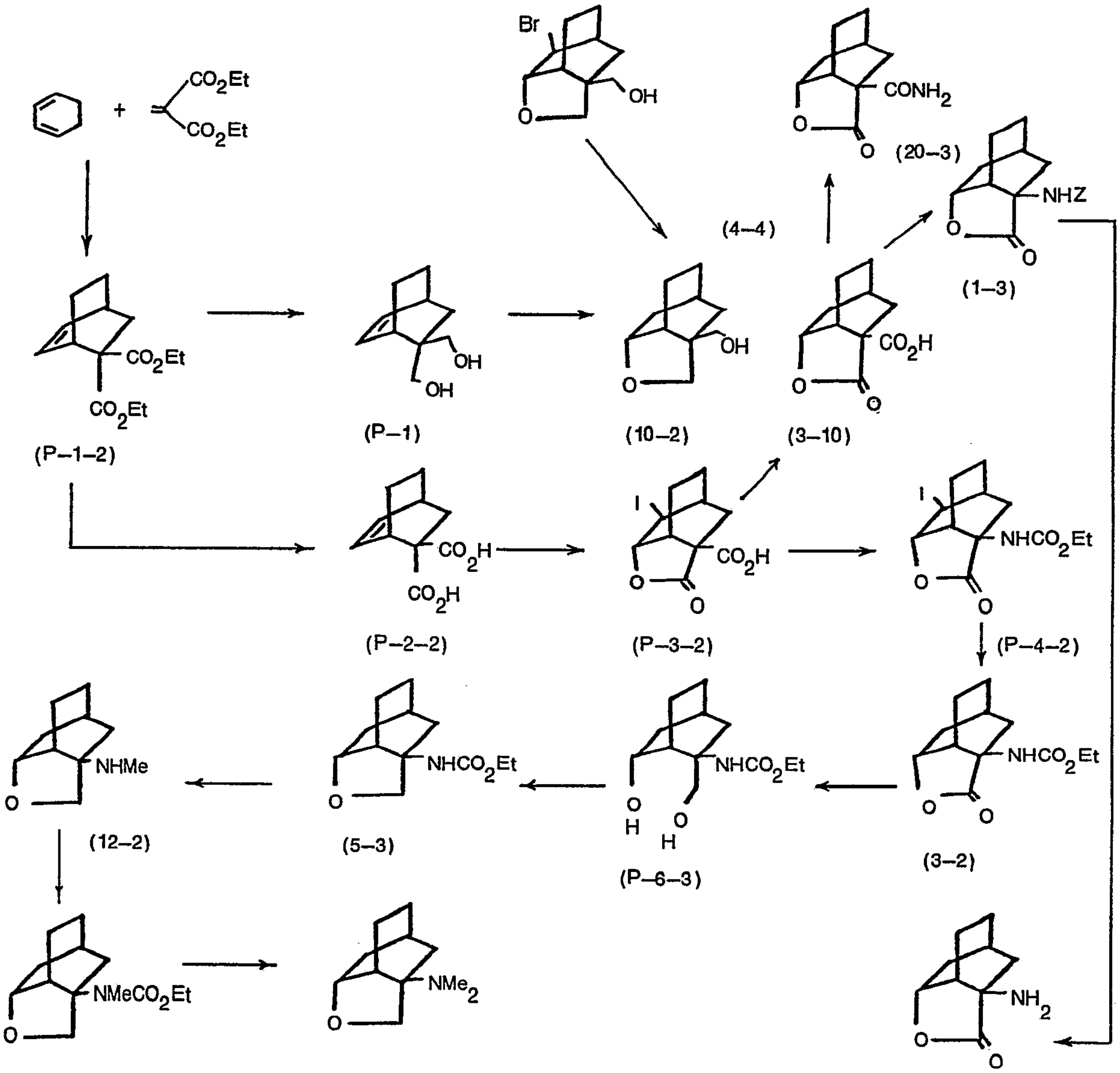

(20–3)

(4–4)

(1–3)

(P–1–2)

(P–1)

(10–2)

(3–10)

(P–2–2)

(P–3–2)

(P–4–2)

(12–2)

(5–3)

(P–6–3)

(3–2)

(13–2)

(12–5)

(26–2)

CHART M

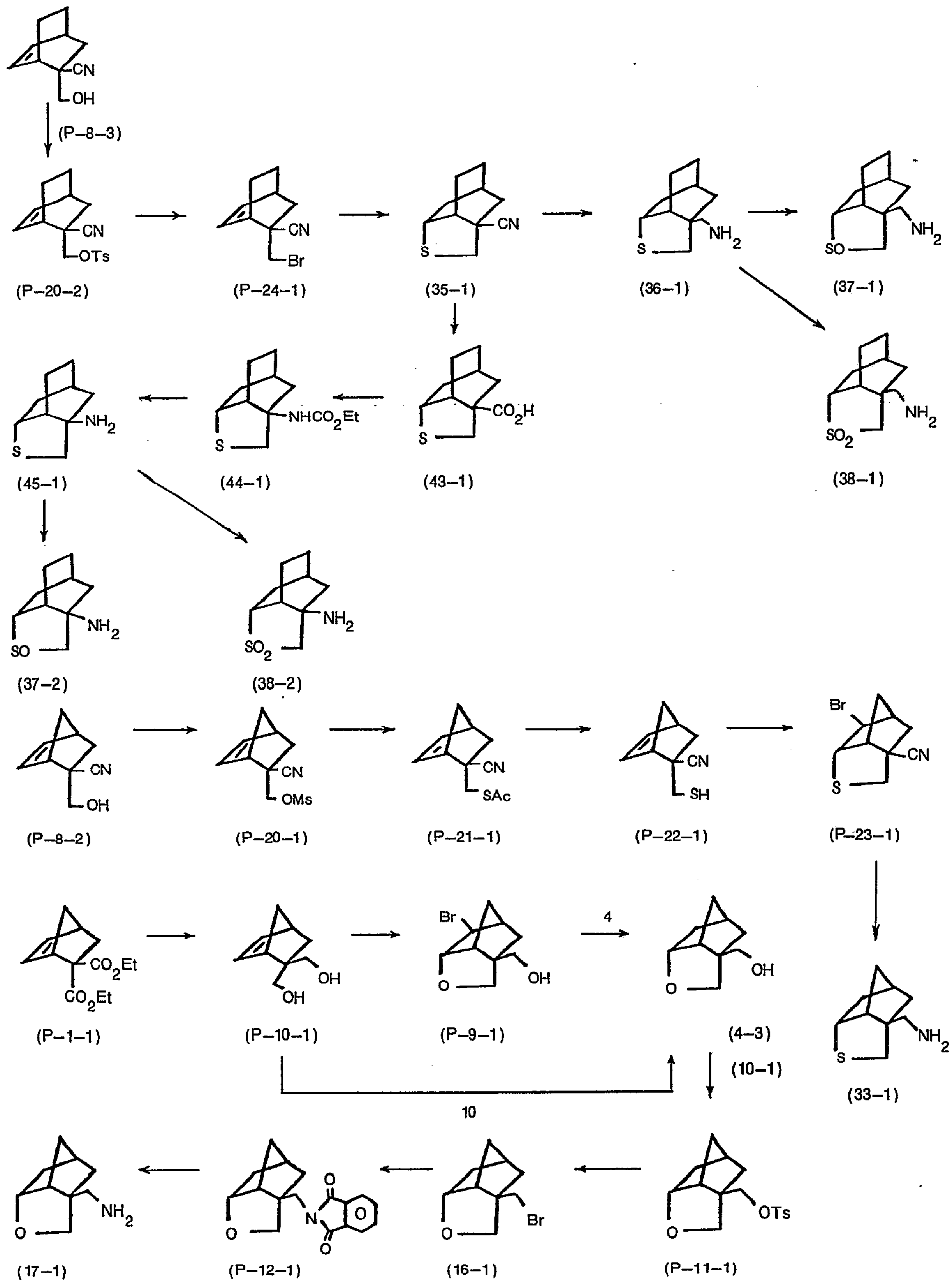


(P-20-2) (P-24-1) (35-1) (36-1) (37-1)

(45-1) (44-1) (43-1) (38-1)

(37-2) (38-2)

(P-8-2) (P-20-1) (P-21-1) (P-22-1) (P-23-1)

(P-1-1) (P-10-1) (P-9-1) (4-3) (33-1)

(17-1) (P-12-1) (16-1) (P-11-1)

**Claims**

1. A pharmaceutical composition which comprises together with a pharmaceutically acceptable inert carrier or diluent as an active ingredient a pharmaceutically effective amount of at least one of the compounds of the general formula:

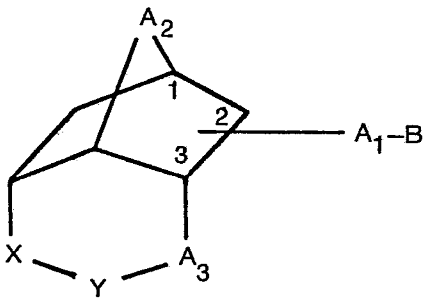

wherein $A_1$ is a single bond or a $C_1$—$C_5$ alkylene or a $C_2$—$C_5$ alkylidene group bonding at the 2- or 3-position, $A_2$ is a $C_1$—$C_3$ alkylene, cyclopropylene or $C_2$—$C_3$ alkylidene group; $A_3$ is a single bond or a methylene group, X—Y is —O—CH—$_2$, —O—CO— or —$SO_n$—$CH_2$— (wherein n is 0 or an integer of 1 or 2) and B is a carboxy group, a $C_2$—$C_5$ alkoxycarbonyl group, a cyano group, a hydroxy group, a $C_2$—$C_5$ alkanoyloxy group, a halogen atom, a carbamoyl group of the formula:

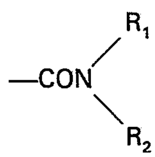

(wherein $R_1$ and $R_2$ are each a hydrogen atom, a $C_1$—$C_5$ alkyl group or a $C_3$—$C_5$ alkenyl group, or, when taken together with the adjacent nitrogen atom may represent a pyrrolidino, piperazino or morpholino group), or an amino group of the formula:

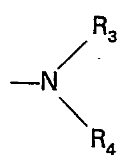

(wherein $R_3$ is a hydrogen atom, a $C_1$—$C_5$ alkyl group, a $C_3$—$C_5$ alkenyl group, a $C_3$—$C_5$ alkynyl group or a $C_7$—$C_9$ aralkyl group and $R_4$ is a hydrogen atom, a $C_1$—$C_5$ alkyl group, a $C_3$—$C_5$ alkenyl group, a $C_3$—$C_5$ alkynyl group, a $C_7$—$C_9$ aralkyl group, a $C_2$—$C_5$ alkoxycarbonyl group, a benzyloxycarbonyl group, a $C_2$—$C_5$ alkanoyl group or a $C_2$—$C_4$ haloalkanoyl group or, when taken together with the adjacent nitrogen atom, $R_3$ and $R_4$ may represent a pyrrolidino, piperazino or morpholino group), and non-toxic salts thereof, provided

that when $A_1$ is a single bond, $A_2$ is a methylene group or an ethylene group, $A_3$ is a single bond, X—Y is —O—CO—, B is a carboxy group, and $A_1$ is bonded at the 3-position;

that when X—Y is —O—$CH_2$—, $A_3$ is a single bond and $A_1$ is a methylene group then B is not a hydroxy group;

and that the alkenyl and alkynyl groups do not contain a double or a triple bond at the 1-position.

2. A compound of the formula:

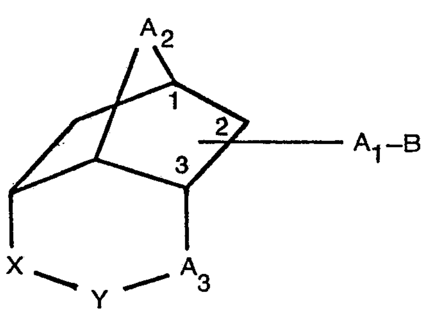

and a non-toxic salt thereof wherein $A_1$ is a single bond or a $C_1$—$C_5$ alkylene or $C_2$—$C_5$ alkylidene group bonding at the 2- or 3-position, $A_2$ is a $C_1$—$C_3$ alkylene, cyclopropylene or $C_2$—$C_3$ alkylidene group, $A_3$ is a single bond or a methylene group, X—Y is —O—$CH_2$—, —O—CO— or —$SO_n$—$CH_2$—

(wherein n is 0 or an integer of 1 or 2) and B is a carboxy group, a $C_2$—$C_5$ alkoxycarbonyl group, a cyano group, a halogen atom, a carbamoyl group of the formula:

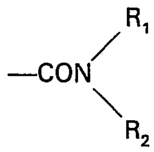

(wherein $R_1$ and $R_2$ are each a hydrogen atom, a $C_1$—$C_5$ alkyl group or a $C_3$—$C_5$ alkenyl group or, when taken together with the adjacent nitrogen atom, may represent a pyrrolidino, piperazino or morpholino group), or an amino group of the formula:

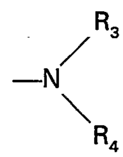

(wherein $R_3$ is a hydrogen atom, a $C_1$—$C_5$ alkyl group, a $C_3$—$C_5$ alkenyl group, a $C_3$—$C_5$ alkynyl group or a $C_7$—$C_9$ aralkyl group and $R_4$ is a hydrogen atom, a $C_1$—$C_5$ alkyl group, a $C_3$—$C_5$ alkenyl group, a $C_3$—$C_5$ alkynyl group, a $C_7$—$C_9$ aralkyl group, a $C_2$—$C_5$ alkoxycarbonyl group, a benzyloxycarbonyl group, a $C_2$—$C_5$ alkanoyl group or a $C_2$—$C_4$ haloalkanoyl group or, when taken together with the adjacent nitrogen atom, $R_3$ and $R_4$ may represent a pyrrolidino, piperazino or morpholino group), provided

that when X—Y is —O—CO—, B is an amino group of the formula:

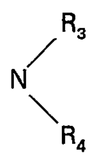

and that the alkenyl and alkynyl groups do not contain a double or a triple bond at the 1-position.

3. A compound of the formula:

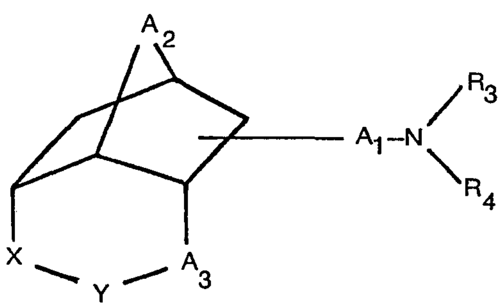

wherein $A_1$, $A_2$, $A_3$, X—Y, $R_3$ and $R_4$ are as defined in claim 2, or a non toxic-salt thereof.

4. A compound of the formula:

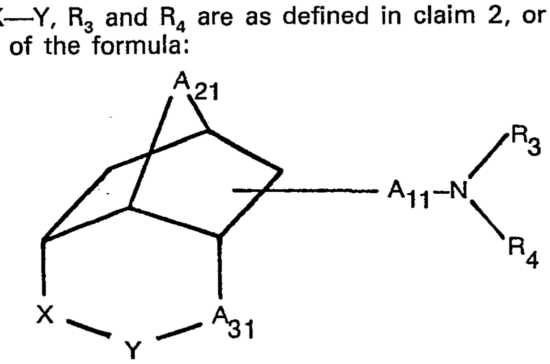

wherein $A_{11}$ is a single bond or a methylene group bonding at the 2- or 3-position, $A_{21}$ is a methylene group or an ethylene group, $A_{31}$ is a single bond or a methylene group, X—Y is —O—$CH_2$—, —O—CO— or —$SO_n$—$CH_2$— (wherein n is an integer of 0 to 2), and $R_3$ and $R_4$ are as defined in claim 2, or a non-toxic salt thereof.

5. A compound of the formula:

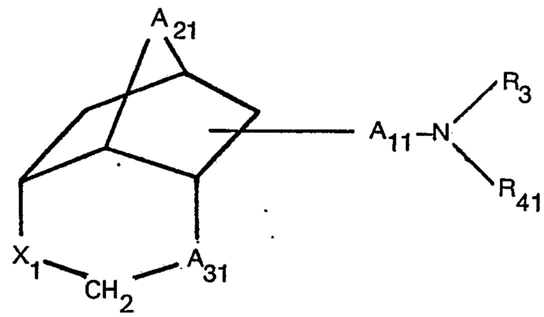

or a non-toxic salt thereof, wherein $A_{11}$, $A_{21}$, $A_{31}$, $R_{41}$ and $R_3$ are as defined in claim 4 and $R_{41}$ is a hydrogen atom, a $C_1$—$C_5$ alkyl group or a $C_2$—$C_5$ alkoxycarbonyl group and $X_1$ is —O— or —S—.

6. A compound of the formula:

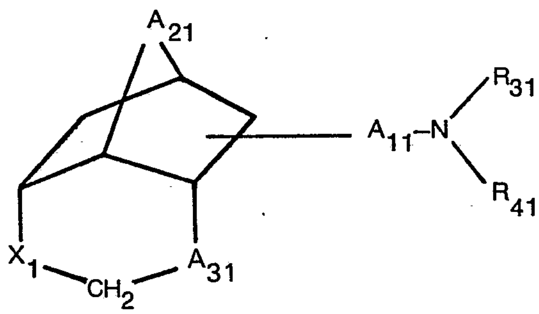

wherein $A_{11}$, $A_{21}$, $A_{31}$, $R_{41}$ and $X_1$ are as defined in claim 5 and $R_{31}$ is a hydrogen atom or a $C_1$—$C_5$ alkyl group; or a non-toxic salt thereof.

7. A compound of the formula:

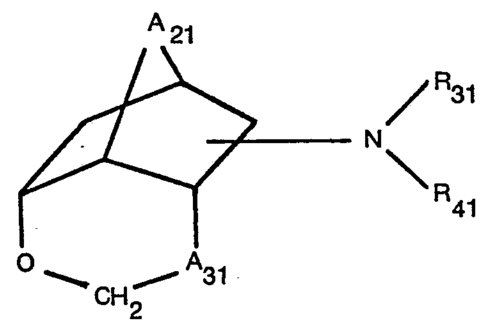

wherein $A_{21}$, $A_{31}$, $R_{31}$ and $R_{41}$ are as defined in claim 6, or a non-toxic salt thereof.

8. A compound of the formula:

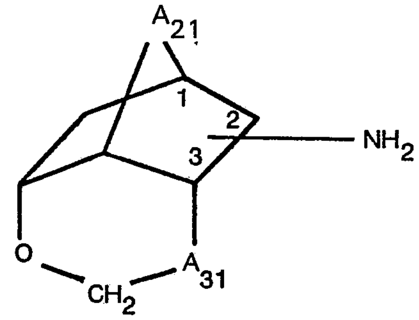

wherein $A_{21}$ is a methylene group or an ethylene group, $A_{31}$ is a single bond or a methylene group and the amino group is bonded at the 2- or 3-position, or a non-toxic salt thereof.

9. A compound of the formula:

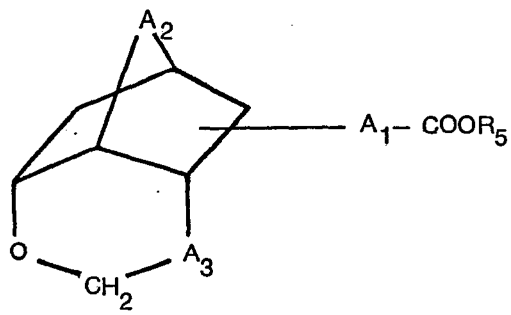

wherein $A_1$, $A_2$ and $A_3$ are as defined in claim 2 and $R_5$ is a hydrogen atom or a $C_1$—$C_4$ alkyl group, or a non-toxic salt thereof.

10. A compound of the formula:

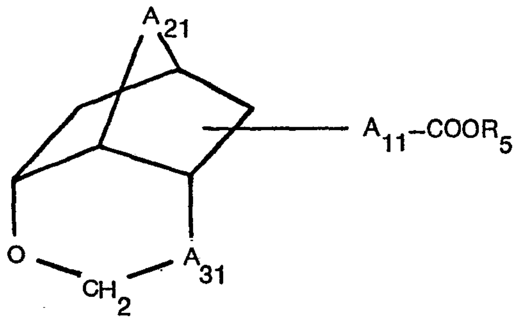

wherein $A_{11}$, $A_{21}$ and $A_{31}$ are as defined in claim 4 and $R_5$ is a hydrogen atom or a $C_1$—$C_4$ alkyl group, or a non-toxic salt thereof.

11. A compound of the formula:

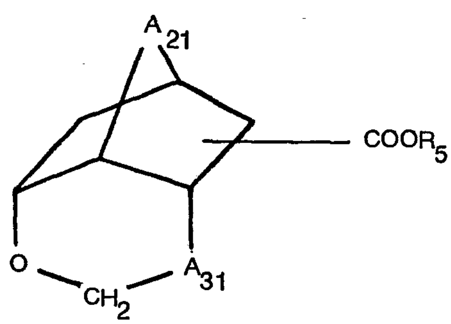

wherein $A_{21}$, $A_{31}$ and $R_5$ are as defined in claim 10 or a non-toxic salt thereof.

12. A process for producing a compound as claimed in claim 2 of the formula:

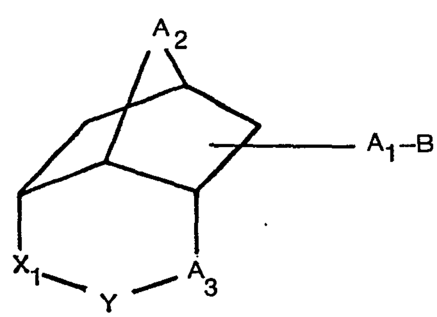

wherein $A_1$, $A_2$, $A_3$ and B are each as defined in claim 2 and $X_1$—Y is —O—$CH_2$—, —S—$CH_2$— or

—O—CO—, which comprises cyclizing a compound of the formula:

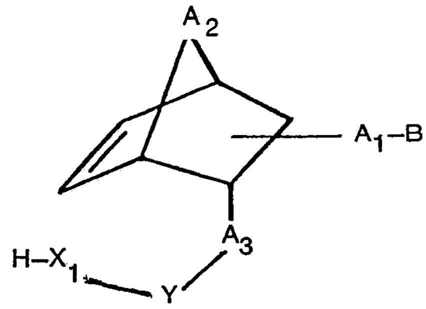

wherein $A_1$, $A_2$, $A_3$, B and $X_1$—Y are each as defined above.

13. A process for producing a compound as claimed in claim 2 of the formula:

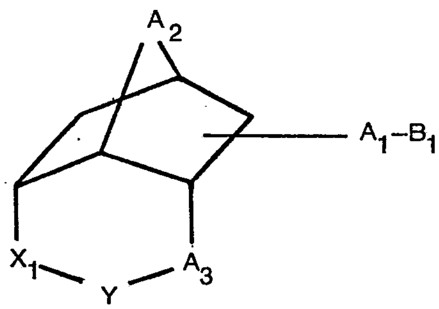

wherein $A_1$, $A_2$ and $A_3$ are each as defined in claim 2 and $B_1$ is the same as B excluding a halogen atom and $X_1$—Y is —O—$CH_2$—, —S—$CH_2$— or —O—CO—, which comprises reducing a compound of the formula:

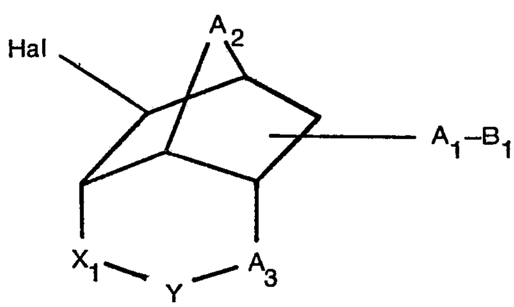

wherein $A_1$, $A_2$, $A_3$, $B_1$ and $X_1$—Y are each as defined above and Hal is a halogen atom.

14. A process for producing a compound as claimed in claim 2 of the formula:

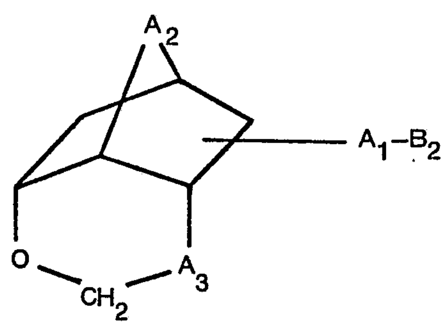

wherein $A_1$, $A_2$ and $A_3$ are each as defined in claim 2 and $B_2$ is a cyano group, a carbamoyl group of the formula:

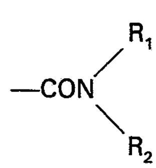

(wherein $R_1$ and $R_2$ are each as defined in claim 2) or an amino group of the formula:

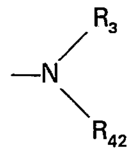

(wherein $R_3$ is as defined in claim 2 and $R_{42}$ is a $C_2$—$C_5$ alkoxycarbonyl group, a benzyloxycarbonyl group, a $C_2$—$C_5$ alkanoyl group or a $C_2$—$C_4$ haloalkanoyl group), which comprises reacting a compound of the formula:

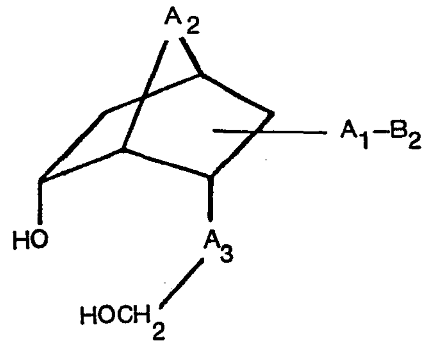

wherein $A_1$, $A_2$, $A_3$ and $B_2$ are each as defined above, with a dehydrating agent.

15. A process for producing a compound as claimed in claim 2 of the formula:

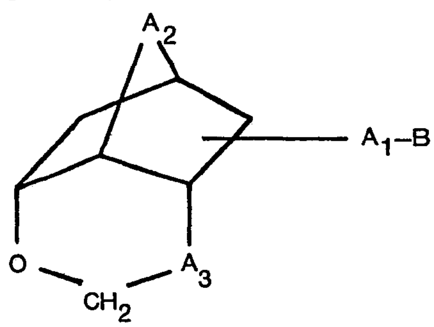

wherein $A_1$, $A_2$, $A_3$ and B are each as defined in claim 2 which comprises cyclizing a compound of the formula:

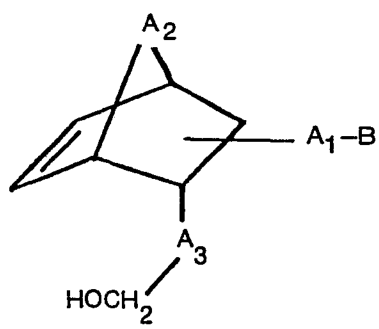

wherein $A_1$, $A_2$, $A_3$ and B are each as defined above, via a oxymercuration-demercuration reaction.

16. A process for producing a compound as claimed in claim 2 of the formula:

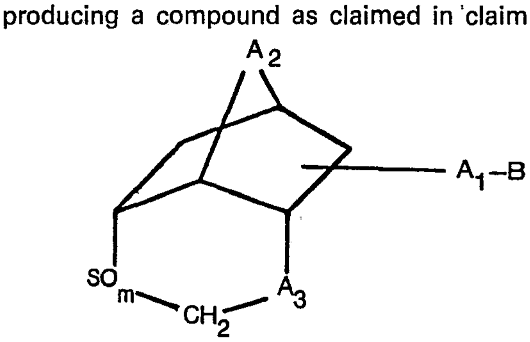

wherein $A_1$, $A_2$, $A_3$ and B are each as defined in claim 2 and m is an integer of 1 or 2, which comprises oxidizing a compound of the formula:

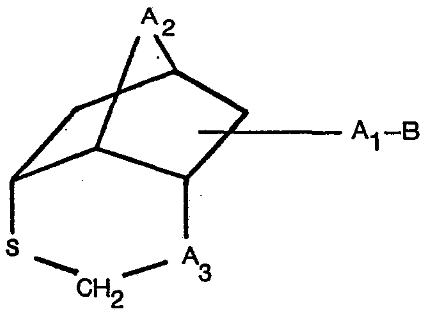

wherein $A_1$, $A_2$, $A_3$ and B are each as defined above.

17. A process for producing a compound as claimed in claim 2 of the formula:

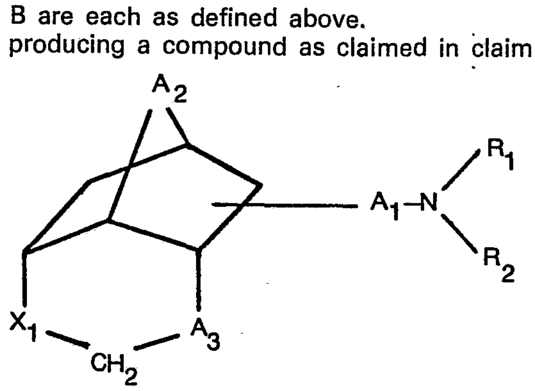

wherein $A_1$, $A_2$, $A_3$, $R_1$ and $R_2$ are each as defined in claim 2 and $X_1$ is —O— or —S—, which comprises reducing

a) a compound of the formula:

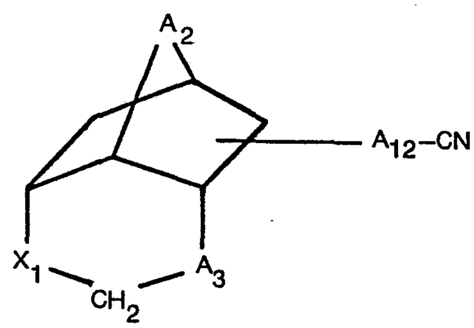

wherein $A_2$ and $A_3$ are each as defined above, $A_{12}$ is a single bond or a $C_1$—$C_4$ alkylene group and $X_1$ is —O— or —S—, with a metal hydride compound or hydrogen gas in the presence of a catalyst;

b) a compound of the formula:

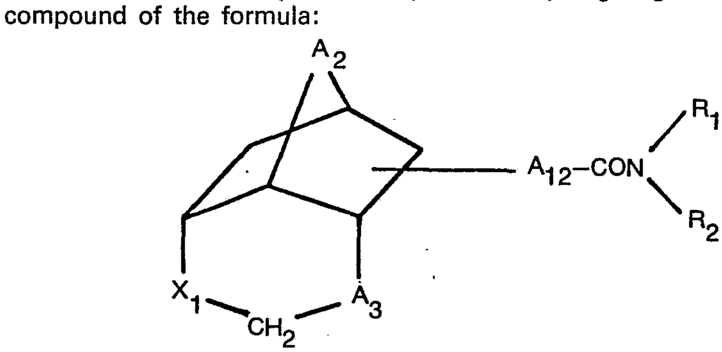

wherein $A_{12}$, $A_2$, $A_3$, $R_1$, $R_2$ and $X_1$ are each as defined above, with a metal hydride compound;

c) a compound of the formula:

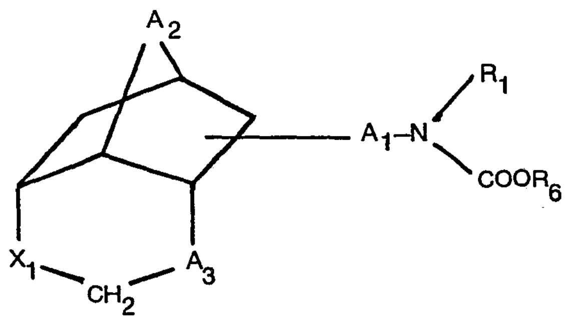

wherein $A_1$, $A_2$, $A_3$, $X_1$ and $R_1$ are each as defined above and $R_6$ is a $C_1$—$C_4$ alkyl group, with a metal hydride compound;

d) a compound of the formula:

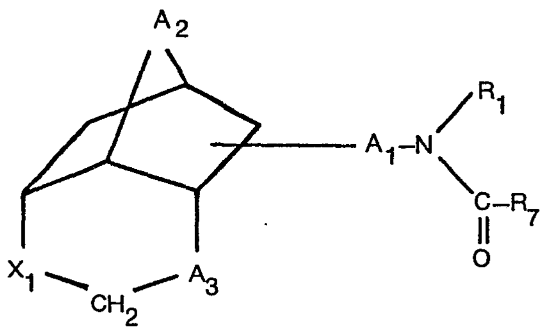

wherein $A_1$, $A_2$, $A_3$, $X_1$ and $R_1$ are each as defined above and $R_7$ is a hydrogen atom, a $C_1$—$C_4$ alkyl group or a $C_2$—$C_4$ alkenyl group, with a metal hydride compound;

e) a compound of the formula:

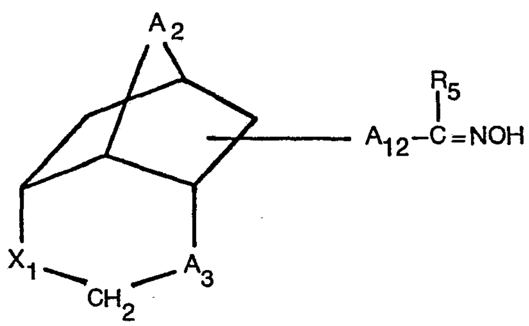

wherein $A_{12}$, $A_2$, $A_3$ and $X_1$ are each as defined above and $R_5$ is a hydrogen atom or a $C_1$—$C_4$ alkyl group, with a metal hydride compound or hydrogen gas in the presence of a catalyst; or

f) a compound of the formula:

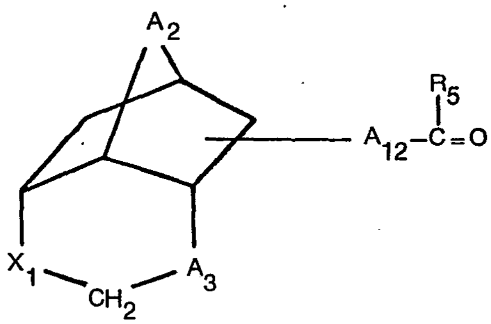

wherein $A_{12}$, $A_2$, $A_3$, $R_5$ and $X_1$ are each as defined above, with a reducing agent in the presence of an amine compound of the formula:

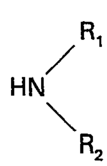

wherein $R_1$ and $R_2$ are each as defined above.

18. A process for producing a compound as claimed in claim 2 of the formula:

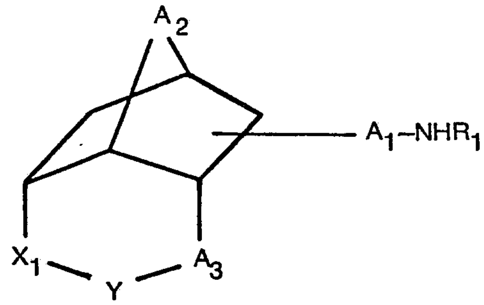

wherein $A_1$, $A_2$, $A_3$ and $R_1$ are each as defined in claim 2 and $X_1$—Y is —O—$CH_2$—, —S—$CH_2$— or —O—CO—, which comprises debenzylation and decarboxylation of the compound of the formula:

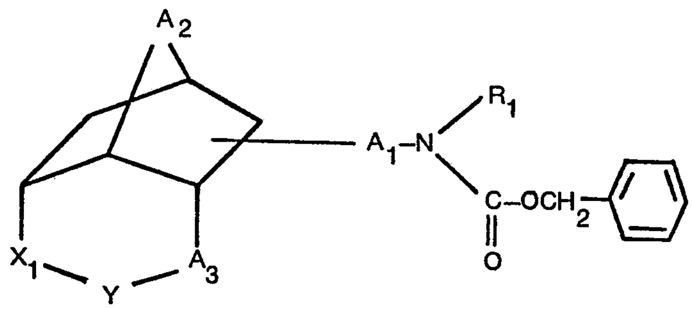

wherein $A_1$, $A_2$, $A_3$, $R_1$ and $X_1$—Y are each as defined above.

19. A process for producing compounds as claimed in claim 2 of the formulae:

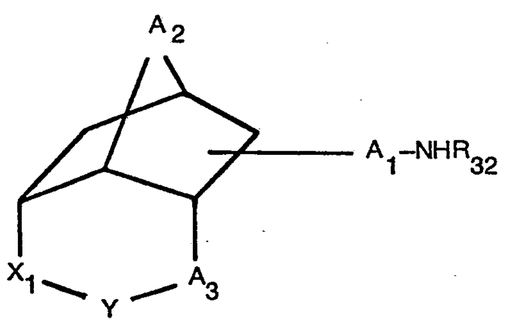

;

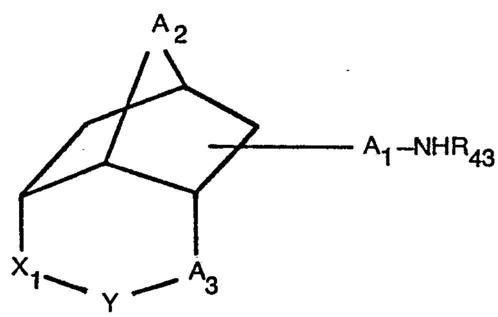

and

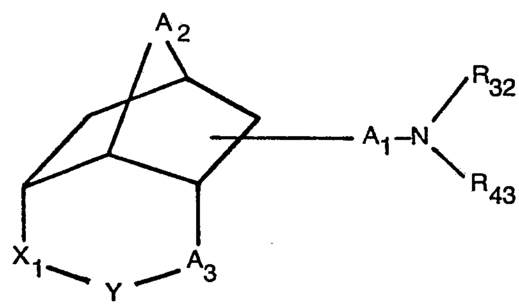

wherein $A_1$, $A_2$ and $A_3$ are each as defined in claim 2, $X_1$—Y is —O—$CH_2$—, —S—$CH_2$— or —O—CO—, $R_{32}$ is the same as $R_3$ in claim 2 excluding a hydrogen atom and $R_{43}$ is the same as $R_4$ in

claim 2 excluding a hydrogen atom, which comprises reacting a compound of the formula:

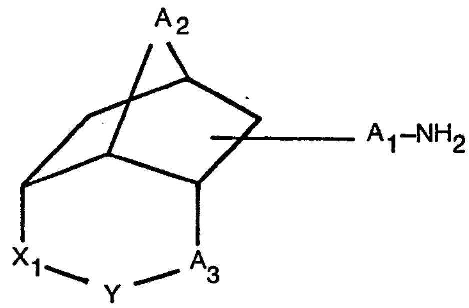

wherein $A_1$, $A_2$, $A_3$ and $X_1$—Y are each as defined above, with $R_{32}$—Z or $R_{43}$—Z (wherein Z is a strong acid residue).

20. A process for producing a compound as claimed in claim 2 of the formula:

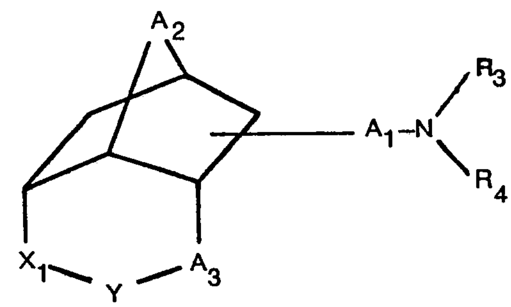

wherein $A_1$, $A_2$, $A_3$, $R_3$ and $R_4$ are each as defined in claim 2 and $X_1$—Y is —O—$CH_2$—, —S—$CH_2$— or —O—CO—, which comprises reacting a compound of the formula:

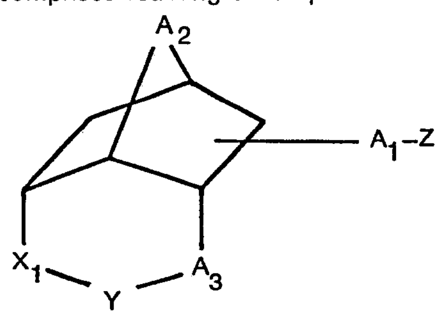

wherein $A_1$, $A_2$, $A_3$ and $X_1$—Y are each as defined above and Z is the residue of a strong acid, with an amine compound of the formula:

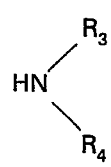

wherein $R_3$ and $R_4$ are each as defined above.

21. A process for producing a compound as claimed in claim 2 of the formula:

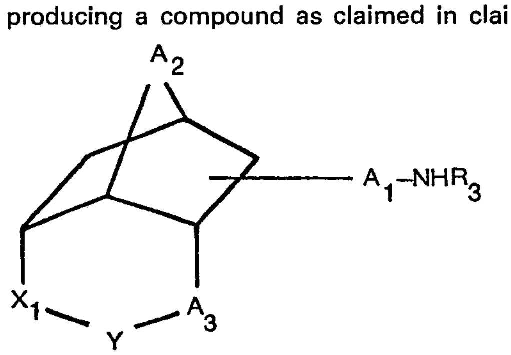

wherein $A_1$, $A_2$, $A_3$ and $R_3$ are each as defined in claim 2 and $X_1$—Y is —O—$CH_2$—, —S—$CH_2$ or —O—CO—, which comprises hydrolyzing a compound of the formula:

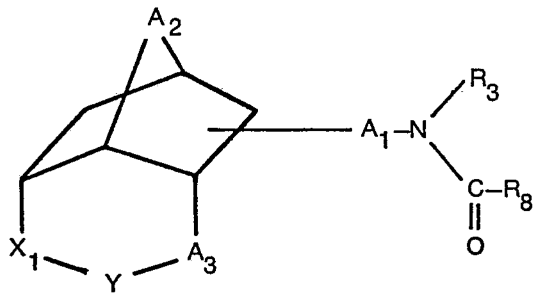

wherein $A_1$, $A_2$, $A_3$, $R_3$ and $X_1$—Y are each as defined above and $R_8$ is a hydrogen atom, a $C_1$—$C_4$ alkyl group, a $C_1$—$C_3$ haloalkyl group, a $C_1$—$C_4$ alkoxy group or a benzyloxy group.

22. A process for producing a compound as claimed in claim 2 of the formula:

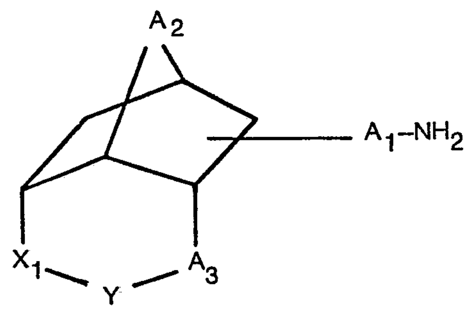

wherein $A_1$, $A_2$ and $A_3$ are each as defined in claim 2 and $X_1$—Y is —O—$CH_2$—, —S—$CH_2$— or —O—CO—, which comprises hydrolyzing a compound of the formula:

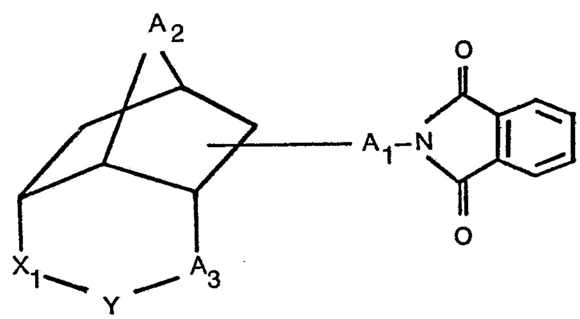

wherein $A_1$, $A_2$, $A_3$ and $X_1$—Y are each as defined above.

23. A process for producing a compound as claimed in claim 2 of the formula:

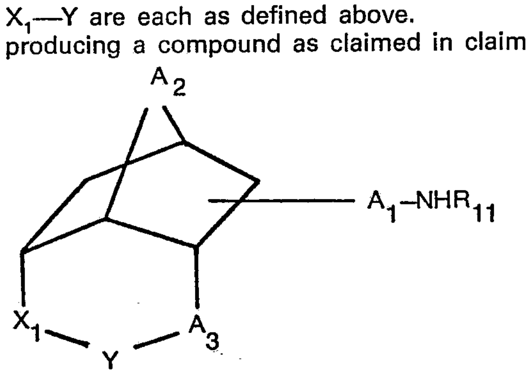

wherein $A_1$, $A_2$, $A_3$ are each as defined in claim 2 and $X_1$—Y is —O—$CH_2$—, —S—$CH_2$— or

—O—CO— and $R_{11}$ is a hydrogen atom, a $C_2$—$C_5$ alkyloxycarbonyl group or a benzyloxycarbonyl group, which comprises treatment of

a) a compound of the formula:

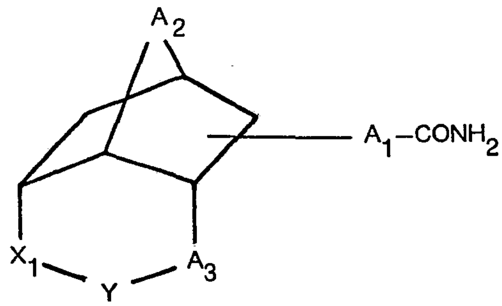

wherein $A_1$, $A_2$, $A_3$ and $X_1$—Y are each as defined above, with alkali hypohalite, followed by hydrolysis or treatment of the resulting isocyanate with an alcohol compound of the formula: $R_9$—OH (wherein $R_9$ is a $C_1$—$C_4$ alkyl group or a benzyl group);

b) a compound of the formula:

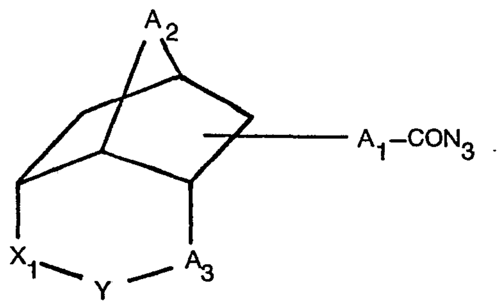

wherein $A_1$, $A_2$, $A_3$ and $X_1$—Y are each as defined above, under heating, followed by hydrolysis or treatment of the resulting isocyanates with an alcohol compound of the formula: $R_9$—OH (wherein $R_9$ is a $C_1$—$C_4$ alkyl group or a benzyl group);

c) a compound of the formula:

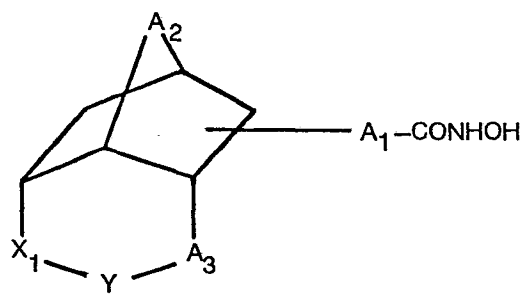

wherein $A_1$, $A_2$, $A_3$ and $X_1$—Y are each as defined above, under heating with or without a base, followed by hydrolysis or treatment of the resulting isocyanate with an alcohol compound of the formula: $R_9$—OH (wherein $R_9$ is a $C_1$—$C_4$ alkyl group or a benzyl group).

24. A process for producing a compound as claimed in claim 2 of the formula:

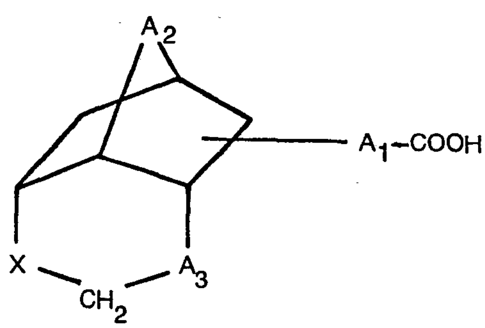

wherein $A_1$, $A_2$ and $A_3$ are each as defined in claim 2 and X is —O— or —$SO_n$— (n = 0, 1 or 2), which comprises hydrolysis of a compound of the formula:

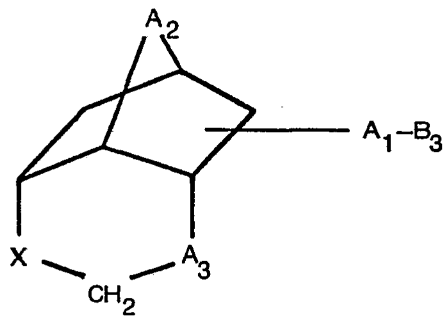

wherein $A_1$, $A_2$, $A_3$ and X are each as defined above and $B_3$ is a cyano group, a $C_2$—$C_5$ alkoxycarbonyl group, a benzyloxycarbonyl group or a carbamoyl group of the formula:

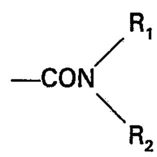

(wherein $R_1$ and $R_2$ are each as defined in claim 2).

25. A process for producing a compound as claimed in claim 2 of the formula:

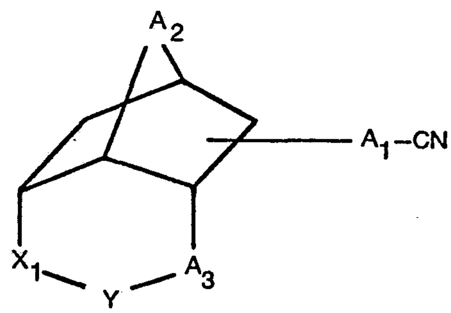

wherein $A_1$, $A_2$ and $A_3$ are each as defined in claim 2 and $X_1$—Y is —O—$CH_2$—, or —S—$CH_2$— which comprises reacting a compound of the formula:

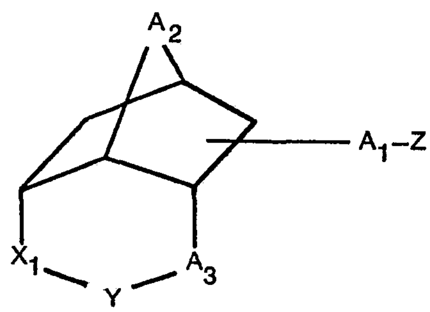

wherein $A_1$, $A_2$, $A_3$ and $X_1$—Y are each as defined above and Z is the residue of a strong acid, with a cyanide compound.

26. A process for producing a compound as claimed in claim 2 of the formula:

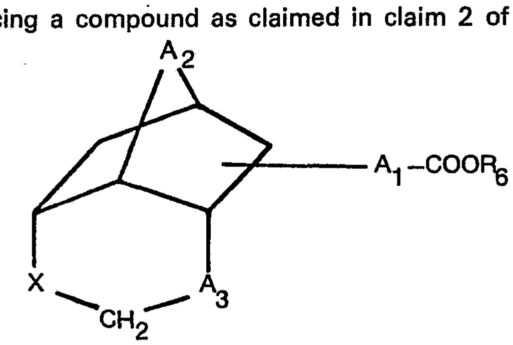

wherein $A_1$, $A_2$ and $A_3$ are each as defined in claim 2, X is —O— or —S— and $R_6$ is a $C_1$—$C_4$ alkyl group, which comprises reacting

a) a compound of the formula:

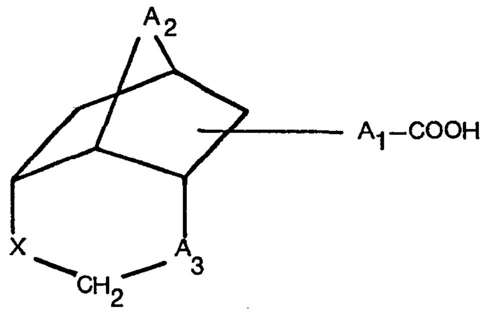

wherein $A_1$, $A_2$, $A_3$ and X are each as defined above, with an alcohol of the formula: $R_6$—OH (wherein $R_6$ is as defined above), in the presence of an acid catalyst;

b) a compound of the formula:

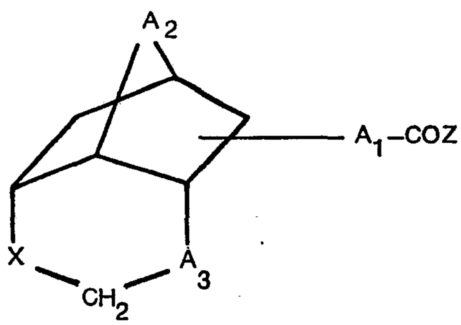

wherein $A_1$, $A_2$, $A_3$ and X are each as defined above and —COZ means an activated ester group, with an alcohol of the formula: $R_6$—OH (wherein $R_6$ is as defined above).

27. A process for producing a compound as claimed in claim 2 of the formula:

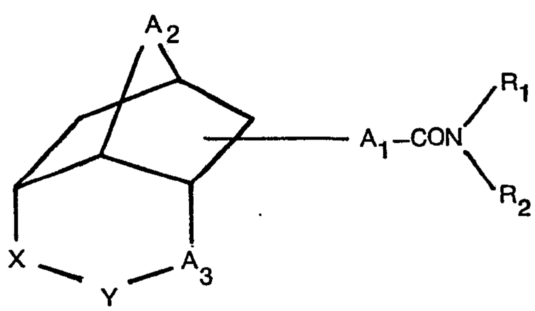

wherein X—Y is —O—$CH_2$— or —S—$CH_2$— and $A_1$, $A_2$, $A_3$, $R_1$ and $R_2$ are each as defined in claim 2, which comprises reacting a carboxylic acid or its derivative of the formula

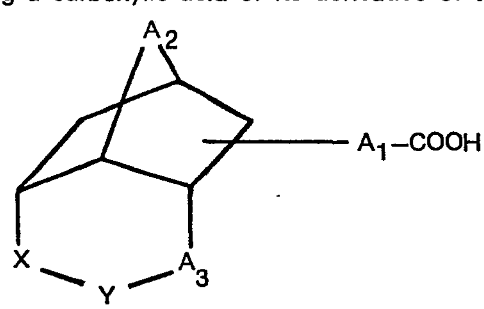

wherein $A_1$, $A_2$, $A_3$ and X—Y are each as defined above, with an amine of the formula:

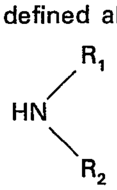

wherein $R_1$ and $R_2$ are each as defined above.

**Patentansprüche**

1. Arzneimittel, enthaltend zusammen mit einem pharmazeutisch verträglichen inerten Träger oder Verdünnungsmittel als Wirkstoff eine pharmazeutisch wirksame Menge mindestens einer der Verbindungen der allgemeinen Formel

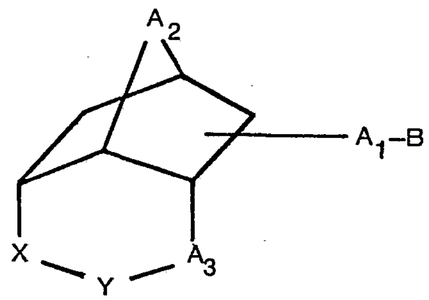

in der $A_1$ eine Einfachbindung oder eine $C_1$- bis $C_5$-Alkylen- oder $C_2$- bis $C_5$-Alkylidengruppe in der 2- oder 3-Stellung ist, $A_2$ eine $C_1$- bis $C_3$-Alkylen-, Cyclopropylen- oder $C_2$- bis $C_3$-Alkylidengruppe ist, $A_3$ eine Einfachbindung oder eine Methylengruppe bedeutet, X—Y die Gruppe —O—$CH_2$—, —O—CO— oder —$SO_n$—$CH_2$— (wobei n den Wert 0, 1 oder 2 hat) und B eine Carboxyl-, $C_2$- bis $C_5$-Alkoxycarbonyl-, Cyano-, Hydroxyl- oder $C_2$- bis $C_5$-Alkanoyloxygruppe, ein Halogenatom, eine Carbamoylgruppe der Formel

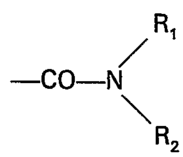

(in der $R_1$ und $R_2$ jeweils ein Wasserstoffatom, eine $C_1$- bis $C_5$-Alkyl- oder eine $C_3$- bis $C_5$-Alkenylgruppe bedeuten oder zusammen mit dem Stickstoffatom eine Pyrrolidino-, Piperazino- oder Morpholinogruppe bilden), oder eine Aminogruppe der Formel

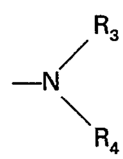

(in der $R_3$ ein Wasserstoffatom, eine $C_1$- bis $C_5$-Alkyl-, $C_3$- bis $C_5$-Alkenyl-, $C_3$- bis $C_5$-Alkinyl- oder $C_7$- bis $C_9$-Aralkylgruppe und $R_4$ ein Wasserstoffatom, eine $C_1$- bis $C_5$-Alkyl-, $C_3$- bis $C_5$-Alkenyl, $C_3$- bis $C_5$-Alkinyl-, $C_7$- bis $C_9$-Aralkyl-, $C_2$- bis $C_5$-Alkoxycarbonyl-, Benzyloxycarbonyl-, $C_2$- bis $C_5$-Alkanoyl- oder $C_2$- bis $C_4$-Halogenalkanoylgruppe bedeuten oder $R_3$ und $R_4$ zusammen mit dem Stickstoffatom eine Pyrrolidino-, Piperazino- oder Morpholinogruppe bilden), und ihre nichttoxischen Salze, mit der Maßgabe, wenn $A_1$ eine Einfachbindung ist, $A_2$ eine Methylen- oder Äthylengruppe, $A_3$ eine Einfachbindung, X—Y die Gruppe —O—CO—, B eine Carboxylgruppe und $A_1$ in der 3-Stellung steht, mit der Maßgabe, wenn X—Y die Gruppe —O—$CH_2$— ist, $A_3$ eine Einfachbindung und $A_1$ eine Methylengruppe und B keine Hydroxylgruppe bedeutet, und mit der Maßgabe, daß die Alkenyl- und Alkinylgruppen keine Doppelbindung bzw. Dreifachbindung in der 1-Stellung enthalten.

2. Eine Verbindung der Formel

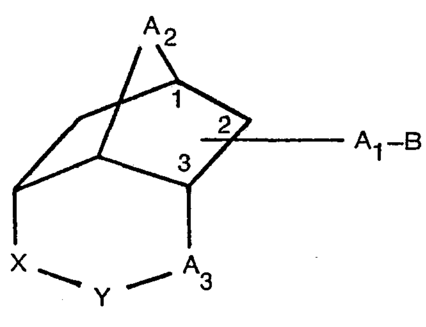

in der $A_1$ eine Einfachbindung oder eine $C_1$- bis $C_5$-Alkylen- oder $C_2$- bis $C_5$-Alkylidengruppe in der 2- oder 3-Stellung ist, $A_2$ eine $C_1$- bis $C_3$-Alkylen-, Cyclopropylen- oder $C_2$- bis $C_3$-Alkylidengruppe ist, $A_3$

eine Einfachbindung oder eine Methylengruppe ist, X—Y die Gruppe, —O—$CH_2$—, —O—CO— oder —$SO_n$—$CH_2$— (in der n den Wert 0, 1 oder 2 hat) und B eine Carboxyl-, $C_2$- bis $C_5$-Alkoxycarbonyl- oder Cyanogruppe, ein Halogenatom, eine Carbamoylgruppe der Formel

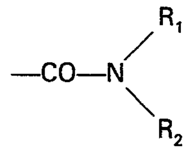

(in der $R_1$ und $R_2$ jeweils ein Wasserstoffatom, eine $C_1$- bis $C_5$-Alkyl- oder $C_3$- bis $C_5$-Alkenylgruppe bedeuten oder $R_1$ und $R_2$ zusammen mit dem Stickstoffatom eine Pyrrolidino-, Piperazino- oder Morpholinogruppe bilden) oder eine Aminogruppe der Formel

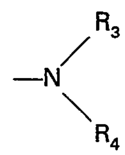

bedeutet (in der $R_3$ ein Wasserstoffatom, eine $C_1$- bis $C_5$-Alkyl-, $C_3$- bis $C_5$-Alkenyl-, $C_3$- bis $C_5$-Alkinyl- oder $C_7$- bis $C_9$-Aralkylgruppe und $R_4$ ein Wasserstoffatom, eine $C_1$ bis $C_5$-Alkyl-, $C_3$- bis $C_5$-Alkenyl-, $C_3$- bis $C_5$-Alkinyl-, $C_7$- bis $C_9$-Aralkyl-, $C_2$- bis $C_5$-Alkoxycarbonyl-, Benzyloxycarbonyl-, $C_2$- bis $C_5$-Alkanoyl- oder $C_2$- bis $C_4$-Halogenalkanoylgruppe bedeuten oder $R_3$ und $R_4$ zusammen mit dem Stickstoffatom eine Pyrrolidino-, Piperazino- oder Morpholinogruppe bilden), mit der Maßgabe, wenn X—Y die Gruppe —O—CO— bedeutet, B eine Aminogruppe der Formel

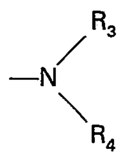

ist und daß die Alkenyl- und Alkinylgruppe keine Doppelbindung bzw. Dreifachbindung in der 1-Stellung enthalten.

3. Eine Verbindung der Formel

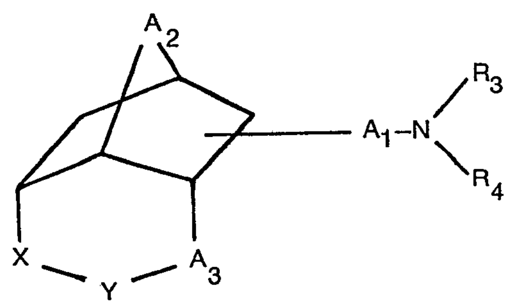

in der $A_1$, $A_2$, $A_3$, X—Y, $R_3$ und $R_4$ die in Anspruch 2 angegebene Bedeutung haben, oder ein nichttoxisches Salz dieser Verbindung

4. Eine Verbindung der Formel

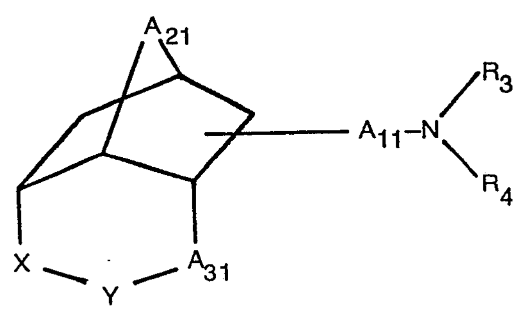

in der $A_{11}$ eine Einfachbindung oder eine Methylengruppe in der 2- oder 3-Stellung, $A_{21}$ eine Methylen- oder Äthylengruppe, $A_{31}$ eine Einfachbindung oder eine Methylengruppe, X—Y die Gruppe

—O—$CH_2$—, —O—CO— oder —$SO_n$—$CH_2$— (in der n den Wert 0, 1 oder 2 hat) bedeuten und $R_3$ und $R_4$ die in Anspruch 2 angegebene Bedeutung haben, oder ein nichttoxisches Salz dieser Verbindung.

5. Ein Verbindung der Formel

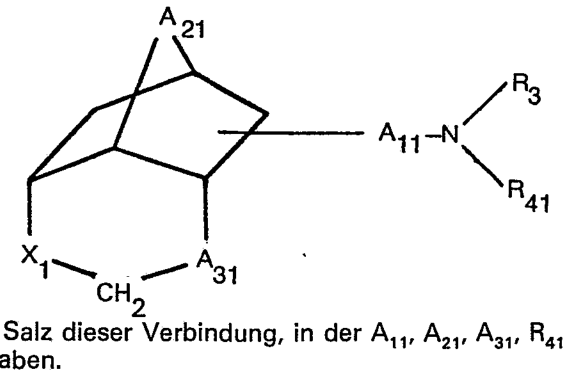

oder ein nichttoxisches Salz dieser Verbindung, in der $A_{11}$, $A_{21}$, $A_{31}$, $R_{41}$ und $R_3$ die in Anspruch 4 angegebene Bedeutung haben.

6. Eine Verbindung der Formel

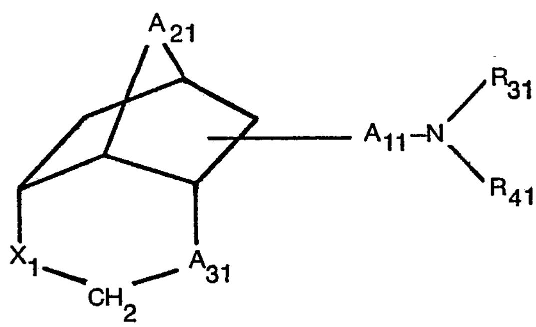

in der $A_{11}$, $A_{21}$, $A_{31}$, $R_{41}$ und $X_1$ die in Anspruch 5 angegebene Bedeutung haben und $R_{31}$ ein Wasserstoffatom oder ein $C_1$- bis $C_5$-Alkylrest ist, oder ein nichttoxisches Salz dieser Verbindung.

7. Eine Verbindung der Formel

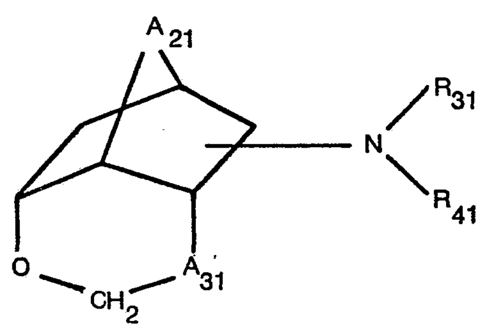

in der $A_{21}$, $A_{31}$, $R_{31}$ und $R_{41}$ die in Anspruch 6 angegebene Bedeutung haben, oder ein nichttoxisches Salz dieser Verbindung.

8. Eine Verbindung der Formel

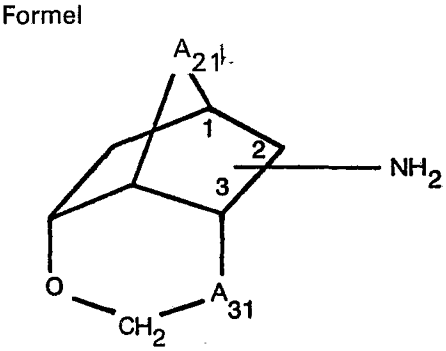

in der $A_{21}$ eine Methylen- oder Äthylengruppe und $A_{31}$ eine Einfachbindung oder eine Methylengruppe ist und die Aminogruppe in der 2- oder 3-Stellung steht, oder ein nichttoxisches Salza dieser Verbindung.

9. Eine Verbindung der Formel

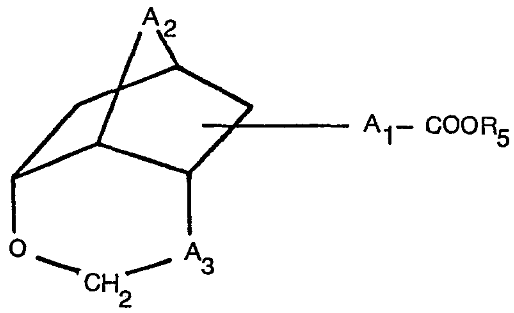

in der $A_1$, $A_2$ und $A_3$ die in Anspruch 2 angegebene Bedeutung haben und $R_5$ ein Wasserstoffatom oder eine $C_1$- bis $C_4$-Alkylgruppe ist, oder ein nichttoxisches Salz dieser Verbindung.

10. Eine Verbindung der Formel

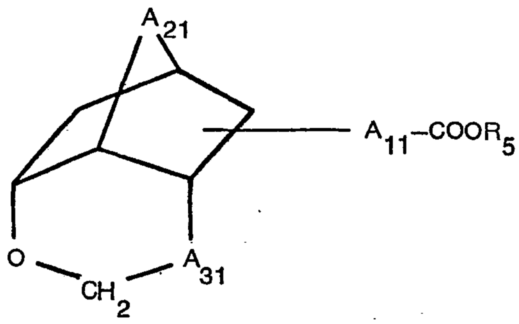

in der $A_{11}$, $A_{21}$ und $A_{31}$ die in Anspruch 4 angegebene Bedeutung haben und $R_5$ ein Wasserstoffatom oder eine $C_1$- bis $C_4$-Alkylgruppe ist, oder ein nichttoxisches Salz dieser Verbindung.

11. Eine Verbindung der Formel

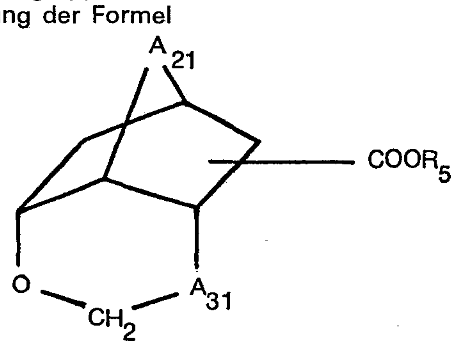

in der $A_{21}$, $A_{31}$ und $R_5$ die in Anspruch 10 angegebene Bedeutung haben, oder ein nichttoxisches Salz dieser Verbindung.

12. Verfahren zur Herstellung einer Verbindung nach Anspruch 2 der Formel

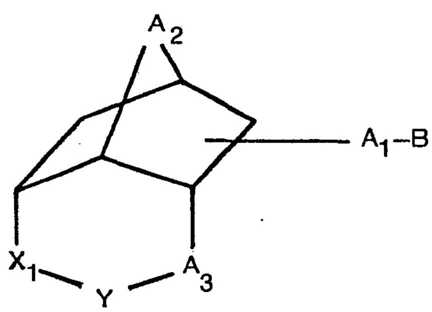

in der $A_1$, $A_2$, $A_3$ und B die in Anspruch 2 angegebene Bedeutung haben und $X_1$—Y die Gruppe

—O—$CH_2$—, —S—$CH_2$— oder —O—CO—, bedeutet, dadurch gekennzeichnet, daß man eine Verbindung der Formel

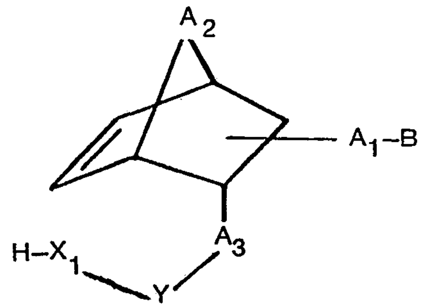

in der $A_1$, $A_2$, $A_3$, B und $X_1$—Y die vorstehende Bedeutung haben, cyclisiert.

13. Verfahren zur Herstellung einer Verbindung nach Anspruch 2 der Formel

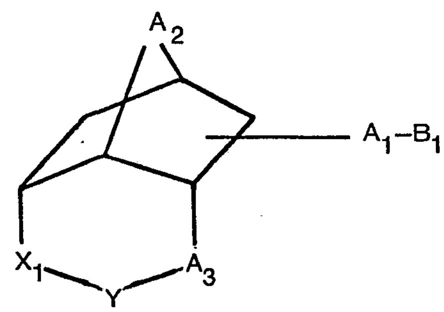

in der $A_1$, $A_2$ und $A_3$ die in Anspruch 2 angegebene Bedeutung haben und $B_1$ die gleiche Bedeutung wie B hat, jedoch kein Halogenatom ist, und $X_1$—Y die Gruppe —O—$CH_2$—, —S—$CH_2$— oder —O—CO— ist, dadurch gekennzeichnet, daß man eine Verbindung der Formel

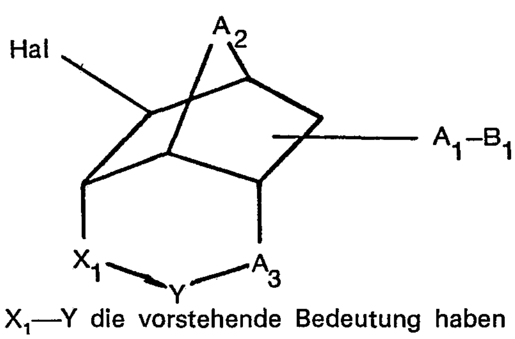

in der $A_1$, $A_2$, $A_3$, $B_1$ und $X_1$—Y die vorstehende Bedeutung haben und Hal ein Halogenatom ist, reduziert.

14. Verfahren zur Herstellung einer Verbindung nach Anspruch 2 der Formel

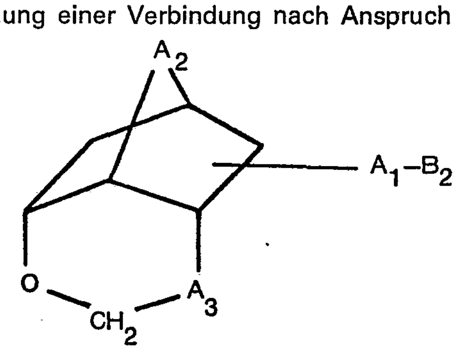

in der $A_1$, $A_2$ und $A_3$ die in Anspruch 2 angegebene Bedeutung haben und $B_2$ eine Cyanogruppe, eine Carbamoylgruppe der Formel

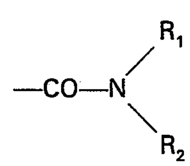

(in der $R_1$ und $R_2$ die in Anspruch 2 angegebene Bedeutung haben) oder eine Aminogruppe der Formel

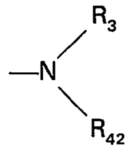

(in der $R_3$ die in Anspruch 2 angegebene Bedeutung hat und $R_{42}$ eine $C_2$- bis $C_5$-Alkoxycarbonyl-, Benzyloxycarbonyl-, $C_2$- bis $C_5$-Alkanoyl- oder $C_2$- bis $C_4$-Halogenalkanoylgruppe ist) bedeutet, dadurch gekennzeichnet, daß man eine Verbindung der Formel

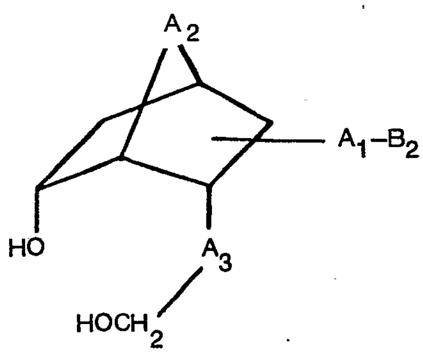

in der $A_1$, $A_2$, $A_3$ und $B_2$ die vorstehende Bedeutung haben, mit einem Dehydratisierungsmittel umsetzt.

15. Verfahren zur Herstellung einer Verbindung nach Anspruch 2 der Formel

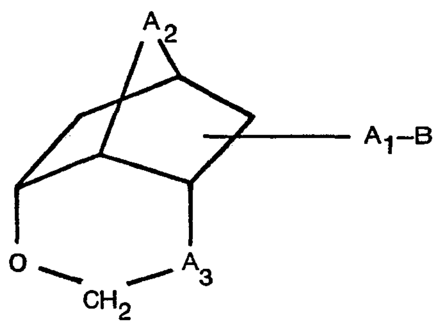

in der $A_1$, $A_2$, $A_3$ und B die in Anspruch 2 angegebene Bedeutung haben, dadurch gekennzeichnet, daß man eine Verbindung der Formel

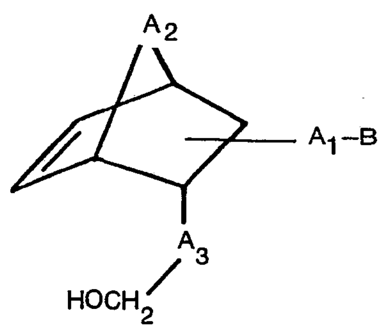

in der $A_1$, $A_2$, $A_3$ und B die vorstehende Bedeutung haben, über eine Oxymerkurierungs- Demerkurierungsreaktion cyclisiert.

16. Verfahren zur Herstellung einer Verbindung nach Anspruch 2 der Formel

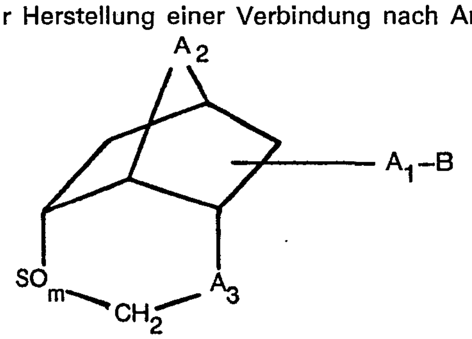

in der $A_1$, $A_2$, $A_3$ und B die in Anspruch 2 angegebene Bedeutung haben und m den Wert 1 oder 2 hat, dadurch gekennzeichnet, daß man eine Verbindung der Formel

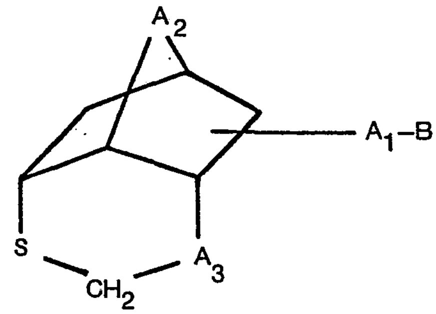

in der $A_1$, $A_2$, $A_3$ und B die vorstehende Bedeutung haben, oxidiert.

17. Verfahren zur Herstellung einer Verbindung nach Anspruch 2 der Formel

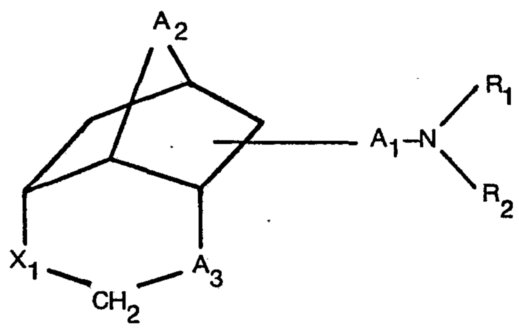

in der $A_1$, $A_2$, $A_3$, $R_1$ und $R_2$ die in Anspruch 2 angegebene Bedeutung haben und $X_1$ ein Sauerstoff- oder Schwefelatom ist, dadurch gekennzeichnet, daß man

a) eine Verbindung der Formel

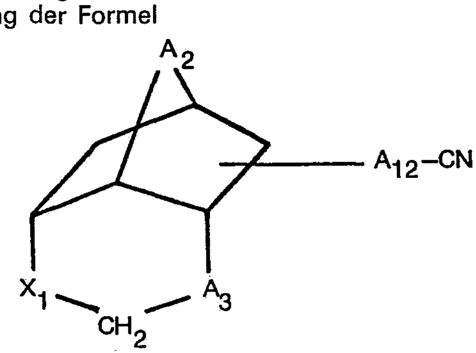

in der $A_2$ und $A_3$ die vorstehend angegebene Bedeutung haben, $A_{12}$ eine Einfachbindung oder eine $C_1$- bis $C_4$-Alkylengruppe und $X_1$ ein Sauerstoff- oder Schwefelatom bedeutet, mit einer Metallhydridverbindung oder Wasserstoff in Gegenwart eines Katalysators reduziert;

b) eine Verbindung der Formel

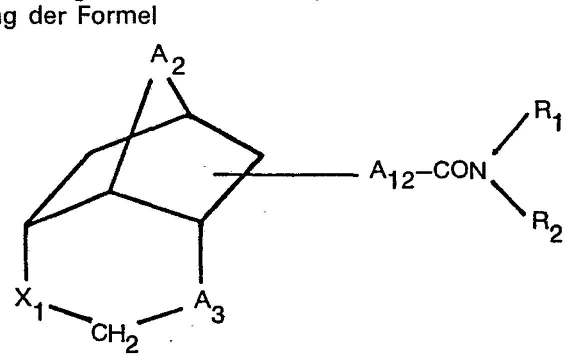

in der $A_{12}$, $A_2$, $A_3$, $R_1$, $R_2$ und $X_1$ die vorstehend angegebene Bedeutung haben, mit einer Metallhydridverbindung reduziert;

c) eine Verbindung der Formel

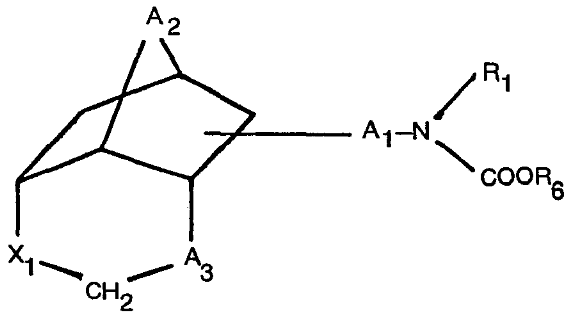

in der $A_1$, $A_2$, $A_3$, $X_1$ und $R_1$ die vorstehend angegebene Bedeutung haben und $R_6$ eine $C_1$- bis $C_4$-Alkylgruppe ist, mit einer Metallhydridverbindung reduziert;

d) eine Verbindung der Formel

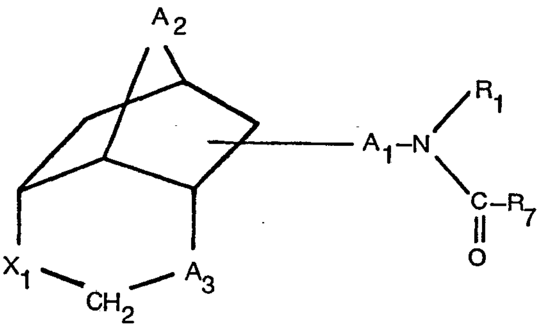

in der $A_1$, $A_2$, $A_3$, $X_1$ und $R_1$ die vorstehend angegebene Bedeutung haben und $R_7$ ein Wasserstoffatom, eine $C_1$- bis $C_4$-Alkyl- oder $C_2$- bis $C_4$-Alkenylgruppe bedeutet, mit einer Metallhydridverbindung reduziert;

e) eine Verbindung der Formel

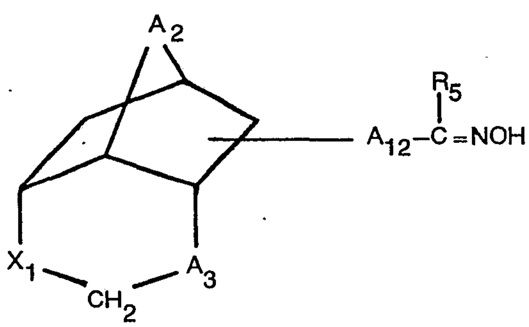

in der $A_{12}$, $A_2$, $A_3$ und $X_1$ die vorstehend angegebene Bedeutung haben und $R_5$ ein Wasserstoffatom oder eine $C_1$- bis $C_4$-Alkylgruppe bedeutet, mit einer Metallhydridverbindung oder Wasserstoff in Gegenwart eines Katalysators reduziert oder

f) eine Verbindung der Formel

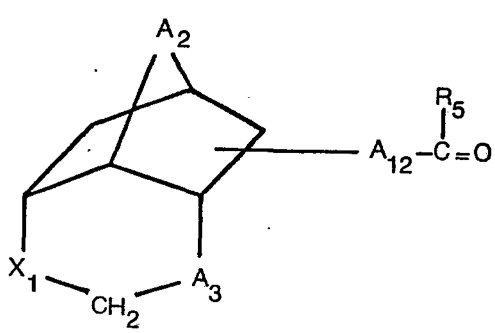

in der $A_{12}$, $A_2$, $A_3$, $R_5$ und $X_1$ die vorstehend angegebene Bedeutung haben, mit einem Reduktionsmittel in Gegenwart eines Amins der Formel

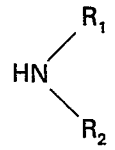

in der $R_1$ und $R_2$ die vorstehend angegebene Bedeutung haben, reduziert.

18. Verfahren zur Herstellung einer Verbindung nach Anspruch 2 der Formel

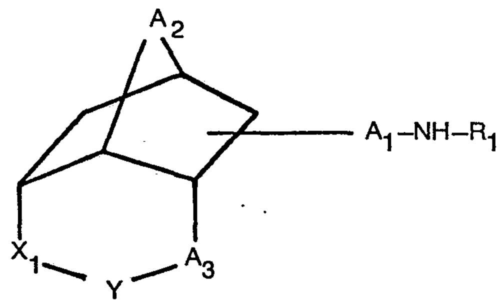

in der $A_1$, $A_2$, $A_3$ und $R_1$ die in Anspruch 2 angegebene Bedeutung haben und $X_1$—Y die Gruppe —O—$CH_2$—, —S—$CH_2$— oder —O—CO— ist, dadurch gekennzeichnet, daß man die Verbindung der Formel

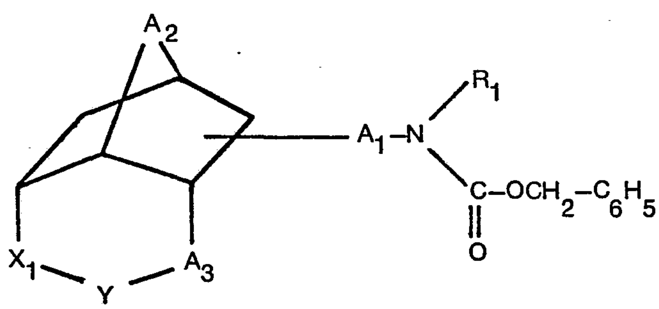

in der $A_1$, $A_2$, $A_3$, $R_1$ und $X_1$—Y die vorstehend angegebene Bedeutung haben, entbenzyliert und decarboxyliert.

19. Verfahren zur Herstellung von Verbindungen nach Anspruch 2 der Formeln

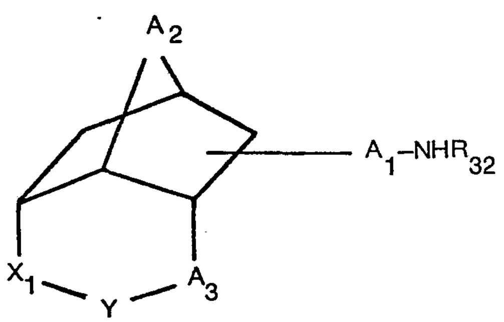

;

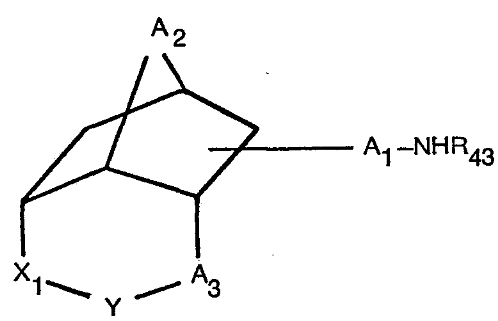

und

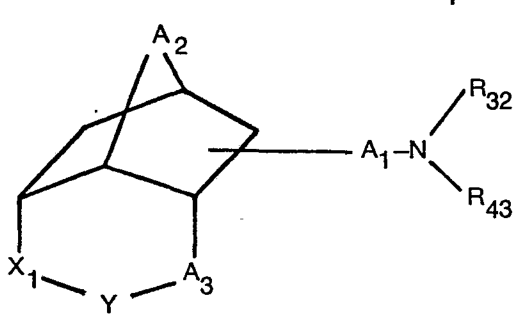

in der $A_1$, $A_2$ und $A_3$ die in Anspruch 2 angegebene Bedeutung haben, $X_1$—Y die Gruppe —O—$CH_2$—, —S—$CH_2$— oder —O—CO— ist, $R_{32}$ die gleiche Bedeutung hat wie $R_3$ in Anspruch 2, ausgenommen

ein Wasserstoffatom, und $R_{43}$ die gleiche Bedeutung hat wie $R_4$ in Anspruch 2, ausgenommen ein Wasserstoffatom, dadurch gekennzeichnet, daß man eine Verbindung der Formel

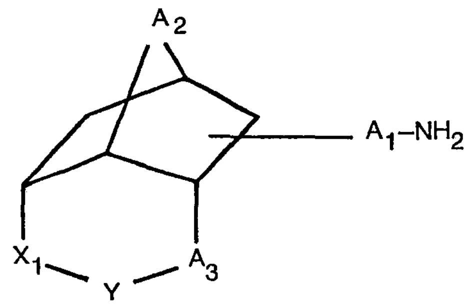

in der $A_1$, $A_2$, $A_3$ und $X_1$—Y die vorstehend angegebene Bedeutung haben, mit einer aktivierten Verbindung der Formel $R_{32}$—Z oder $R_{43}$—Z (in der Z den Rest einer starken Säure bedeutet) zur Umsetzung bringt.

20. Verfahren zur Herstellung einer Verbindung nach Anspruch 2 der Formel

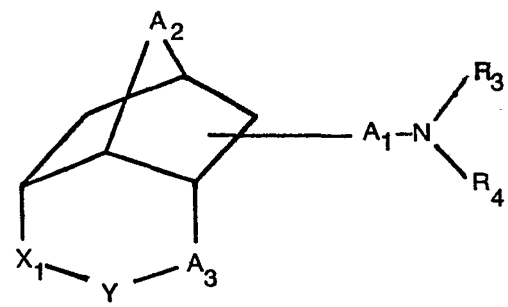

in der $A_1$, $A_2$, $A_3$, $R_3$ und $R_4$ die in Anspruch 2 angegebene Bedeutung haben und $X_1$—Y die Gruppe —O—$CH_2$—, —S—$CH_2$— oder —O—CO— ist, dadurch gekennzeichnet, daß man eine Verbindung der Formel

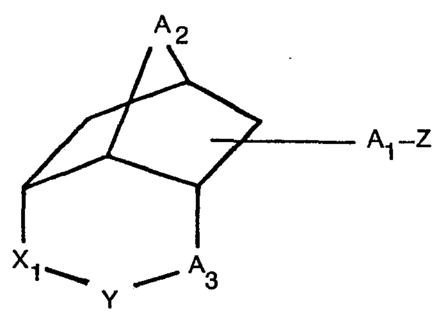

in der $A_1$, $A_2$, $A_3$ und $X_1$—Y die vorstehend angegebene Bedeutung haben und Z der Rest einer starken Säure ist, mit einem Amin der Formel

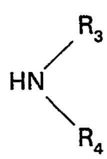

in der $R_3$ und $R_4$ die vorstehend angegebene Bedeutung hat, zur Umsetzung bringt.

21. Verfahren zur Herstellung einer Verbindung nach Anspruch 2 der Formel

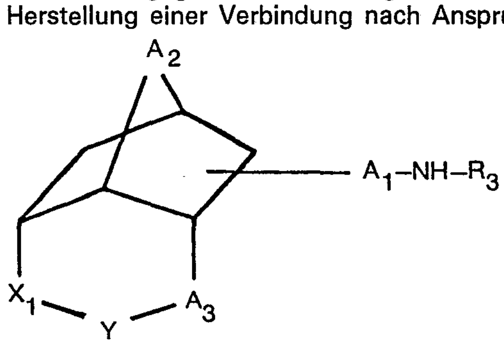

in der $A_1$, $A_2$, $A_3$ und $R_3$ die in Anspruch 2 angegebene Bedeutung haben und $X_1$—Y die Gruppe —O—$CH_2$—, —S—$CH_2$— oder —O—CO— ist, dadurch gekennzeichnet, daß man eine Verbindung der Formel

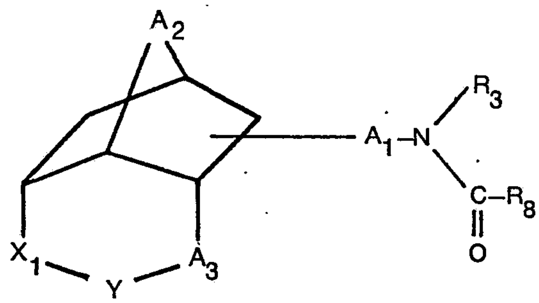

in der $A_1$, $A_2$, $A_3$, $R_3$ und $X_1$—Y die vorstehend angegebene Bedeutung haben und $R_8$ ein Wasserstoffatom, eine $C_1$- bis $C_4$-Alkyl-, $C_1$- bis $C_3$-Halogenalkyl-, $C_1$- bis $C_4$-Alkoxy- oder Benzyloxygruppe bedeutet, hydrolysiert.

22. Verfahren zur Herstellung einer Verbindung nach Anspruch 2 der Formel

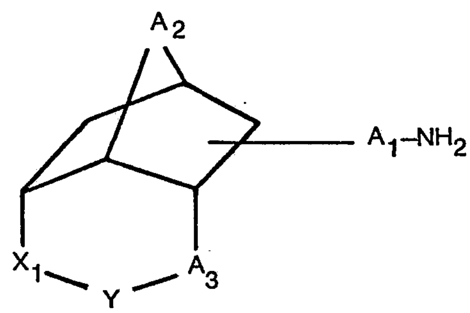

in der $A_1$, $A_2$ und $A_3$ die in Anspruch 2 angegebene Bedeutung haben und $X_1$—Y die Gruppe —O—$CH_2$—, —S—$CH_2$— oder —O—CO— ist, dadurch gekennzeichnet, daß man eine Verbindung der Formel

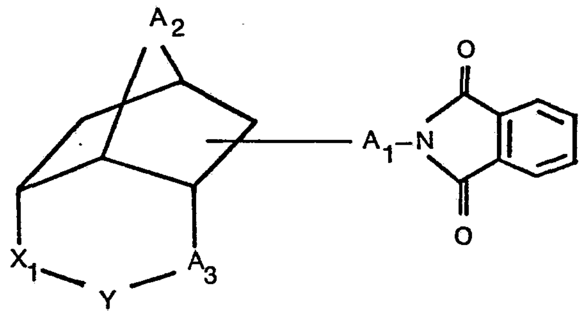

in der $A_1$, $A_2$, $A_3$ und $X_1$—Y die vorstehend angegebene Bedeutung haben, hydrolysiert.

23. Verfahren zur Herstellung einer Verbindung nach Anspruch 2 der Formel

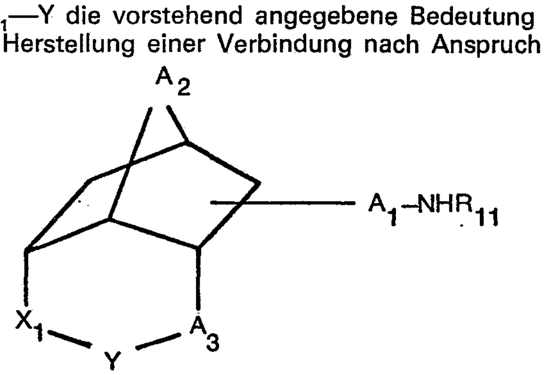

in der $A_1$, $A_2$ und $A_3$ die in Anspruch 2 angegebene Bedeutung haben und $X_1$—Y die Gruppe —O—$CH_2$—, —S—$CH_2$— oder —O—CO— und $R_{11}$ ein Wasserstoffatom, eine $C_2$- bis $C_5$-Alkyloxycarbonyl- oder Benzyloxycarbonylgruppe bedeutet, dadurch gekennzeichnet, daß man

a) eine Verbindung der Formel

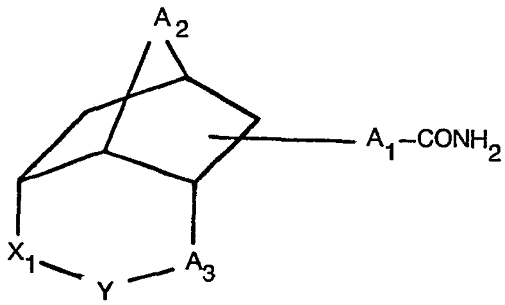

in der $A_1$, $A_2$, $A_3$ und $X_1$—Y die vorstehend angegebene Bedeutung haben, mit einem Alkalihypohalogenit behandelt und anschließend hydrolysiert oder das erhaltene Isocyanat mit einem Alkohol der Formel $R_9$—OH (in der $R_9$ eine $C_1$- bis $C_4$-Alkyl- oder Benzylgruppe ist) umsetzt;

b) eine Verbindung der Formel

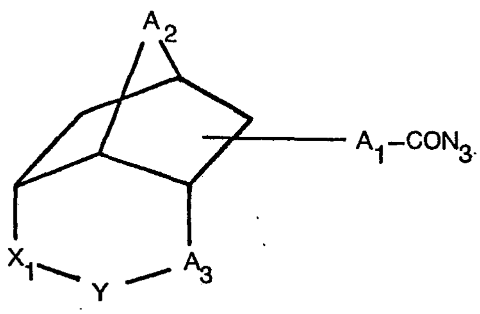

in der $A_1$, $A_2$, $A_3$ und $X_1$—Y die vorstehend angegebene Bedeutung haben, erhitzt, anschließend hydrolysiert oder das erhaltene Isocyanat mit einem Alkohol der Formel $R_9$—OH (in der $R_9$ eine $C_1$- bis $C_4$-Alkyl- oder Benzylgruppe ist) umsetzt;

c) eine Verbindung der Formel

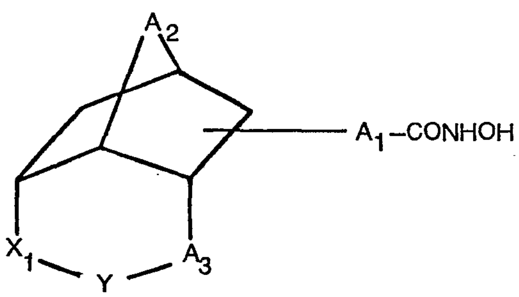

in der $A_1$, $A_2$, $A_3$ und $X_1$—Y die vorstehend angegebene Bedeutung haben, in Gegenwart oder Abwesenheit einer Base erhitzt, anschließend hydrolysiert oder das erhaltene Isocyanat mit einem Alkohol der Formel $R_9$—OH (in der $R_9$ eine $C_1$- bis $C_4$-Alkyl- oder Benzylgruppe bedeutet) umsetzt.

24. Verfahren zur Herstellung einer Verbindung nach Anspruch 2 der Formel

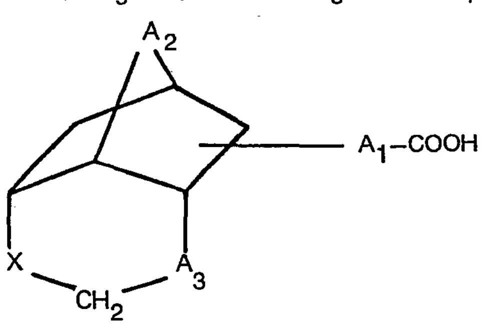

in der $A_1$, $A_2$ und $A_3$ die in Anspruch 2 angegebene Bedeutung haben und X ein Sauerstoffatom oder die

Gruppe $—SO_n—$ ist, wobei n den Wert 0, 1 oder 2 hat, dadurch gekennzeichnet, daß man eine Verbindung der Formel

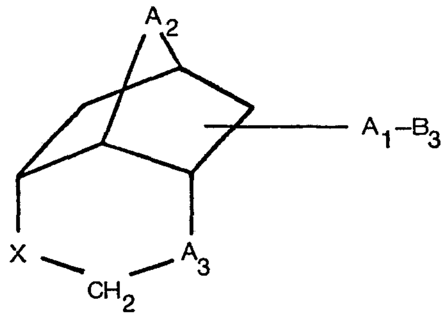

in der $A_1$, $A_2$, $A_3$ und X die vorstehend angegebene Bedeutung haben und $B_3$ eine Cyano-, $C_2$- bis $C_5$-Alkoxycarbonyl-, Benzyloxycarbonyl- oder Carbamoylgruppe der Formel

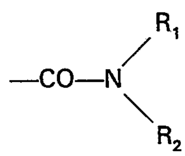

(in der $R_1$ und $R_2$ die in Anspruch 2 angegebene Bedeutung haben), bedeutet, hydrolysiert.

25. Verfahren zur Herstellung einer Verbindung nach Anspruch 2 der Formel

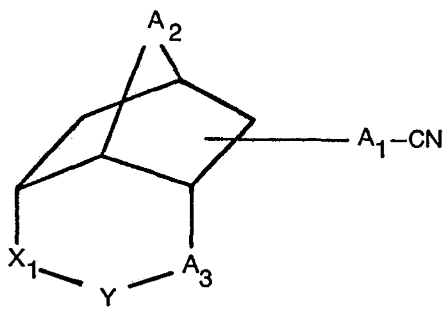

in der $A_1$, $A_2$ und $A_3$ die in Anspruch 2 angegebene Bedeutung haben und $X_1—Y$ die Gruppe $—O—CH_2—$ oder $—S—CH_2—$ ist, dadurch gekennzeichnet, daß man eine Verbindung der Formel

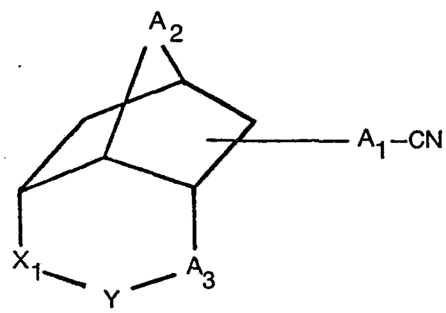

in der $A_1$, $A_2$, $A_3$ und $X_1—Y$ die vorstehend angegebene Bedeutung haben und Z der Rest einer starken Säure ist, mit einer Cyanid-verbindung umsetzt.

26. Verfahren zur Herstellung einer Verbindung nach Anspruch 2 der Formel

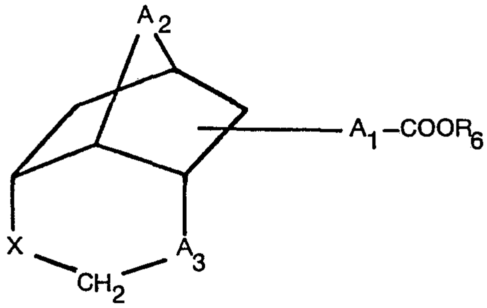

in der $A_1$, $A_2$ und $A_3$ die in Anspruch 2 angegebene Bedeutung haben, X ein Sauerstoff- oder Schwefelatom und $R_6$ eine $C_1$- bis $C_4$-Alkylgruppe ist, dadurch gekennzeichnet, daß man

a) eine Verbindung der Formel

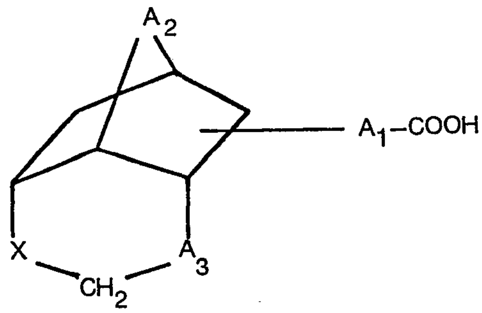

in der $A_1$, $A_2$, $A_3$ und X die vorstehend angegebene Bedeutung haben, mit einem Alkohol der Formel $R_6$—OH (in der $R_6$ die vorstehend angegebene Bedeutung hat) in Gegenwart eines sauren Katalysators zur Umsetzung bringt;

b) eine Verbindung der Formel

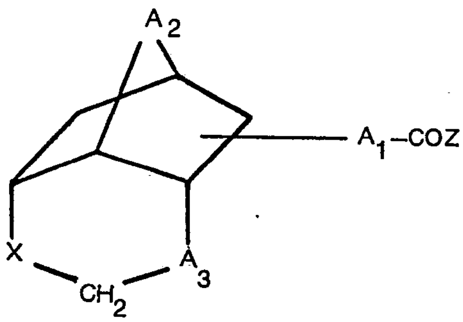

in der $A_1$, $A_2$, $A_3$ und X die vorstehend angegebene Bedeutung haben und —COZ eine aktivierte Estergruppe darstellt, mit einem Alkohol der Formel $R_6$—OH (in der $R_6$ die vorstehend angegebene Bedeutung hat) zur Umsetzung bringt.

27. Verfahren zur Herstellung einer Verbindung nach Anspruch 2 der Formel

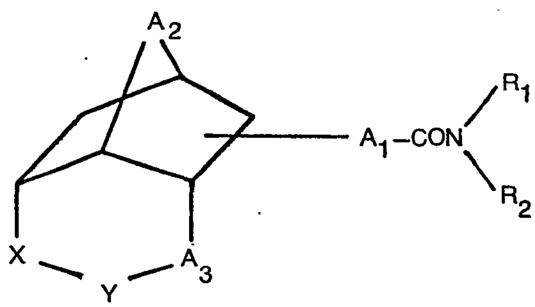

in der X—Y die Gruppe —O—$CH_2$— oder —S—$CH_2$— ist und $A_1$, $A_2$, $A_3$, $R_1$ und $R_2$ die in Anspruch 2 angegebene Bedeutung haben, dadurch gekennzeichnet, daß man eine Carbonsäure oder deren Derivat der Formel

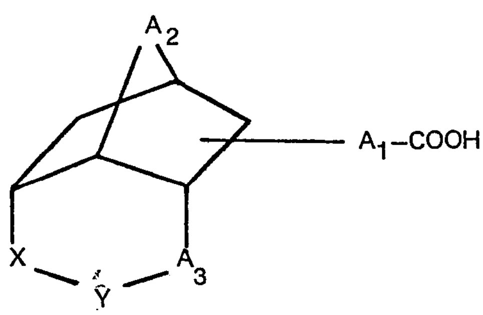

in der $A_1$, $A_2$, $A_3$ und X—Y die vorstehend angegebene Bedeutung haben, mit einem Amin der Formel

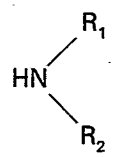

in der $R_1$ und $R_2$ die vorstehend angegebene Bedeutung haben, umsetzt.

**Revendications**

1. Composition pharmaceutique, caractérisée en ce qu'elle comprend, en même temps qu'un diluant ou véhicule inerte, pharmaceutiquement acceptable, à titre d'ingrédient actif, une quantité pharmaceutiquement efficace d'au moins un des composés de la formule générale suivante:

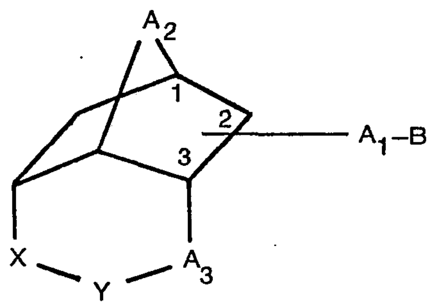

dans laquelle $A_1$ représente une simple liaison ou un radical alkylène en $C_1$—$C_5$ ou un radical alkylidène en $C_2$—$C_5$, lié en position 2 ou en position 3; $A_2$ représente un groupe alkylène en $C_1$ à $C_3$, cyclopropylène ou alkylidène en $C_2$—$C_3$; $A_3$ représente une simple liaison ou un groupe méthylène, X—Y représente un groupement —O—$CH_2$, —O—CO— ou —$SO_n$—$CH_2$— (où n est égal à 0 ou représente un nombre entier égal à 1 ou à 2) et B représente un groupe carboxyle, un groupe alcoxycarbonyle en $C_2$—$C_5$, un groupe cyano, un groupe hydroxyle, un groupe alcanoyloxy en $C_2$—$C_5$, un atome d'halogène, un groupe carbamoyle de la formule:

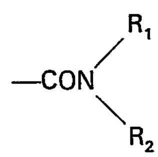

(dans laquelle $R_1$ et $R_2$ représentent chacun un atome d'hydrogène, un groupe alkyle en $C_1$—$C_5$ ou un groupe alcényle en $C_3$—$C_5$, ou, lorsqu'on les considère ensemble avec l'atome d'azote voisin, ils peuvent représenter un groupe pyrrolidino, pipérazino ou morpholino), ou un groupe amino de la formule:

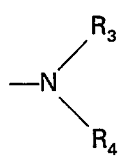

(dans laquelle $R_3$ représente un atome d'hydrogène, un groupe alkyle en $C_1$—$C_5$, un groupe alcényle en $C_3$—$C_5$, un groupe alcynyle en $C_3$—$C_5$, ou un groupe aralkyle en $C_7$—$C_9$ et $R_4$ représente un atome d'hydrogène, un groupe alkyle en $C_1$—$C_5$, un groupe alcényle en $C_3$—$C_5$, un groupe alcynyle en $C_3$—$C_5$, un groupe aralkyle en $C_7$—$C_9$, un groupe alcoxycarbonyle en $C_2$—$C_5$, un groupe benzyloxycarbonyle, un groupe alcanoyle en $C_2$—$C_5$ ou un groupe haloalcanoyle en $C_2$—$C_3$, ou bien, lorsqu'on les considère ensemble avec l'atome d'azote voisin, $R_3$ et $R_4$ peuvent représenter un groupe pyrrolidino, pipérazino ou morpholino) et leurs sels atoxiques, à condition que:

lorsque $A_1$ représente une simple liaison, $A_2$ désigne un groupe méthylène ou un groupe éthylène, $A_3$ représente un simple liaison, X—Y désigne —O—CO—, B désigne un groupe carboxyle et $A_1$ soit lié en position 3;

lorsque X—Y représente —O—$CH_2$—, $A_3$ représente une simple liaison et $A_1$ représente un groupe méthylène, B ne représente pas de groupe hydroxyle; et

les radicaux alcényle et alcynyle ne contiennent pas de double ou triple liaison en position 1.

2. Composé de la formule:

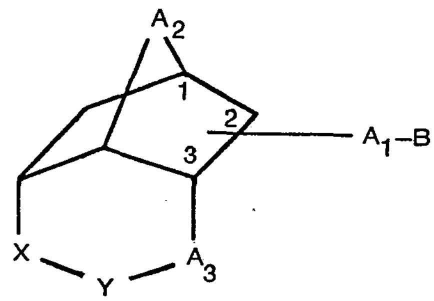

dans laquelle $A_1$ représente une simple liaison ou un groupe alkylène en $C_1$—$C_5$ ou un groupe alkylidène en $C_2$—$C_5$, lié en position 2 ou en position 3, $A_2$ représente un groupe alkylène en $C_2$—$C_3$, cyclopropylène ou alkylidène en $G_2$—$G_3$ (lire $C_2$—$C_3$), $A_3$ représente une simple liaison ou un groupe méthylène, X—Y représente un groupe —O—$CH_2$—, —O—CO— ou —$SO_n$—$CH_2$— (où n est égal à 0 ou représente un nombre entier égal à 1 ou 2) et B représente un groupe carboxyle, un groupe alcoxycarbonyle en $C_2$—$C_5$, un groupe cyano, un atome d'halogène, un groupe carbamoyle de la formule:

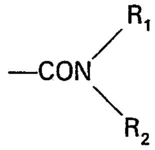

(dans laquelle $R_1$ et $R_2$ représentent chacun un atome d'hydrogène, un groupe alkyle en $C_1$—$C_5$ ou un groupe alcényle en $C_3$—$C_5$ ou, lorsqu'ils sont considérés ensemble avec l'atome d'azote voisin, ils peuvent représenter un groupe pyrrolidino, pipérazino ou morpholino), ou un groupe amino de la formule:

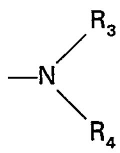

(dans laquelle $R_3$ représente un atome d'hydrogène, un groupe alkyle en $C_1$—$C_5$, un groupe alcényle en $C_3$—$C_5$, un groupe alcynyle en $C_3$—$C_5$, ou un groupe aralkyle en $C_7$—$C_9$ et R représente un atome d'hydrogène, un groupe alkyle en $C_1$—$C_5$, un groupe alcényle en $C_3$—$C_5$, un groupe alcynyle en $C_3$—$C_5$, un groupe aralkyle en $C_7$—$C_9$, un groupe alcoxycarbonyle en $C_2$—$C_5$, un groupe benzyloxycarbonyle, un groupe alcanoyle en $C_2$—$C_5$, ou un groupe halo-alcanoyle en $C_2$—$C_4$, ou, lorsqu'ils sont considérés ensemble avec l'atome d'azote voisin, $R_3$ et $R_4$ peuvent représenter un groupe pyrrolidino, pipérazino ou morpholino), ou les sels atoxiques de ce composé, à condition que:

lorsque X—Y représente un groupe —O—CO—, B représente un groupe amino de la formule:

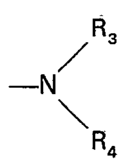

et que les groupes alcényle et alcynyle ne contiennent pas de double ou de triple liaison en position 1.

3. Composé de la formule

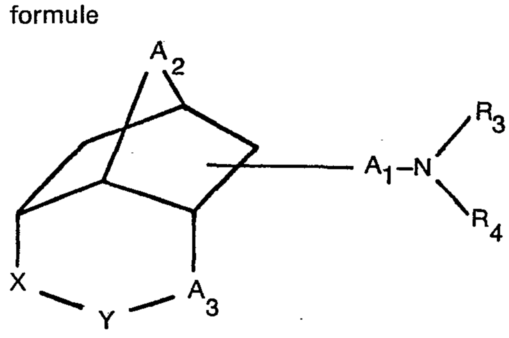

dans laquelle $A_1$, $A_2$, $A_3$, X—Y, $R_3$ et $R_4$ possèdent les significations indiquées dans le revendication 2, ou un sel atoxique d'un tel composé.

4. Composé de la formule:

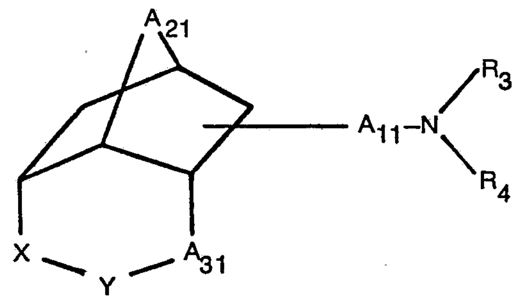

dans laquelle $A_{11}$ représente une simple liaison ou un groupe méthylène lié en position 2 ou en position 3, $A_{21}$ représente un groupe méthylène ou un groupe éthylène, $A_{31}$ représente une simple liaison ou un groupe méthylène, X—Y représente un groupe —O—$CH_2$—, —O—CO— ou —$SO_n$—$CH_2$— (où n est un nombre entier dont la valeur varie de 0 à 2) et $R_3$ et $R_4$ possèdent les significations indiquées dans la revendication 2, ou un sel atoxique d'un tel composé.

5. Composé de la formule:

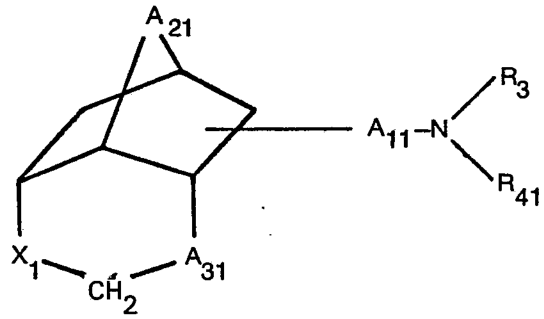

dans laquelle $A_{11}$, $A_{21}$, $A_{31}$, $R_{41}$, et $R_3$ possèdent les significations indiquées dans la revendication 4, ou un sel atoxique d'un tel composé.

6. Composé de la formule:

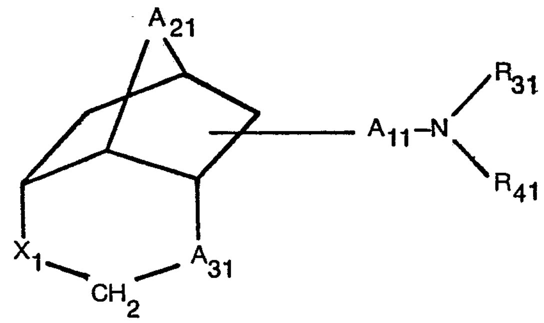

dans laquelle $A_{11}$, $A_{21}$, $A_{31}$, $R_{41}$ et $X_1$ possèdent les significations indiquées dans la revendication 5 et $R_{31}$ représente un atome d'hydrogène ou un groupe alkyle en $C_1$—$C_5$, ou un sel atoxique d'un tel composé.

7. Composé de la formule:

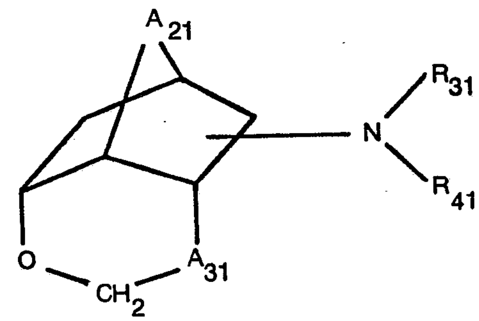

dans laquelle $A_{21}$, $A_{31}$, $R_{31}$ et $R_{41}$ possèdent les significations indiquées dans la revendication 6, ou un sel atoxique d'un tel composé.

8. Composé de la formule

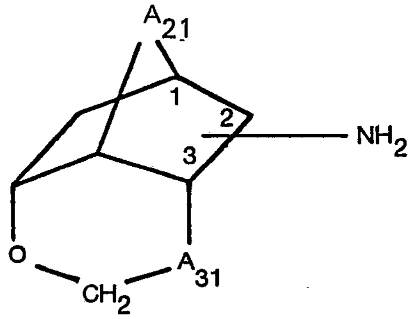

dans laquelle $A_{21}$ représente un groupe méthylène ou un groupe éthylène, $A_{31}$ représente une simple liaison ou un groupe méthylène et le groupe amino est lié en position 2 ou en position 3, ou un sel atoxique d'un tel composé.

9. Composé de la formule:

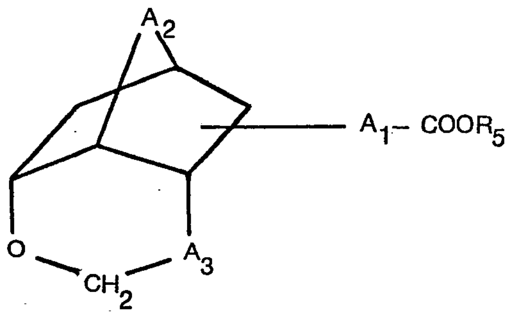

dans laquelle $A_1$, $A_2$ et $A_3$ possèdent les significations indiquées dans la revendication 2 et $R_5$ représente un atome d'hydrogène ou un groupe alkyle en $C_1$—$C_4$, ou un sel atoxique d'un tel composé.

10. Composé de la formule:

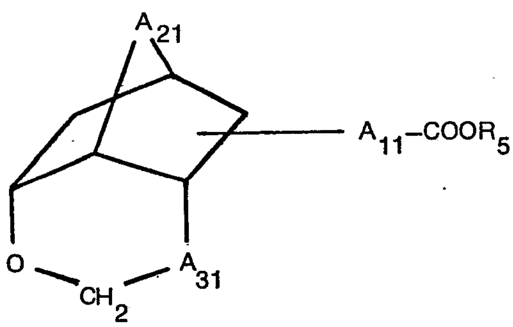

dans laquelle $A_{11}$, $A_{21}$ et $A_{31}$ possèdent les significations indiquées dans la revendication 4 et $R_5$ représente un atome d'hydrogène ou un groupe alkyle en $C_1$—$C_4$, ou un sel atoxique d'un tel composé.

11. Composé de la formule:

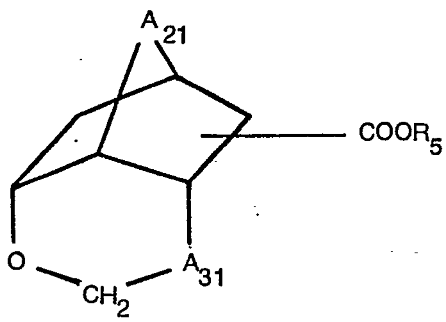

dans laquelle $A_{21}$, $A_{31}$ et $R_5$ possèdent les significations indiquées dans la revendication 10, ou un sel atoxique d'un tel composé.

12. Procédé de production d'un composé tel que défini dans la revendication 2, de la formule:

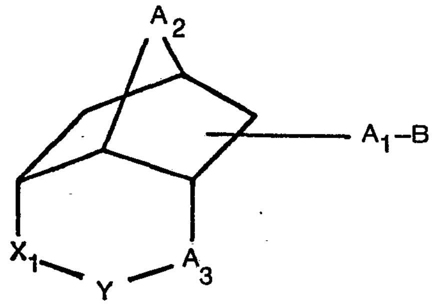

dans laquelle $A_1$, $A_2$, $A_3$ et B sont chacun tels que défini dans la revendication 2 et $X_1$—Y représente un groupe —O—$CH_2$—, —S—$CH_2$— ou O—CO—, caractérisé en ce que l'on cyclise un composé de la formule:

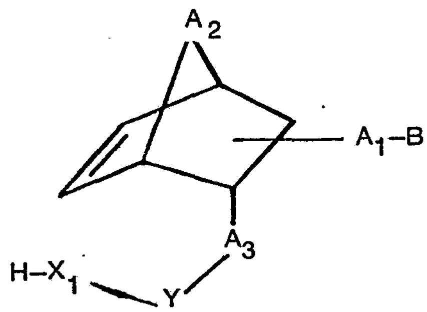

dans laquelle $A_1$, $A_2$, $A_3$, B et $X_1$—Y sont chacun tels que définis plus haut.

13. Procédé de production d'un composé suivant la revendication 2, de la formule:

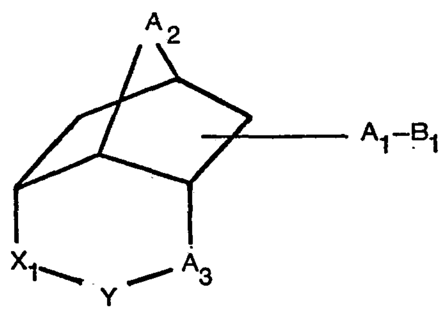

dans laquelle, $A_1$, $A_2$ et $A_3$ sont chacun tels que définis dans la revendication 2 et $B_1$ est identique à B à l'exclusion d'un atome d'halogène et $X_1$—Y représente un groupe —O—$CH_2$—, —S—$CH_2$— ou —O—CO—, caractérisé en ce que l'on réduit un composé de la formule:

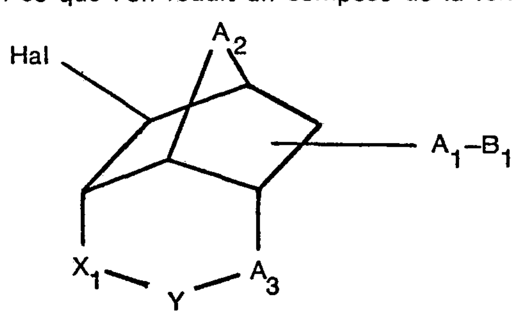

dans laquelle $A_1$, $A_2$, $A_3$, $B_1$ et $X_1$—Y possèdent chacun les significations indiquées plus haut et Hal représentent un atome d'halogène.

14. Procédé de production d'un composé suivant la revendication 2, de la formule:

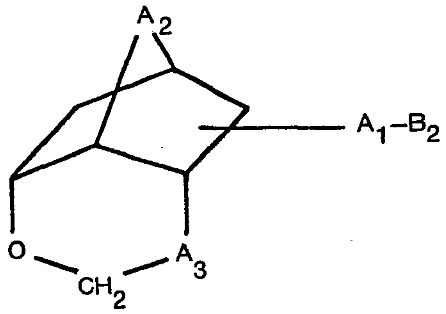

dans laquelle $A_1$, $A_2$ et $A_3$ sont chacun tels que définis dans la revendication 2 et $B_2$ représente un groupe cyano, un groupe carbamoyle de la formule:

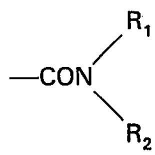

(dans laquelle $R_1$ et $R_2$ sont chacun tels que définis dans la revendication 2) ou un groupe amino de la formule:

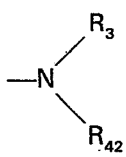

(dans laquelle $R_3$ est tel que défini dans la revendication 2 et $R_{42}$ représente un groupe alcoxycarbonyle en $C_2$—$C_5$, un groupe benzyloxycarbonyle, un groupe alcanoyle en $C_2$—$C_5$ ou un groupe haloalcanoyle en $C_2$—$C_4$), caractérisé en ce que l'on fait réagir un composé de la formule:

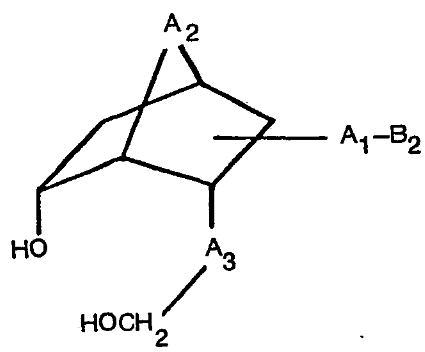

dans laquelle $A_1$, $A_2$, $A_3$ et $B_2$ sont chacun tels que définis ci-dessus, sur un agent de déshydration.

15. Procédé de production d'un composé suivant la revendication 2, de la formule:

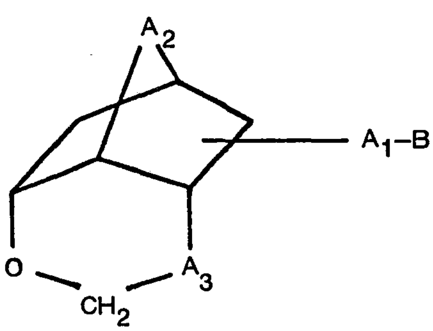

dans laquelle $A_1$, $A_2$, $A_3$ et B sont chacun tels que définis dans la revendication 2, caractérisé en ce que l'on cyclise un composé de la formule:

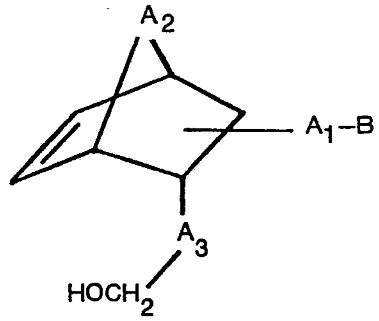

dans laquelle $A_1$, $A_2$, $A_3$ et B sont chacun tels que définis plus haut, par l'intermédiaire d'une réaction d'oxymercuration-démercuration.

16. Procédé de production d'un composé suivant la revendication 2, de la formule:

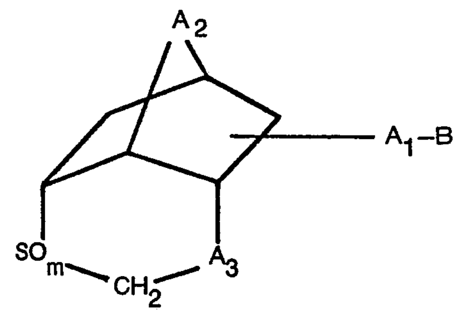

dans laquelle $A_1$, $A_2$, $A_3$ et B sont chacun tels que définis dans la revendication 2 et m représente un nombre entier dont la valeur est égale à 1 ou à 2, caractérisé en ce que l'on oxyde un composé de la formule:

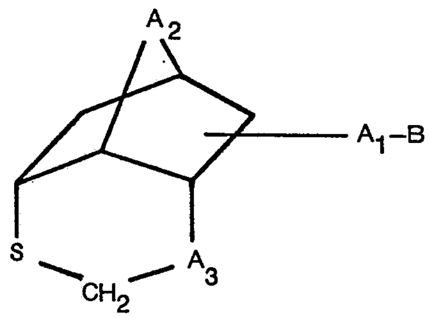

dans laquelle $A_1$, $A_2$, $A_3$ et B sont chacun tels que définis plus haut.

17. Procédé de production d'un composé suivant la revendication 2, de la formule:

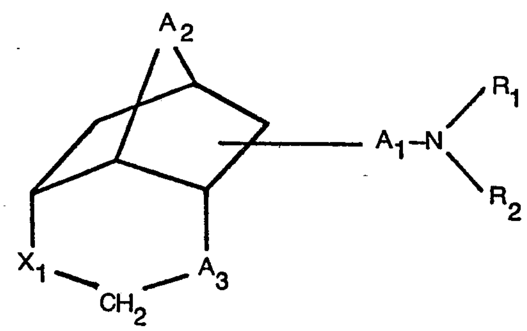

dans laquelle $A_1$, $A_2$, $A_3$, $R_1$ et $R_2$ sont chacun tels que définis dans la revendication 2, et $X_1$ représente —O— ou —S—, caractérisé en ce que l'on réduit:

a) un composé de la formule:

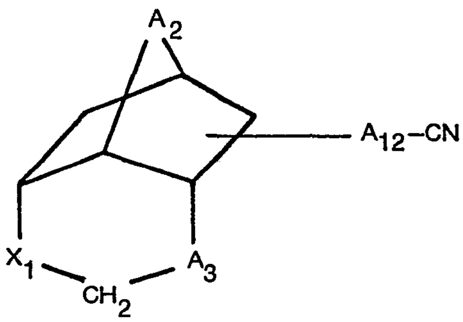

dans laquelle $A_2$ et $A_3$ sont chacun tels que définis plus haut, $A_{12}$ représente une simple liaison ou un groupe alkylène en $C_1$—$C_4$ et $X_1$ représente —O— ou —S—, avec un composé du type hydrure de métal ou de l'hydrogène gazeux, en présence d'un catalyseur;

b) un composé de la formule:

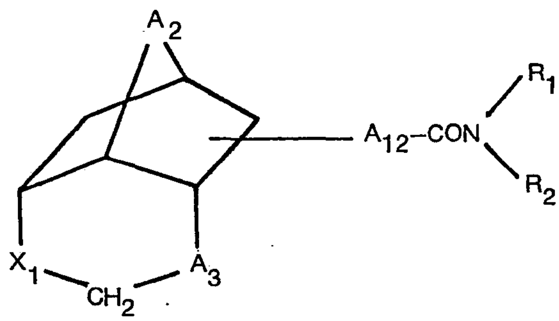

dans laquelle $A_{12}$, $A_2$, $A_3$, $R_1$, $R_2$ et $X_1$ sont chacun tels que définis plus haut, avec un composé du type hydrure de métal;

c) un composé de la formule:

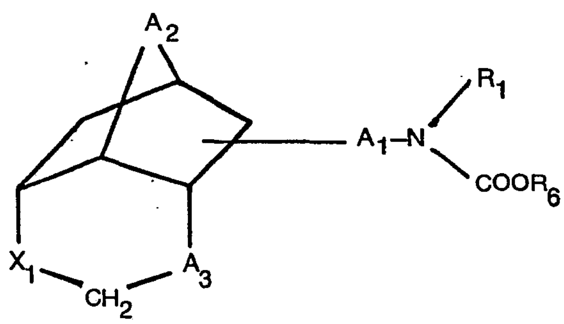

dans laquelle $A_1$, $A_2$, $A_3$, $X_1$ et $R_1$ sont chacun tels que définis plus haut et $R_6$ représente un groupe alkyle en $C_1$—$C_4$, avec un composé du type hydrure de métal;

d) un composé de la formule:

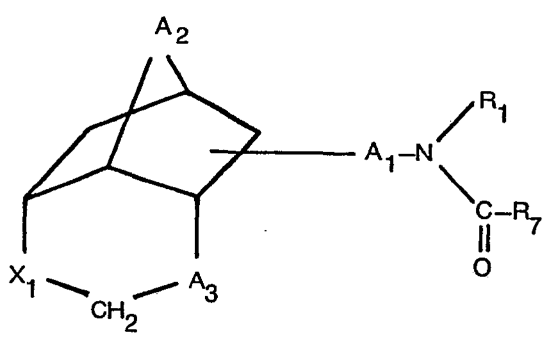

dans laquelle $A_1$, $A_2$, $A_3$, $X_1$ et $R_1$ sont chacun tels que définis plus haut et $R_7$ représente un atome d'hydrogène, un groupe alkyle en $C_1$—$C_4$ ou un groupe alcényle en $C_2$—$C_4$, avec un composé du type hydrure de métal;

e) un composé de la formule:

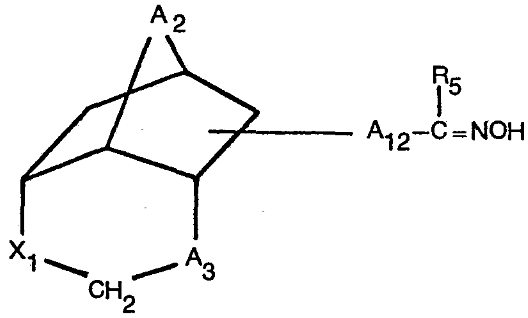

dans laquelle $A_{12}$, $A_2$, $A_3$ et $X_1$ sont chacun tels que définis plus haut et $R_5$ représente un atome d'hydrogène ou un groupe alkyle en $C_1$—$C_4$, avec un composé du type hydrure de métal ou de l'hydrogène gazeux, en présence d'un catalyseur; ou

f) un composé de la formule:

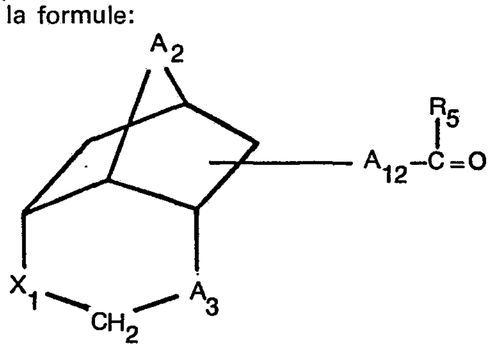

dans laquelle $A_{12}$, $A_2$, $A_3$, $R_5$ et $X_1$ sont chacun tels que définis plus haut, avec un agent réducteur, en présence d'un composé du type amine de la formule:

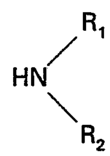

dans laquelle $R_1$ et $R_2$ sont chacun tels que définis plus haut.

18. Procédé de production d'un composé suivant la revendication 2, de la formule:

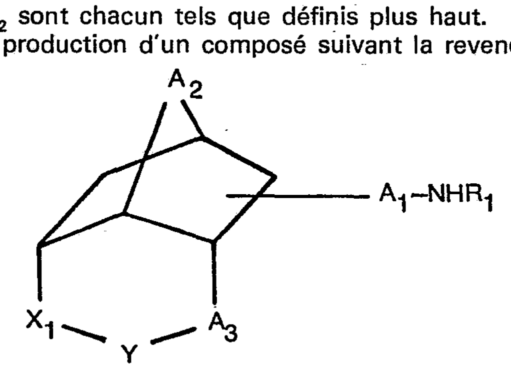

dans laquelle $A_1$, $A_2$, $A_3$ et $R_1$ sont chacun tels que définis dans la revendication 2, X—Y représente un groupe —O—$CH_2$—, —S—$CH_2$— ou —O—CO—, caractérisé en ce que l'on débenzyle et décarboxyle un composé de la formule:

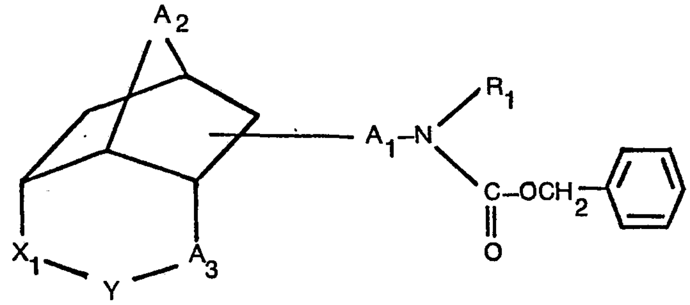

dans laquelle $A_1$, $A_2$, $A_3$, $R_1$ et $X_1$—Y sont chacun tels que définis plus haut.

19. Procédé de production de composés suivant la revendication 2, des formules:

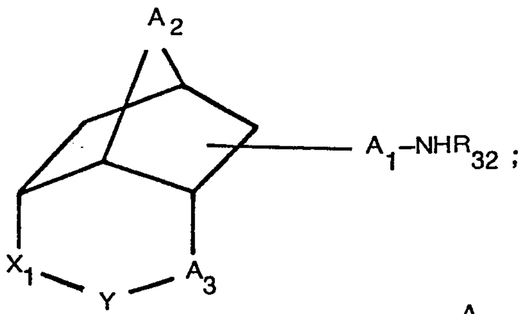

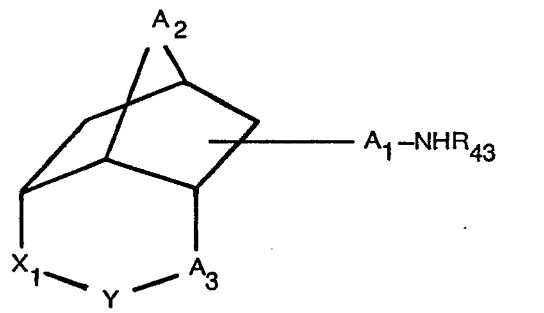

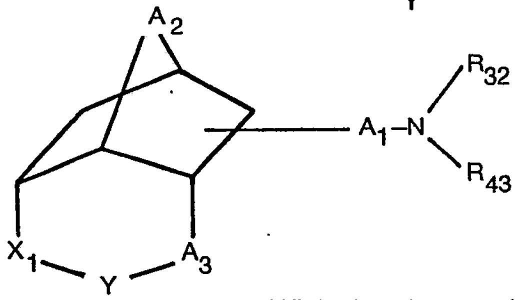

dans lesquelles $A_1$, $A_2$ et $A_3$ sont chacun tels que définis dans la revendication 2, $X_1$—Y représente un groupe —O—$CH_2$—, —S—$CH_2$— ou —O—CO—, $R_{32}$ est identique à $R_3$ dans la revendication 2 à l'exclusion d'un atome d'hydrogène et $R_{43}$ est identique à $R_4$ dans la revendication 2 à l'exclusion d'un atome d'hydrogène, caractérisé en ce que l'on fait réagir un composé de la formule

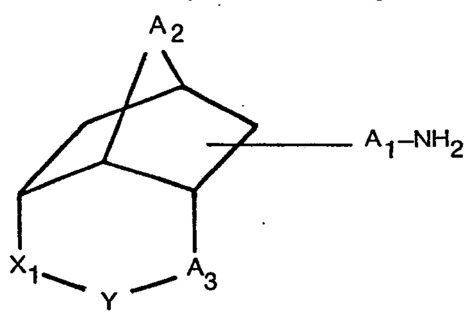

dans laquelle $A_1$, $A_2$, $A_3$ et $X_1$—Y sont chacun tels que définis plus haut, sur un radical activé $R_{32}$—Z ou $R_{43}$—Z (où Z est un reste d'acide fort).

20. Procédé de production d'un composé suivant la revendication 2, de la formule:

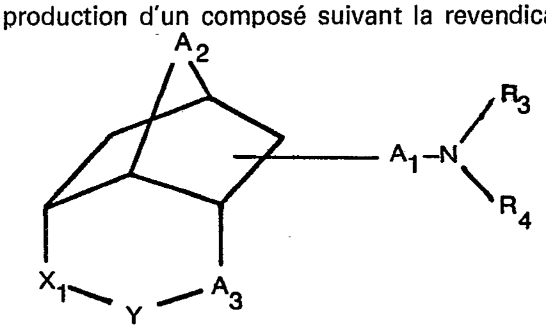

dans laquelle $A_1$, $A_2$, $A_3$, $R_3$ et $R_4$ sont chacun tels que définis dans la revendication 2 et $X_1$—Y représente un groupe —O—$CH_2$—, —S—$CH_2$— ou —O—CO—, caractérisé en ce que l'on fait réagir un composé de la formule:

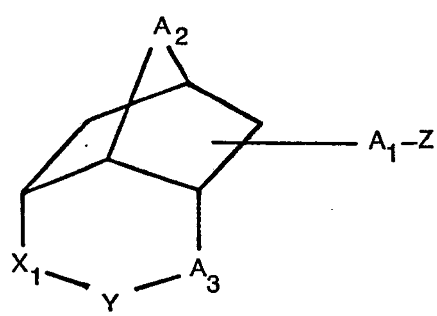

dans laquelle $A_1$, $A_2$, $A_3$ et $X_1$—Y sont chacun tels que définis plus haut et Z représente le reste d'un acide fort, sur un composé du type amine de la formule:

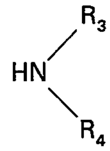

dans laquelle $R_3$ et $R_4$ sont chacun tels que définis plus haut.

21. Procédé de production d'un composé suivant la revendication 2, de la formule

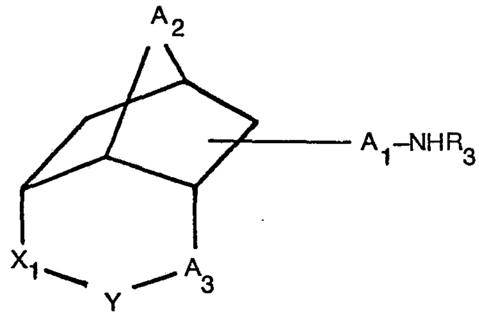

dans laquelle $A_1$, $A_2$, $A_3$ et $R_3$ sont chacun tels que définis dans la revendication 2 et $X_1$—Y représente un groupe —O—$CH_2$—, —S—$CH_2$ ou —O—CO—, caractérisé en ce que l'on hydrolyse un composé de la formule:

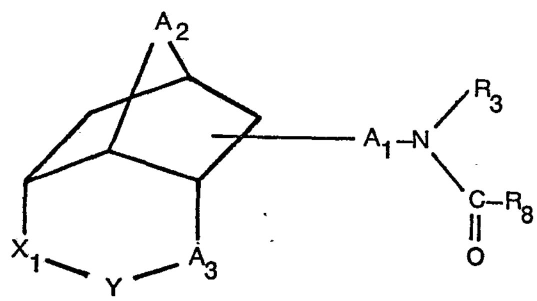

dans laquelle $A_1$, $A_2$, $A_3$, $R_3$ et $X_1$—Y sont chacun tels que définis plus haut et $R_8$ représente un atome d'hydrogène, un groupe alkyle en $C_1$—$C_4$, un groupe haloalkyle en $C_1$—$C_3$, un groupe alcoxy en $C_1$—$C_4$ ou un groupe benzyloxy.

22. Procédé de production d'un composé suivant la revendication 2, de la formule:

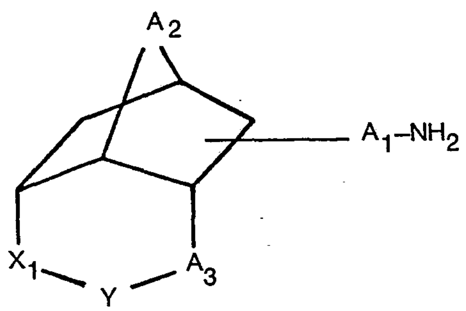

dans laquelle $A_1$, $A_2$ et $A_3$ sont chacun tels que définis dans la revendication 2 et $X_1$—Y représente un groupe —O—$CH_2$—, —S—$CH_2$— ou —O—CO—, caractérisé en ce que l'on hydrolyse un composé de la formule:

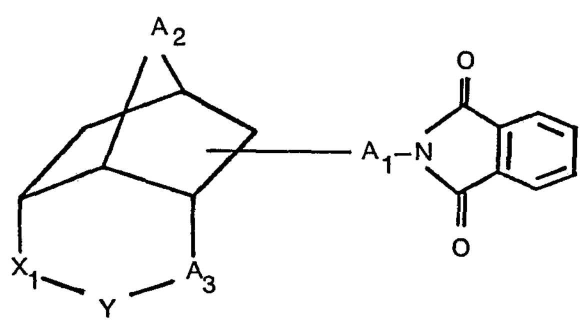

dans laquelle $A_1$, $A_2$, $A_3$ et $X_1$—Y sont chacun tels définis plus haut.

23. Procédé de production d'un composé selon la revendication 2, de la formule:

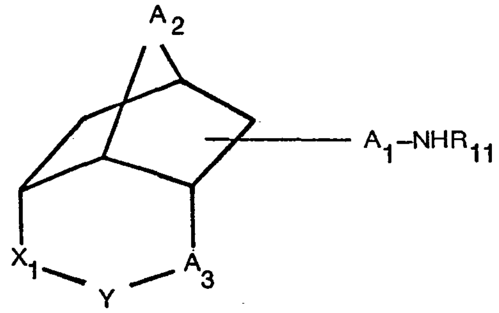

dans laquelle $A_1$, $A_2$, $A_3$ sont chacun tels que définis dans la revendication 2 et $X_1$—Y représente un groupe —O—$CH_2$—, —S—$CH_2$— ou —O—CO— et $R_{11}$ représente un atome d'hydrogène, un groupe alkyloxycarbonyle en $C_2$—$C_5$ ou un groupe benzyloxycarbonyle, caractérisé en ce que l'on procédé au traitement:

a) d'un composé de la formule:

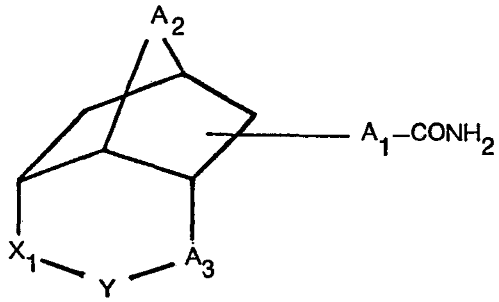

dans laquelle $A_1$, $A_2$, $A_3$ et $X_1$—Y sont chacun tels que définis plus haut, par un hypohalite d'alcali, ce traitement étant suivi d'une hydrolyse ou du traitement de l'isocyanate ainsi obtenu par un composé du type alcool de la formule: $R_9$—OH (dans laquelle $R_9$ représente un groupe alkyle en $C_1$—$C_4$ ou un groupe benzyle);

b) d'un composé de la formule:

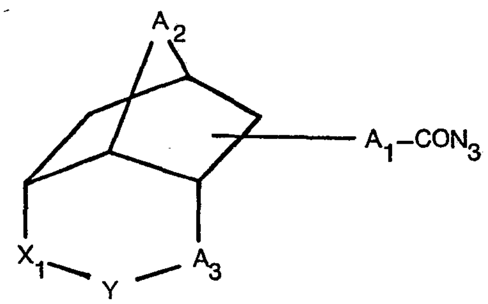

dans laquelle $A_1$, $A_2$, $A_3$ et $X_1$—Y sont chacun tels que définis plus haut, par chauffage, ce traitement étant suivi d'une hydrolyse ou du traitement des isocyanates ainsi obtenus par un composé du type alcool de la formule: $R_9$—OH (dans laquelle $R_9$ représente un groupe alkyle en $C_1$—$C_4$ ou un groupe benzyle);

c) d'un composé de la formule:

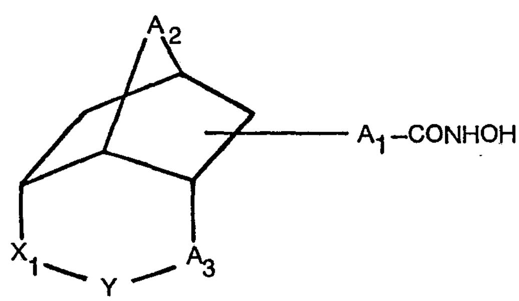

dans laquelle $A_1$, $A_2$, $A_3$ et $X_1$—Y sont chacun tels que définis plus haut, par chauffage avec ou sans base, ce traitement étant suivi de l'hydrolyse ou du traitement de l'isocynate ainsi obtenu par un composé du type alcool de la formule: $R_9$—OH (dans laquelle $R_9$ représente un groupe alkyle en $C_1$—$C_4$ ou un groupe benzyle).

24. Procédé de production d'un composé suivant la revendication 2, de la formule:

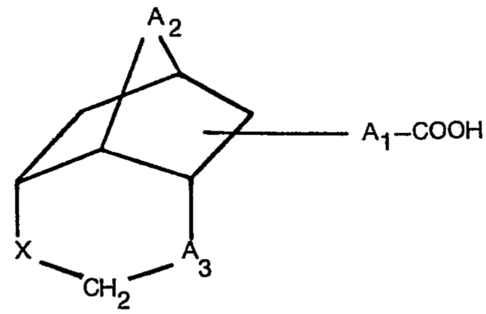

dans laquelle $A_1$, $A_2$ et $A_3$ sont chacun tels que définis dans la revendication 2 et X représente —O— ou —$SO_n$— (n=0,1 ou 2), caractérisé en ce que l'on hydrolyse un composé de la formule:

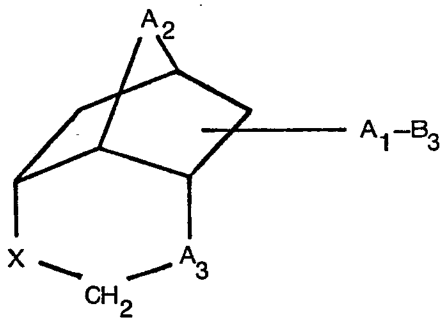

dans laquelle $A_1$, $A_2$, $A_3$ et X sont chacun tels que définis plus haut et $B_3$ représente un groupe cyano, un groupe alcoxycarbonyle en $C_2$—$C_5$, un groupe benzyloxycarbonyle ou un groupe carbamoyle de la formule:

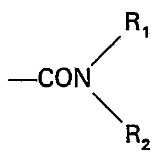

(dans laquelle $R_1$ et $R_2$ sont chacun tels que définis dans la revendication 2).

25. Procédé de production d'un composé suivant la revendication 2, de la formule:

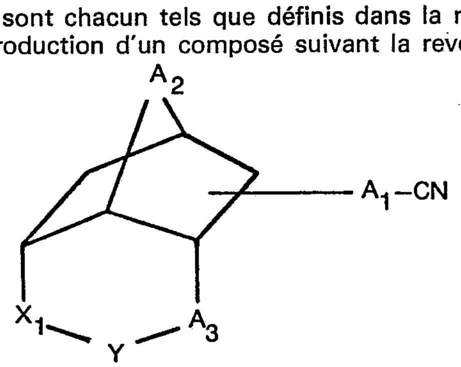

dans laquelle $A_1$, $A_2$ et $A_3$ sont chacun tels que définis dans la revendication 2 et $X_1$—Y représente un groupe —O—$CH_2$—, ou —S—$CH_2$— caractérisé en ce que l'on fait réagir un composé de la formule:

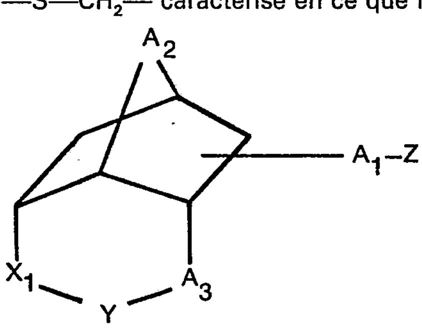

dans laquelle $A_1$, $A_2$, $A_3$ et $X_1$—Y sont chacun tels que définis plus haut et Z représente le reste d'un acide fort, sur un composé du type cyanure.

26. Procédé de production d'un composé suivant la revendication 2, de la formule:

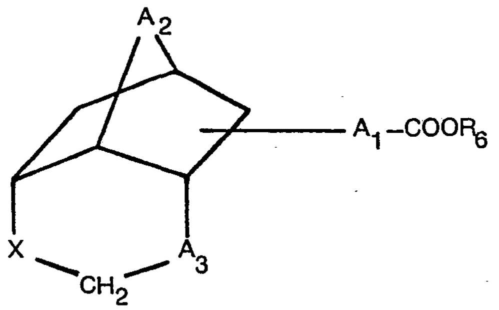

dans laquelle $A_1$, $A_2$ et $A_3$ sont chacun tels que définis dans la revendication 2, X représente —O— ou —S— et $R_6$ représente un groupe alkyle en $C_1$—$C_4$, caractérisé en ce que l'on fait réagir:

a) un composé de la formule:

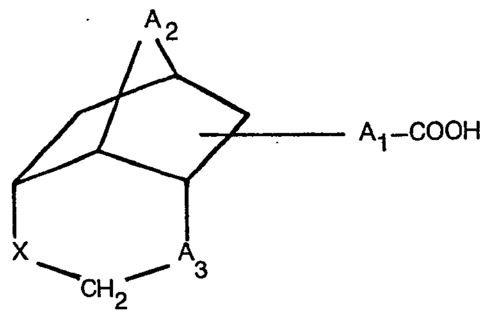

dans laquelle $A_1$, $A_2$, $A_3$ et X sont chacun tels que définis plus haut, sur un alcool de la formule: $R_6$—OH (dans laquelle $R_6$ est tel que défini plus haut), en présence d'un catalyseur acide;

b) un composé de la formule:

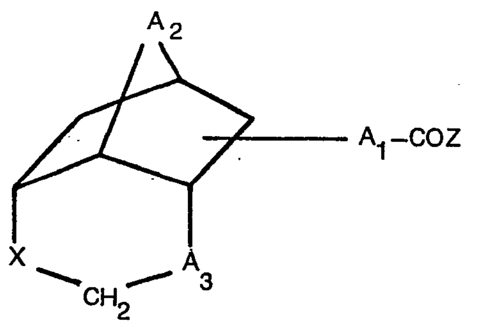

dans laquelle $A_1$, $A_2$, $A_3$ et X sont chacun tels que définis plus haut et —COZ désigne un groupe ester activé, sur un alcool de la formule : $R_6$—OH (dans laquelle $R_6$ est tel que défini plus haut).

27. Procédé de production d'un composé suivant la revendication 2 de la formule:

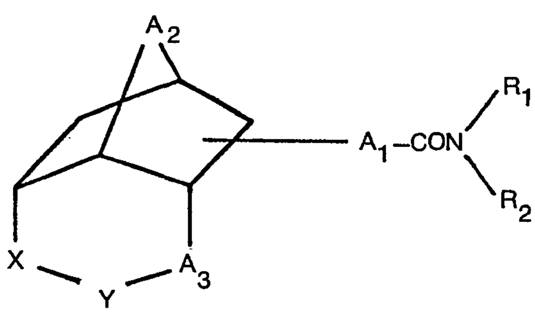

dans laquelle X—Y représente un groupe —O—$CH_2$— ou —S—$CH_2$ et $A_1$, $A_2$, $A_3$, $R_1$ et $R_2$ sont chacun

tels que définis dans la revendication 1, caractérisé en ce que l'on fait réagir un acide carboxylique ou un dérivé de ce dernier, de la formule:

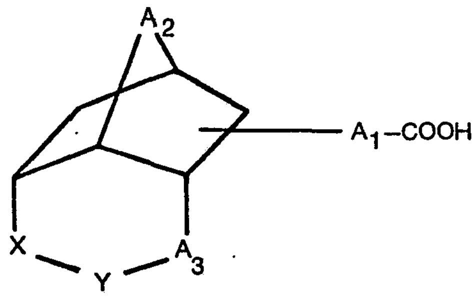

dans laquelle $A_1$, $A_2$, $A_3$ et X—Y sont chacun tels que définis plus haut, sur une amine de la formule:

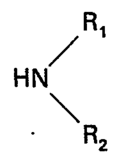

dans laquelle $R_1$ et $R_2$ sont chacun définis plus haut.